Europäisches Patentamt

European Patent Office

Office européen des brevets

(19)

(11) **EP 1 630 236 A2**

(12) **EUROPÄISCHE PATENTANMELDUNG**

(43) Veröffentlichungstag:
**01.03.2006 Patentblatt 2006/09**

(51) Int Cl.:
***C12Q 1/68*** (2006.01)

(21) Anmeldenummer: **05018838.2**

(22) Anmeldetag: **30.08.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **30.08.2004 EP 04020543**

(71) Anmelder: **FBF- Förderverein Biologieforschung der Deutschen Schweineproduktion e.V.
53113 Bonn (DE)**

(72) Erfinder:
• **Brenig, Bertram, Prof. Dr. Dr.
37079 Göttingen (DE)**
• **Harlizius, Barbara, Dr.
53804 Much (DE)**
• **Knorr, Christoph, Dr.
37085 Göttingen (DE)**
• **Bornemann-Kolatzki, Kirsten
37130 Gleichen (DE)**

(74) Vertreter: **Vossius & Partner
Siebertstrasse 4
81675 München (DE)**

(54) **Hernia inguinalis/scrotalis assozieerte Genomregionen**

(57) Die Erfindung betrifft die Verwendung einer ersten Nukleinsäure zur Bestimmung der Prädisposition zur Ausprägung oder Vererbung des Phänotypus "Hernia inguinalis/scrotalis" bei einem Säuger, wobei die erste Nukleinsäure eine Länge von mindestens 8 Nukleotiden aufweist und identisch oder im wesentlichen identisch ist mit einer zweiten Nukleinsäure, die vorkommt, (a) auf Chromosom 12 von Sus scrofa im Bereich zwischen 19,3 cM und 85,05 cM; (b) auf Chromosom 6 von Sus scrofa im Bereich zwischen 71,4 cM und 96,1 cM oder im Bereich von 0 cM; (c) auf Chromosom 3 von Sus scrofa im Bereich zwischen 12,4 cM und 19 cM oder im Bereich zwischen 98,2 cM und 102,2 cM; (d) auf Chromosom 7 von Sus scrofa im Bereich zwischen 58,5 cM und 59,7 cM; (e) auf Chromosom 15 von Sus scrofa im Bereich zwischen 13,8 cM und 25,7 cM; oder (f) in dem chromosomalen Bereich eines anderen Säugers, der zu den in (a) bis (e) genannten Bereichen ortholog ist; wobei die erste Nukleinsäure in dem oben genannten Bereich lokalisiert ist oder 5 Megabasen stromaufwärts und/oder stromabwärts hiervon. Weiterhin betrifft die Erfindung ein in vitro Verfahren zur Bestimmung der Prädisposition zur Ausprägung oder Vererbung des Phänotypus "Hernia inguinalis/scrotalis", wobei man die Säuger, deren befruchtete oder unbefruchtete Eizellen, oder deren Sperma auf die Anwesenheit, Beschaffenheit oder Ausprägung einer der in den erfindungsgemäßen Ausführungsformen genannten zweiten Nukleinsäure testet. Schließlich betrifft die Erfindung ein Kit mindestens enthaltend (a) ein Primerpaar zur Amplifikation der in den erfindungsgemäßen Ausführungsformen genannten zweiten Nukleinsäure, wobei jeweils ein Primer an den + Strang und ein weiterer Primer an den - Strang dieser Nukleinsäure bindet; oder (b) eine Hybridisierungssonde mit einer Länge von mindestens 8 Nukleotiden die an die in den in den erfindungsgemäßen Ausführungsformen genannte zweite Nukleinsäure bindet; oder (c) einen spezifischen Antikörper oder ein Antikörperfragment oder ein Antikörperderivat oder ein Aptamer das an die in den erfindungsgemäßen Ausführungsformen genannte erste oder zweite Nukleinsäure bindet; in einem oder mehreren Behältern.

EP 1 630 236 A2

## SEQUENCE LISTING

<110> FBF-Förderverein Biotechnologieforschung der Deutschen Schweineproduktion e.V.

<120> Hernia Inguinalis/Scrotalis Assoziierte Genomregionen

<130> K2299 EP

<160> 48

<170> PatentIn version 3.1

<210> 1

<211> 22

<212> DNA

<213> Sus scrofa

<400> 1
gcgacacatg agataaaact gc                                          22

<210> 2

<211> 22

<212> DNA

<213> Sus scrofa

<400> 2
aatccacagg cttactcaga tg                                          22

<210> 3

<211> 21

<212> DNA

<213> Sus scrofa

<400> 3
tctttttagg gtggaggatg g                                           21

<210> 4

<211> 22

<212> DNA

<213> Sus scrofa

<400> 4
catgtcccct atgaactctg tg                                     22

<210> 5

<211> 20

<212> DNA

<213> Sus scrofa

<400> 5
ctgactgttt tgctgcagtg                                        20

<210> 6

<211> 22

<212> DNA

<213> Sus scrofa

<400> 6
tccactgagg tctctcactc tc                                     22

<210> 7

<211> 18

<212> DNA

<213> Sus scrofa

<400> 7
atcagaacag tgcgccgt                                          18

<210> 8

<211> 20

<212> DNA

<213> Sus scrofa

<400> 8
tttgaaaatg gggtgtttcc                                                    20

<210> 9

<211> 20

<212> DNA

<213> Sus scrofa

<400> 9
tctaaacaaa gggcaggtgg                                                    20

<210> 10

<211> 20

<212> DNA

<213> Sus scrofa

<400> 10
ggacttccat atgctgtggg                                                    20

<210> 11

<211> 20

<212> DNA

<213> Sus scrofa

<400> 11
gaagccaaag agacaactgc                                                    20

<210> 12

<211> 20

<212> DNA

<213> Sus scrofa

<400> 12
gttctttacc cactgagcca                                                                    20

<210> 13

<211> 23

<212> DNA

<213> Sus scrofa

<400> 13
ctgccaagag aggctcttct caa                                                                23

<210> 14

<211> 23

<212> DNA

<213> Sus scrofa

<400> 14
catccgcctg gttccctccc tat                                                                23

<210> 15

<211> 18

<212> DNA

<213> Sus scrofa

<400> 15
tgctggccag tgactctg                                                                      18

<210> 16

<211> 20

<212> DNA

<213> Sus scrofa

<400> 16
ccgggggatt aaacaaaaag                                                                    20


<210> 17

<211> 23

<212> DNA

<213> Sus scrofa

<400> 17
gtgctggatg tcctgtgttc cct                                                                23


<210> 18

<211> 25

<212> DNA

<213> Sus scrofa

<400> 18
ctggtgacat agttgatgag gtttg                                                              25


<210> 19

<211> 20

<212> DNA

<213> Sus scrofa

<400> 19
tgatttacaa tgttgggtcg                                                                    20


<210> 20

<211> 22

<212> DNA

<213> Sus scrofa

<400> 20
gatcatgtga gaaaaaagaa tg                                                                 22

<210> 21

<211> 23

<212> DNA

<213> Sus scrofa

<400> 21
ttgtcttttt attttgcttt tgg                                                23


<210> 22

<211> 22

<212> DNA

<213> Sus scrofa

<400> 22
caaaaaaggc aaaagattga ca                                                 22


<210> 23

<211> 21

<212> DNA

<213> Sus scrofa

<400> 23
ctgggtttac agtgttctgc c                                                  21


<210> 24

<211> 21

<212> DNA

<213> Sus scrofa

<400> 24
tgtgatggag cctgatagag g                                                  21


<210> 25

<211> 22

<212> DNA

<213> Sus scrofa

<400> 25
ttgttatgcc tacctgtgtt gg                                                    22

<210> 26

<211> 20

<212> DNA

<213> Sus scrofa

<400> 26
ctccatatgc cacaggtgtg                                                       20

<210> 27

<211> 20

<212> DNA

<213> Sus scrofa

<400> 27
ttctccagcc atcatgagtg                                                       20

<210> 28

<211> 20

<212> DNA

<213> Sus scrofa

<400> 28
aatgaccatt cctgaggctg                                                       20

<210> 29

<211> 18<

212>   DNA

<213> Sus scrofa

<400> 29
aggtaacctg ggcttggg                                                          18


<210> 30

<211> 18

<212> DNA

<213> Sus scrofa

<400> 30
accaatatgc tgggcacc                                                          18


<210> 31

<211> 22

<212> DNA

<213> Sus scrofa

<400> 31
aaaatgaacc ctctccagtt tc                                                     22


<210> 32

<211> 19

<212> DNA

<213> Sus scrofa

<400> 32
tctgaacact acagcccgc                                                         19


<210> 33

<211> 26

<212> DNA

<213> Sus scrofa

<400> 33
ttggcattta ctgtctttag tgacga                                                    26

<210> 34

<211> 24

<212> DNA

<213> Sus scrofa

<400> 34
ggccatatct ggtattgggt gtct                                                      24

<210> 35

<211> 25

<212> DNA

<213> Sus scrofa

<400> 35
tcatccttcc ttttctttat ttctt                                                     25

<210> 36

<211> 18

<212> DNA

<213> Sus scrofa

<400> 36
tctggctcgg atgcaatc                                                             18

<210> 37

<211> 22

<212> DNA

<213> Sus scrofa

<400> 37
ctggggagct tacaaactcc tt                                                        22

<210> 38

<211> 24

<212> DNA

<213> Sus scrofa

<400> 38
ttatccatta gcacatgcct gcca                                24


<210> 39

<211> 22

<212> DNA

<213> Sus scrofa

<400> 39
aaaagaaccc aactacagca gc                                  22


<210> 40

<211> 22

<212> DNA

<213> Sus scrofa

<400> 40
tttatgaggg tatcctgaca cc                                  22


<210> 41

<211> 20

<212> DNA

<213> Sus scrofa

<400> 41
gcactttctt cccttacccc                                     20


<210> 42

<211> 22

<212> DNA

<213> Sus scrofa

<400> 42
cagtgtaaag catggaagat gc            22

<210> 43

<211> 24

<212> DNA

<213> Sus scrofa

<400> 43
ccaagactgc cttgtaggtg aata            24

<210> 44

<211> 24

<212> DNA

<213> Sus scrofa

<400> 44
gctatcaagt attgtaccat tagg            24

<210> 45

<211> 26

<212> DNA

<213> Sus scrofa

<400> 45
tctggctcct acactccttc ttgatg            26

<210> 46

<211> 24

<212> DNA

<213> Sus scrofa

<400> 46
ttgggtgggt gctgaaaaat agga 24

<210> 47

<211> 23

<212> DNA

<213> Sus scrofa

<400> 47
tccaaagtca tgaagattta ttc 23

<210> 48

<211> 23

<212> DNA

<213> Sus scrofa

<400> 48
tcacagacct aaatgtaaga gct 23

**Beschreibung**

Die Erfindung betrifft die Verwendung einer ersten Nukleinsäure zur Bestimmung der Prädisposition zur Ausprägung oder Vererbung des Phänotypus "Hernia inguinalis/scrotalis" bei einem Säuger, wobei die erste Nukleinsäure eine Länge von mindestens 8 Nukleotiden aufweist und identisch oder im wesentlichen identisch ist mit einer zweiten Nukleinsäure, die vorkommt, (a) auf Chromosom 12 von Sus scrofa im Bereich zwischen 19,3 cM und 85,05 cM; (b) auf Chromosom 6 von Sus scrofa im Bereich zwischen 71,4 cM und 96,1 cM oder im Bereich von 0 cM; (c) auf Chromosom 3 von Sus scrofa im Bereich zwischen 12,4 cM und 19 cM oder im Bereich zwischen 98,2 cM und 102,2 cM; (d) auf Chromosom 7 von Sus scrofa im Bereich zwischen 58,5 cM und 59,7 cM; (e) auf Chromosom 15 von Sus scrofa im Bereich zwischen 13,8 cM und 25,7 cM; oder (f) in dem chromosomalen Bereich eines anderen Säugers, der zu den in (a) bis (e) genannten Bereichen ortholog ist; wobei die erste Nukleinsäure in dem oben genannten Bereich lokalisiert ist oder 5 Megabasen stromaufwärts und/oder stromabwärts hiervon. Weiterhin betrifft die Erfindung ein in vitro Verfahren zur Bestimmung der Prädisposition zur Ausprägung oder Vererbung des Phänotypus "Hernia inguinalis/scrotalis", wobei man die Säuger, deren befruchtete oder unbefruchtete Eizellen, oder deren Sperma auf die Anwesenheit, Beschaffenheit oder Ausprägung einer der in den erfindungsgemäßen Ausführungsformen genannten zweiten Nukleinsäure testet. Schließlich betrifft die Erfindung ein Kit mindestens enthaltend (a) ein Primerpaar zur Amplifikation der in den erfindungsgemäßen Ausführungsformen genannten zweiten Nukleinsäure, wobei jeweils ein Primer an den + Strang und ein weiterer Primer an den - Strang dieser Nukleinsäure bindet; oder (b) eine Hybridisierungssonde mit einer Länge von mindestens 8 Nukleotiden die an die in den in den erfindungsgemäßen Ausführungsformen genannte zweite Nukleinsäure bindet; oder (c) einen spezifischen Antikörper oder ein Antikörperfragment oder ein Antikörperderivat oder ein Aptamer das an die in den erfindungsgemäßen Ausführungsformen genannte erste oder zweite Nukleinsäure bindet; in einem oder mehreren Behältern.

Erblich bedingte Anomalien stellen einen ökonomischen Verlust bei der Produktion von Zuchtschweinen sowie bei der Ferkelproduktion dar. Der ökonomische Schaden einzelner Geburtsfehler ist abhängig von der Häufigkeit der Merkmalsträger in der Population sowie den durch Erbfehler entstehenden Behandlungskosten und geringeren Zunahmen (STIGLER et al., 1991). Hernien verursachen wirtschaftliche Schäden durch anfallende Behandlungskosten und geringere Tageszunahmen der betroffenen Tiere, die außerdem oft verfrüht und damit untergewichtig geschlachtet wenden müssen. Pro Brüchling ergeben sich Kosten von ca. 25 € (FBF-POLYKOPIE 2001). Aus den geschätzten Frequenzen in der deutschen Schweineproduktion und den Kosten pro Brüchling ergeben sich Verluste durch die Anomalie Hodensackbruch (unterstellte Häufigkeit: 1,24) von etwa 1.812.302 € und durch Leisten- und Nabelbrüche (unterstellte Häufigkeit: 0,65) von etwa 1.899.992 € (ALTHOFF 1985). Nach Berechnungen des FBF kommt in 2 % aller 4 Millionen Würfe in Deutschland im Jahr mindestens ein betroffenes Ferkel vor. Es ergeben sich damit 80.000 betroffene Tiere und hieraus ein ökonomischer Verlust in Höhe von etwa 2 Millionen € (FBF-POLYKOPIE 2001).

Die Entwicklung molekulargenetischer Methoden, besonders die Entdeckung der Mikrosatelliten als polymorphe Marker, hat es ermöglicht, die molekulargenetischen Grundlagen vieler wirtschaftlich wichtiger Merkmale und Erbdefekte zu identifizieren. Die Zahl der genetisch kartierten Loci beim Schwein beträgt zur Zeit (Rothschild, 2001) ungefähr 2000 Marker und Gene. Dies ermöglicht eine genomweite Suche nach Genomregionen, die Einfluss auf das betrachtete Merkmal ausüben. Eine große Anzahl von Untersuchungen wurden und werden im Hinblick auf Merkmale wie Reproduktionsleistung, Mastleistung und Schlachtkörperwert, Fleischqualität, Krankheitsresistenz, Immunantwort und andere Merkmale durchgeführt.

In den letzten zehn Jahren hat sich die Genkartierung beim Schwein rasch entwickelt. Dies war hauptsächlich die Leistung von drei Forschergruppen, nämlich in Europa das PiGMaP-Programm (Archibald et al. 1991; Archibald et al. 1995; Yerle et al. 1997) und das nordische Konsortium (Ellegren et al. 1994; Marklund et al. 1996) und in den USA das Meat Animal Research Center des US Department of Agriculture (USDA) (Rohrer et al. 1994). Genetische Karten werden durch Kopplungsanalysen erstellt (Matise, T.C., Perlin, M. and Chakravarti, 1994: Automated construction of genetic linkage maps using an expert system (MultiMap): a human genome linkage map. Nature Genetics, 6:384-389). Dafür sind zwei Voraussetzungen erforderlich, nämlich eine Tierpopulation mit bekannter Abstammung (Familienstruktur) und zahlreiche polymorphe Loci (Archibald and Haley 1998). Als polymorphe Loci kommen molekulargenetische Marker in Frage (Jame and Lagoda 1996; Hui Liu 1998; Luikart and England 1999). Ein molekulargenetischer Marker kann definiert werden als ein Abschnitt des Erbmaterials, der eine bestimmte Eigenschaft hat oder hervorruft. Es handelt sich dabei um einen gekennzeichneten Locus, der von Generation zu Generation vererbt wird (Nagel 1996; O'Brien et al. 1999). Dass man die molekulargenetischen Marker relativ einfach identifizieren kann und dass sie zahlreich zur Verfügung stehen, sind Vorteile gegenüber anderen Markersystemen wie biochemischen oder immunologischen Markern. Die Genomanalyse beim Hausschwein wird hauptsächlich von zwei molekularen Markertypen dominiert. Diese Markertypen sind Restriktionsfragment-Längenpolymorphismen (RFLPs) und Mikrosatelliten (Jarne and Lagoda 1996; Montaldo and Herrera-Meza 1998). Die meisten RFLPs sind diallelisch und haben nach Hui Liu (1998) im Vergleich mit Mikrosatelliten niedrige PIC-Werte (Polymorphism Information Content). Darüber hinaus ist die Darstellung von RFLPs zeit- und kostenaufwendig (Botstein et al. 1980; Winter et al, 1992; Yue 1999). Mikrosatelliten dagegen sind zahlreich vorhanden und haben hohe PIC-Werte. Etwa 65.000 bis 100.000 Mikrosatellitenloci sind im Schweinegenom gleichmäßig verteilt (Ellegren 1993; Schlötterer 1997; Dounavi 2000). Die Identifizierung von Mikrosatelliten wird von verschiedenen Labors durchgeführt, die Zahl von identifizierten Mikrosatelliten beträgt etwa 1600 (5/2004).

Bei Genotypisierungen oder Genomscreening-Verfahren wird festgestellt, ob das Vorhandensein bestimmter polymorpher Abschnitte der DNA oder spezifischer Allele eines Gens mit der Vererbung oder Ausprägung eines Phänotyps korreliert (Assoziation). Hierbei kann man davon ausgehen, dass mit dem Merkmal assoziierte polymorphe DNA in Nachbarschaft des für den Phänotyp verantwortlichen Gens gelegen ist und dadurch, mit einer gewissen Wahrscheinlichkeit, gemeinsam mit diesem vererbt wird. Werden zwei oder mehrere polymorphe Marker mit hoher Frequenz gemeinsam vererbt, so definieren sie einen quasi-stabilen genetischen "Haplotyp". Die Assoziation eines spezifischen Haplotyps mit einem Phänotyp (z.B. dem der "Hernia inguinalis/scrotalis") kann als diagnostischer oder prognostischer Marker Verwendung finden, der es ermöglicht, Aussagen über die Wahrscheinlichkeit des Auftretens oder über die Vererbung eines Phänotyps zu treffen. Hierbei gilt zu bedenken, dass solche prognostischen oder diagnostischen Nachweisverfahren unabhängig sind von der Identifizierung des Phänotyp-verursachenden Gens. Dies ist bedeutend, da die Etablierung der molekularen Grundlagen eines Phänotyps oft sehr schwierig und arbeitsaufwendig ist, insbesondere im Zusammenhang mit multifaktoriell-bedingten Phänotypen.

Eine geringe Anzahl von Merkmalen von Schweinen und anderen Säugern können mit Hilfe von genetischen Markern vorhergesagt werden. Es gibt bis heute allerdings keine Möglichkeit, eine Prädisposition zur Vererbung oder Ausprägung des Phänotyps "Hernia inguinalis/scrotalis" nachzuweisen.

Der vorliegenden Erfindung liegt daher die Aufgabe, zugrunde, Möglichkeiten zur Verfügung zu stellen, durch die Tiere identifiziert werden können, die zur Ausprägung des Phänotyps "Hernia inguinalis/scrotalis" prädisponiert sind oder eine solche Prädisposition vererben. Diese Aufgabe wird erfindungsgemäß durch die Bereitstellung der in den Patentansprüchen charakterisierten Ausführungsformen gelöst.

Somit betrifft die Erfindung die Verwendung einer ersten Nukleinsäure zur Bestimmung der Prädisposition zur Ausprägung oder Vererbung des Phänotypus "Hernia inguinalis/scrotalis" bei einem Säuger, wobei die erste Nukleinsäure eine Länge von mindestens 8 Nukleotiden aufweist und identisch oder im wesentlichen identisch ist mit einer zweiten Nukleinsäure, die vorkommt, (a) auf Chromosom 12 von Sus scrofa im Bereich zwischen 19,3 cM und 85,05 cM; (b) auf Chromosom 6 von Sus scrofa im Bereich zwischen 71,4 cM und 96,1 cM oder im Bereich von 0 cM; (c) auf Chromosom 3 von Sus scrofa im Bereich zwischen 12,4 cM und 19 cM oder im Bereich zwischen 98,2 cM und 102,2 cM; (d) auf Chromosom 7 von Sus scrofa im Bereich zwischen 58,5 cM und 59,7 cM; (e) auf Chromosom 15 von Sus scrofa im Bereich zwischen 13,8 cM und 25,7 cM; oder (f) in dem chromosomalen Bereich eines anderen Säugers, der zu den in (a) bis (e) genannten Bereichen ortholog ist; wobei die erste Nukleinsäure in dem oben genannten Bereich lokalisiert ist oder 5 Megabasen stromaufwärts und/oder stromabwärts hiervon.

Die oben offenbarte zweite Nukleinsäure ist vorzugsweise genomische DNA oder cDNA, kann aber auch ein RNA Transkript dieser DNA sein. Genomische DNA und cDNA sind meist doppelsträngig, die erfindungsgemäße Verwendung umfasst aber auch einzelsträngige DNA Moleküle. Die erste Nukleinsäure ist bevorzugt ein Oligonukleotid, kann aber in bestimmten Ausführungen auch ein Polynukleotid sein. Sie ist bevorzugt DNA, kann aber auch RNA oder ein DNA- oder RNA-Derivat wie z.B. PNA sein. Als Primer in PCR- oder Sequenzierreaktionen hat die genannte erste Nukleinsäure üblicherweise eine Länge von mindestens 8 Nukleotiden, bevorzugt mindestens 15 Nukleotiden, stärker bevorzugt mindestens 18 Nukleotiden, noch stärker bevorzugt mindestens 21 Nukleotiden, am stärksten bevorzugt mindestens 25 Nukleotiden. Bei einer Verwendung als Hybridisierungssonde z.B. im Southern Blot kann die erste Nukleinsäure aber auch bis zu 50 Nukleotide, stärker bevorzugt bis zu 100 Nukleotide, noch stärker bevorzugt bis zu 1000 Nukleotide und am meisten bevorzugt bis zu 5000 Nukleotide lang oder länger sein. In manchen Fällen umfasst die erste oder zweite Nukleinsäure ganze Gene oder sogar Gruppen von Genen. In diesen Fällen hat die erste oder zweite Nukleinsäure eine Länge von bis zu 1000 Nukleotiden, vorzugsweise bis zu 5000 Nukleotide, beispielsweise bis zu 25000 Nukleotide, wie bis zu 150000 Nukleotide.

Als Hybridisierungssonde sind solche Nukleinsäuren zu verstehen, die in einer Hybridisierung verwendet werden und hierbei an homologe Nukleinsäuren binden. Vorzugsweise ist die Hybridisierungssonde eine radioaktiv-markierte Nukleinsäure oder sie enthält modifizierte Nukleotide. Die Erfindung umfasst auch solche Abwandlungen der vorliegend beanspruchten Nukleinsäuren, Hybridisierungssonden und Primer, die mit den zweiten Nukleinsäuren, bevorzugt unter stringenten Bedingungen, hybridisieren. Unter höher- oder hochstringenten Hybridisierungsbedingungen werden im Sinne dieser Erfindung beispielsweise 0,2-0,5 × SSC (0,03 M NaCl, 0,003M Natriumcitrat, pH 7) bei 65°C verstanden. Bei kürzeren Fragmenten, beispielsweise Nukleinsäuremoleküle aus bis zu 20 Nukleotiden, liegt die Hybridisierungstemperatur unter 65°C, beispielsweise bei über 55°C, bevorzugt über 50°C. Stringente Hybridisierungstemperaturen sind abhängig von der Größe bzw. Länge der Nukleinsäure und ihrer Nukleotidzusammensetzung und sind vom Fachmann durch Handversuche zu ermitteln. Üblicherweise enthält die zur Hybridisierung verwendete Lösung ein Detergenz wie z.B. SDS in einer Konzentration von 0.1 % bis 0.5% und eine Sammlung unspezifischer Nukleinsäuren zur Absättigung unspezifischer Bindungsstellen. Die Grundprinzipien der Hybridisierung und die Anforderungen an eine Hybridisierungssonde sind dem Fachmann hinreichend bekannt. Beispielsweise sei hier auf Maniatis, et al. Molecular Cloning:

A laboratory manual, Cold Spring Harbor Press, New York, 1982 oder Hames und Higgins, Nucleic acid hybridisation: a practical approach, IRL Press, Oxford 1985 verwiesen.

Als Prädisposition" bezeichnet man das Vorhandensein einer Erbanlage, die gegebenenfalls in einer Vererbung der Erbanlage und/oder einer Ausprägung eines Merkmals resultieren kann. Unter dem Merkmal "Hernia inguinalis/ scrotalis" versteht der Fachmann den Leisten- bzw. Hodensackbruch. Inguinal- und Scrotalhernien entstehen durch einen abnorm weiten Leistenkanal. Als Leistenbruch bezeichnet man den Vorfall von Eingeweiden, meist Teilen des Dünndarms und des großen Netzes in den Leistenkanal. Von Hodensackbruch dagegen spricht man, wenn diese Eingeweide bis in den Hodensack herunter treten. Inguinal- und Scrotalhernien treten entweder einseitig oder beidseitig auf, beim Eber kommt es allerdings vermehrt zu linksseitiger Hernienbildung. Beide Bruchformen sind grundsätzlich gleichartige angeborene Defekte hereditärer Genese. Die Tiere sind durch einen unphysiologisch weiten Leistenkanal bereits seit der Geburt prädisponiert.

Bezogen auf die Kopplungskarte des US Meat and Animal Center (Rohrer et al., 1996 Genome Research 6: 371-391, http://www.genome.iastate.edu/ maps/index.html), die anhand von über die Geschlechter gemittelten Rekombinationsraten erstellt wurde, sind die Mikrosatellitenmarker SW2021 an Position 12,4 cM, SW2429 an Position 17,2 cM, SW833 an Position 17,3 cM, SW72 an Position 17,8 cM, SE51018 an Position 19 cM; und S0002 an Position 102,2 cM von Chromosom 3 gelegen. Weiterhin sind die Mikrosatellitenmarker MP35 auf Chromosom 6 an Position 0 cM; SW1067 an Position 71,4 cM, SNPs im Gen RYR1 an Position 75 cM, SW1376 an Position 76,5 cM; SW122 an Position 83,4 cM, SW617 an Position 86,5 cM, SWR987 an Position 86,6 cM, SW316 an Position 89,3 cM, und SW2505 an Position 90,2 cM von Chromosom 6 gelegen. SW472 findet sich an Position 58,9 cM von Chromosom 7. Der Mikrosatellitenmarker S0229 ist an Position 19,3 cM von Chromosom 12 gelegen, SW1307 an Position 40,2 cM, SNPs im Gen GH an Position 45 cM, SW874 an Position 64,7 cM, SW168 an Position 70,5 cM und S0090 an Position 80,2 cM von Chromosom 12. Schließlich ist der Mikrosatellitenmarker S0355 an Position 13,8 cM und SW919 an Position 25,7 cM von Chromosom 15 gelegen.

Da genetische Kartierungspositionen populationsabhängig sind, ist diese Positionsangabe Schwankungen unterworfen, die den Fachmann jedoch in Kenntnis der erfindungsgemäßen Lehre nicht davon abhalten dieselbe ohne weiteres umzusetzen. Der Bereich zwischen 19,3 cM und 85,05 cM auf Chromosom 12 bezeichnet eine für eine Population spezifische Position auf Chromosom 12 und umfasst DNA-Abschnitte 5 cM stromaufwärts und/oder stromabwärts der angegebenen Position, vorzugsweise bis zu 2.5 cM stromaufwärts und/oder stromabwärts, stärker bevorzugt bis zu 1,25 cM stromaufwärts und/oder stromabwärts der angegebenen Position auf dem Chromosom. Entsprechendes gilt für die unten genannten Mikrosatelliten. Ist der Bereich oder Mikrosatellit endständig, d.h. am Ende des Chromosoms gelegen, insbesondere weniger als 5 cM vom Ende entfernt, so kann der stromaufwärts oder stromabwärts gelegene Bereich auch kürzer als 1,25 cM sein. Die genannten Bereiche umfassen, außer wenn dies im Fall von endständigen Bereichen nicht möglich ist, flankierende Nukleotidsequenzen von bis zu 5 Megabasen stromaufwärts und/oder stromabwärts, vorzugsweise bis zu 2,5 Megabasen stromaufwärts und/oder stromabwärts, stärker bevorzugt 1,25 Megabasen stromaufwärts und/oder stromabwärts.

Die vergleichenden Genomkarten zwischen verschiedenen Spezies beruhen auf der Kartierung eines oder mehrer Loci im Genom der betreffenden Spezies. "Orthologe Bereiche" bezeichnet genomische Abschnitte in anderen Säugern die in verschiedenen Organismen vorkommen und eine hohe Übereinstimmung ihrer Basensequenz aufweisen. Diese Bereiche werden als Erbstücke eines gemeinsamen Vorfahrens betrachtet und sind durch Artenbildung entstanden. Diese orthologen Bereiche können regelmäßig über die Sequenzidentität identifiziert werden, da sie über die gesamte Sequenzlänge oder in spezifischen hierin gelegenen Genen oder an einem oder mehreren Loci oder Teilen davon mit mindestens 100 Nukleotiden Länge eine Sequenzidentität mit der oben offenbarten zweiten Nukleinsäure, von mindestens bevorzugt 40%, stärker bevorzugt mindestens 50%, noch stärker bevorzugt mindestens 75% und insbesondere bevorzugt mindestens 95% Sequenzidentität aufweisen. Die Sequenzidentität wird vorzugsweise durch die FASTA, BLAST (Basic Local Alignment Search Tool) oder Bestfit Algorithmen des GCG-Sequenzanalyseprogramms bestimmt (Wisconsin Sequence Analysis Package, Version 8 for Unix, Genetics Computer Group, University Research Park, 575 Madison, WI 53711). Bei der Verwendung von Bestfit werden die Parameter vorzugsweise so eingestellt, dass der prozentuale Anteil der Identität über die gesamte Länge der Referenzsequenz berechnet wird und Homologielücken ("gaps") von bis zu 5% der Gesamtzahl der Nukleotide erlaubt sind. Bei der Verwendung von Bestfit werden die sogenannten optionalen Parameter vorzugsweise bei ihren voreingestellten Werten belassen.

Die in den erfindungsgemäßen Ausführungsformen genannten Mikrosatellitenmarker sind schweinespezifisch, d.h. es gibt beispielsweise keinen Mikrosatelliten mit dem Namen SW2021 im humanen Genom. Es ist jedoch bekannt, dass Chromosomenabschnitte zwischen verschiedenen Säugerspezies stark konserviert sind, auch ist die Anordnung der Gene (Syntänien) bekannt. Unter http://www.toulouse.inra.fr/lgc/pig/compare/SSC.htm sind die bisher bekannten/ vermuteten Übereinstimmungen zwischen den Chromosomensegmenten zwischen Mensch und Schwein aufgeführt. Es bestehen folgende Homologien: Porcines Chromosom 3: Humanchromosomen 2, 7 und 16; Porcines Chromosom 6: Humanchromosomen 1, 4, 16, 18, 19 und 21; Porcines Chromosom 7: Humanchromosomen 6, 14, 15 und 19; Porcines Chromosom 12: Humanchromosom 17; Porcines Chromosom 15: Humanchromosomen 2, 4 und 8. Anhand dieser und anderer Homologien ist es für den Fachmann leicht möglich, auf Grundlage der vorliegenden Erfindung und in Kenntnis seines Fachwissens, orthologe Bereiche zu identifizieren, d.h. Marker zu identifizieren, die für die erfindungsgemäßen Nachweisverfahren bei anderen Säugern geeignet sind.

Der Begriff "im wesentlichen identisch" bedeutet im Sinne dieser Erfindung, dass beispielsweise in einem Bereich von 8 Nukleotiden 7 Nukleotide identisch sind. Allerdings schließt die Erfindung auch solche Ausführungsformen ein, bei denen 4, 5 oder 6 der 8 Nukleotide mit der entsprechenden Sequenz der zweiten Nukleinsäure identisch sind. Dabei kann die erste Nukleinsäure mit dem + Strang oder dem - Strang identisch oder im wesentlichen identisch sein.

Der Begriff "einer ersten Nukleinsäure" schließt im Sinne der Erfindung auch ein, dass mehr als eine (erste) Nukleinsäure in der erfindungsgemäßen Verwendung eingesetzt werden kann. Dies können zum Beispiel zwei, drei oder vier Nukleinsäuren sein. Ferner kann der Begriff "zweite Nukleinsäure" sowohl den + Strang wie auch den -Strang bedeuten. Sofern zwei erste Nukleinsäuren mit der zweiten Nukleinsäure identisch oder im wesentlichen identisch sind, kann die eine erste Nukleinsäure mit dem + Strang identisch oder im wesentlichen identisch sein während die andere ersten Nukleinsäure mit dem - Strang identisch oder im wesentlichen identisch sein kann. In diesem Fall ist bevorzugt, dass die "Ausrichtung" der ersten Nukleinsäure gegenläufig ist, was die Durchführung einer PCR ermöglicht.

Anomalien wie die der "Hernia inguinalis/scrotalis" können, je nach Population, einen deutlich negativen Einfluss auf das betriebsökonomische Ergebnis in der Tierzucht- und produktion haben, da sie in der Regel zum Ausschluss einer Zuchttier führen. Mit der Erfindung werden erstmals Nukleinsäuren bereitgestellt, die eine gezielte molekularbiologische Diagnosestellung der Prädisposition zur Ausprägung des Phänotyps "Hernia inguinalis/scrotalis" erlauben. Durch die hier offenbarte Erfindung kann der Zeitpunkt der Selektion deutlich nach vorne verlagert werden, so dass nicht mehr auf die phänotypische Ausprägung des Merkmals gewartet werden muss. Zusätzlich kann die Einführung von molekularbiologischen Markern eine deutliche Effizienzsteigerung des Selektionsprozesses bewirken. Dabei beinhaltet dieser Selektionsprozess die Bestimmung des Genotypen des Probanden/Säugers an einem oder mehreren Loci in einem Bereich der oben genannten zweiten Nukleinsäuren, bevorzugt in zwei Bereichen, mehr bevorzugt drei, moch mehr bevorzugt vier, stärker bevorzugt fünf, am stärksten bevorzugt in sechs Bereichen und die Bewertung eines Individuums als zur Zucht geeignet oder nicht geeignet unter Einbeziehung von Informationen über die Kopplungsphase zwischen dem genotypisierten Markerlocus und den für die Defektausprägung verantwortlichen Genen. Beispielsweise können sich die Genotypen von geeigneten und ungeeigneten Säugern durch eine unterschiedliche Kopienanzahl einer repetitiven Nukleotidsequenz innerhalb des betrachteten Mikrosatellitenlocus unterscheiden. Diese unterschiedliche Beschaffenheit der Nukleinsäure kann mit Hilfe von geeigneten Primern in einer PCR Reaktion dargestellt werden und schlägt sich in unterschiedlichen PCR-Produktgrößen und/oder unterschiedlichen Restriktionsfragmentlängen nieder. Die Tests werden üblicherweise an Gewebeproben des Säugers, an Eizellen, oder an Proben von Körperflüssigkeiten wie Sperma, Urin, Milch, Blut, Tränenflüssigkeit und weiteren Sekreten durchgeführt. Diese können dem Tier vor der Diagnose entnommen werden.

Selbstverständlich kann eine molekularbiologische Charakterisierung der Prädisposition zur Vererbung oder Ausprägung des Phänotyps "Hernia inguinalis/scrotalis" auch für den Menschen von besonderem Interesse sein: So ist vorstellbar, dass die Identifizierung einer Prädisposition zur Vererbung oder Ausprägung des Phänotyps "Hernia inguinalis/scrotalis" zu neuen therapeutischen Ansätzen gegen diesen Defekt führt.

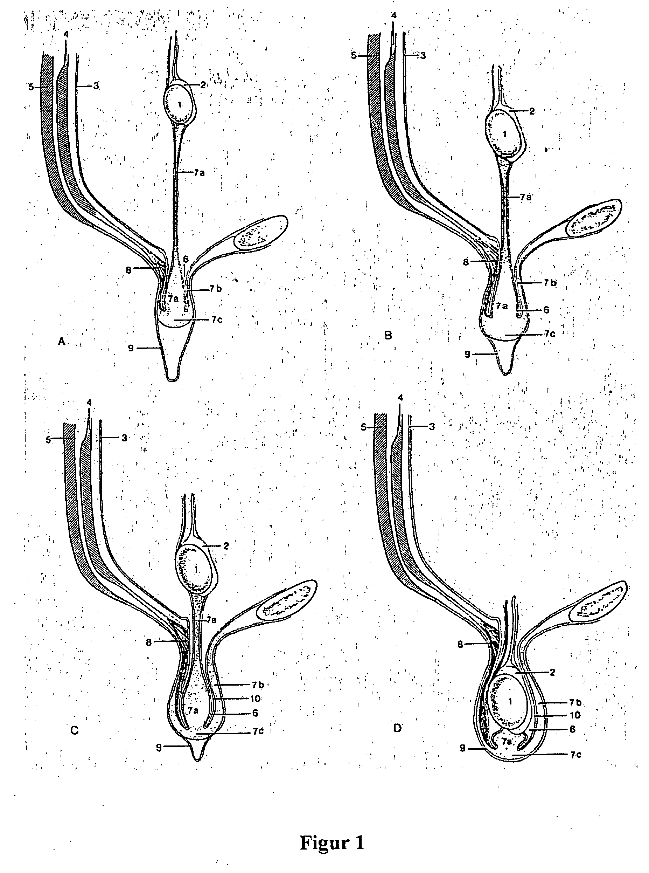

Während die Eierstöcke nur geringe Lageveränderungen innerhalb der Bauchhöhle während der Embryonalentwickelung durchführen, wandern die Hoden bei zahlreichen plazentalen Säugetieren aus ihrer ursprünglichen Lage an der dorsalen Wand der Bauchhöhle nach außen in den Hodensack (Skrotum) (Figur 1). Der Weg durch die Bauchhöhle führt durch den Leistenkanal, der vom äußeren und inneren Leistenring begrenzt wird.

Die Geschlechtsorgane entwickeln sich zunächst als indifferente Anlage. Medial an der Urnierenfalte entsteht die Keimdrüsenleiste, die sich durch Größenzunahme als Keimdrüsenfalte immer mehr von der Unterlage abhebt. Die Keimdrüsenanlage ist von beträchtlicher Länge und reicht vom Thorakal- bis zum Lumbalbereich. Nur der mittlere Abschnitt differenziert sich zur Keimdrüse. Aus dem kranialen Teil der Keimdrüsenanlage entsteht das kraniale Keimdrüsenband, aus dem kaudalen Teil das kaudale Keimdrüsenband. Das kaudale Keimdrüsenband erstreckt sich als Leistenband vom kaudalen Pol der Keimdrüse zum Leistenkanal (SCHNORR 1996). Die bisexuelle Entwicklung der zuvor indifferenten Gonadenanlagen beginnt mit der fetalen Entwicklungsphase der Frucht. Die Gonadenanlagen differenzieren sich zu den Ovarien oder den Hoden. Unter der Einwirkung von Testosteron kommt es zur Regression des kranialen Keimdrüsenbandes beim männlichen Tier, während das kaudale Keimdrüsenband zum Leitband des Hodens (Gubernaculum testis) wird. Der in den Leydigschen Zellen des Hodens produzierte Insulin Like Growth Factor 3 wirkt ebenfalls auf die Regression des kranialen Keimdrüsenbandes und auf die Entwicklung des Gubernaculum testis (ADHAM et al. 2000). Nach Untersuchungen an. "Knock-out"-Mäusen konnte gezeigt werden, dass auch die Gene HoxA10 und HoxA11 eine wichtige Rolle für die normale Entwicklung des Gubernaculum testis und den Hodenabstieg spielen (BRANFORD et al. 2000; SATOKATA et al. 1995).

Der Hodenabstieg ist ein mehrstufiger Prozess, der in der Literatur unter verschiedenen Gesichtspunkten in mehrere Phasen untergliedert wird. Die einfachste Unterteilung des Hodenabstiegs ist die in eine innere und äußere Phase der Wanderung. Die innere Phase umfasst die Wanderung der Hoden durch die Bauchhöhle bis zum Inguinalring, die äußere beschreibt den Durchtritt der Hoden durch den Leistenkanal und die Wanderung innerhalb des Skrotums (HUTSON 1985).

Am Gubernaculum testis lassen sich drei Bereiche unterscheiden: der abdominale, der intestinale und der subkutane Abschnitt. Zunächst besteht keine direkte Verbindung zwischen dem abdominalen Teil des Gubernaculum testis und den sich entwickelnden Hoden. Da das Gubernaculum testis mit dem Mesenchym der Geschlechtsgänge verwachsen ist und die Hoden auf der gegenüberliegenden dorsalen Seite des Mesenchyms mit den Geschlechtsgängen verbunden sind, ist jedoch eine indirekte Verbindung vorhanden. An der Stelle der Insertion des Gubernaculum testis in das Bauchfell entsteht der Scheidenhautfortsatz (Processus vaginalis). Während des Hodenabstiegs zeigen sowohl das Gubernaculum testis wie a uch der Processus vaginalis ein enormes Wachstum. Besonders stark erweitert sich das Gubernaculum testis am inneren Inguinalring.

Die Hoden gleiten über die Geschlechtsgänge hinweg und kommen damit in direkten Kontakt mit dem abdominalen Teil des Gubernaculum testis. Das Hodenleitband beginnt sich zu verkürzen und schafft damit Raum für die weitere Wanderung der Hoden in Richtung Leistenkanal. Das Gubernaculum testis schwillt weiter an und hat schließlich den gleichen Durchmesser wie der Hoden, mit dem es mittlerweile fest verbunden ist. Der subkutane Teil des Gubernaculum testis reicht bis ins Skrotum, mit dem es allerdings keine feste Verbindung hat. Durch das Anschwellen des Gubernaculum testis wird der Inguinalkanal zwischen innerem und äußerem Inguinalring geweitet. Die Hoden liegen zu diesem Zeitpunkt kurz vor dem inneren Inguinalring. Schließlich passieren die Hoden den Leistenkanal. Dieser Durchtritt erfolgt beim Schwein ab dem 90. Tag der Trächtigkeit.

Schon während des Durchtritts beginnt das Gubernaculum testis zu schrumpfen. Die Hoden setzen ihre Wanderung nun innerhalb des Skrotums fort. Das Gubernaculum testis bildet sich weiterhin rasch zurück. Erst im erwachsenen Tier haben die Hoden ihre endgültige Position eingenommen. Sie sind von der inneren Rumpffaszie und dem parietalen Wandblatt des Bauchfells eng umschlossen. Zwischen dem visceralen und dem parietalen Wandblatt des Bauchfells sind die Überreste des Gubernaculum testis als so genanntes Hodenbändchen zu finden (BARTECZKO u. JACOB 2000).

Hernien werden wahrscheinlich oligogen vererbt, das heißt, dass nicht ein einziger Defekt in einem Gen sondern mehrere Mutationen verschiedener Gene für das Auftreten von Leisten- und Hodensackbrüchen verantwortlich sind. Bisher konnte jedoch der Vererbungsmodus dieses Defekts weder beim Menschen noch beim Schwein zweifelsfrei geklärt werden und wird in der Literatur kontrovers diskutiert (Tabelle 1). Außerdem handelt es sich um eine oligofaktoriell bedingte Anomalie, neben der genetisch bedingten Prädispositon bewirken auch Umwelteffekte, wie zum Beispiel die Fütterung und dadurch bedingte übermäßig gefüllte Därme oder auch die Art der Tierhaltung, die Ausbildung von Hodensack- und Leistenbrüchen.

Tabelle 1: Erbgangshypothesen für Brüche beim Schwein

| These | Autoren |
|---|---|
| monofaktoriell, unvollständig dominant | BERGE 1941 |
| difaktoriell rezessiv | SITTMANN 1973 HUTSON et al. 1978 |
| difaktoriell rezessiv, geschlechtsbegrenzt | WARWICK 1926 |
| difaktoriell rezessiv oder polyfaktoriell | OLLIVIER u. SELLIER 1982 |
| polyfaktoriell, quantitativer Erbgang | MIKAMI u. FREDEEN 1979 KNAP 1986 MAGEE 1951 ALTHOFF 1985 ALTHOFF et al. 1988 WRATHALL 1988 |
| gemischter Erbgang mit einem Hauptgenort und Polygenen | THALLER 1992 |

Schon 1926 wurde die Erblichkeit von Hodensackbrüchen von Warwick nachgewiesen, der durch gezielte Anpaarungen unter den Nachkommen zweier Eber das Vorkommen von Bruchferkeln von 7,5 % auf 45,9 % in der $F_2$-Generation steigern konnte (WARWICK 1926).

Althoff ermittelte bei Tieren der Rasse DL (Deutsche Landrasse) nach der *Liability-Methode* Heritabilitäten ($h^2$) für Brüche zwischen 0,21 und 0,46. Weitere Untersuchungen mit gemischten oder hierarchischen Modellen der Varianzanalyse ergaben niedrigere Schätzwerte von $h^2$ = 0,022 bis 0,266 (ALTHOFF 1985). Heritabilitätsschätzungen für Hernien von Falkenberg und Kollegen bei DL-Schweinen belaufen sich auf einen Wert von $h^2$ = 0,018 (FALKENBERG

et al. 1991). Für Hodensackbrüche konnte Lui Heritabilitäten zwischen $h^2$ = 0,07 und 0,11 ermitteln (LUI 1991). Untersuchungen von Mikami und Fredeen zu Hodensackbrüchen ergaben Heritabilitäten von $h^2$ = 0,65 und $h^2$ = 0,86 (MIKAMI u. FREDEEN 1979). Ähnlich hohe Heritabilitäten konnten Thaller und Kollegen finden, allerdings reduzierten sich diese Schätzwerte unter Berücksichtigung der wurfspezifischen Umwelteffekte auf Werte zwischen $h^2$ = 0,029 und 0,187 (THALLER et al. 1996).

Neuere Untersuchungen zur Vererbung von Hodensackbrüchen liegen von den Schweinerassen Deutsche Landrasse und Pietrain in Bayern vor (BEISSNER et al. 2003a). Bei den Nachkommen von Ebern der Deutschen Landrasse wurden Heriteibilitäten von $h^2$ = 0,324 $\pm$ 0,166 und bei den Nachkommen von Pietrain-Ebern Heritabilitäten von $h^2$ = 0,462 $\pm$ 0,100 durch Vater-Sohn-Regressionen ermittelt (BEISSNER et al. 2003b). Mittels Tiermodellen wurden außerdem univariat und multivariat geschätzte Heritabilitäten bestimmt. Die Ergebnisse, unter Verwendung der Tiermodelle, stimmten bei beiden Rassen mit den durch Vater-Sohn-Regressionen ermittelten Werten überein (BEISSNER et al. 2003c).

Die Häufigkeit verschiedener Bruchformen (inguinalis, scrotalis und umbilicalis) schwankt nach Untersuchungen an mehreren Rassen zwischen 0,51 und 2,59 %, wobei große rassenabhängige Unterschiede auftreten (Tabelle 2).

Tab. 2: Frequenzen verschiedener Bruchformen in Prozent der lebendgeborenen Ferkel in der Bundesrepublik Deutschland (BRD) und der ehemaligen Deutschen Demokratischen Republik (DDR) (SAMUELS 1993).

| Frequenz (%) | Land | Rasse[1] | Autoren |
|---|---|---|---|
| Brüche allgemein | | | |
| 1,00 | BRD | NN | AVERDUNK et al. 1979 |
| 1,20 | BRD | BHZP | GLODEK 1977 |
| 1,20 | BRD | DL | LUI 1991 |
| 1,23 | BRD | DL | ALTHOFF 1985 |
| 1,60 | DDR | NN | WIESNER u. WILLER 1974 |
| Hodensackbruch | | | |
| 0,3-0,58 | BRD | div. | STIGLER 1990 |
| 0,57 | BRD | DL, PI | THALLER u. DEMPFLE 1992 |
| 0,73 | DDR | NN | TRIEBLER et al. 1974 |
| 0,88 | DDR | NN | SCHWARK et al. 1970 |
| 1,00 | BRD | NN | MATZKE u. AVERDUNK 1979 |
| Nabelbruch | | | |
| 0,05-0,07 | BRD | div. | STIGLER 1990 |
| 0,07 | BRD | DL, PI | THALLER u. DEMPFLE 1992 |
| 0,09 | BRD | PI, DL | FOERSTER 1985 |
| 0,20 | BRD | NN | WEGENER 1978 |
| 0,20 | BRD | NN | TRIEBLER et al. 1974 |
| Nabel- und Leistenbruch | | | |
| 0,11 | DDR | NN | SCHWARK et al. 1970 |

* **NN** nicht bekannt, **BHZP** Bundeshybridzucht-Programm, **DL** Deutsche Landrasse, **PI** Pietrain, **div.** verschiedene Rassen.

Eukaryontische Genome enthalten ca. 30-50 % repetitive Sequenzen (LANDER et al. 2001; MAKALOWSKI 2001). Diese repetitiven Sequenzen können aufgrund der Sequenzanordnung in direkte Wiederholungen und eingestreute repetitive Sequenzen unterteilt werden. Liegen direkte Wiederholungen ohne Unterbrechungen hintereinander, spricht man auch von Tandemwiederholungen. Je nach Länge der Wiederholungseinheiten werden Tandemwiederholungen in Satelliten, Minisatelliten und Mikrosatelliten eingeteilt. Mikrosatelliten bestehen aus monotonen Wiederholungen sehr kurzer Sequenzmotive von maximal 10 bp, während die Sequenzwiederholungseinheiten der Minisatelliten 10-50 bp und die der Satelliten mehrere kb lang sind. Die Sequenzmotive der Mikrosatelliten sind ca. 10 50mal tandemartig wiederholt.

In der Literatur werden für Mikrosatelliten auch andere Namen verwandt, wie z.B. Sequence-Tagged Microsatellite Sites (STMS; SCHÄFER et al. 1988), Simple Sequences (TAUTZ u. RENZ 1984), Short Sequence Repeats (SSR; EDWARDS et al. 1991) und Simple Sequence Length Polymorphism (SSLP; TAUTZ 1989).

Im Säugergenom sind pro Mb 238 Mikrosatelliten zu erwarten, d.h. alle 4-5 kb kommt ein Mikrosatellit vor (ZANE et al. 2002). Beim Schwein scheinen Tandemrepeats lediglich in den telomeren und centromeren Regionen der Chromosomen unterrepräsentiert zu sein (WINTERØ et al. 1992). Anhand der Struktur der Sequenzmotive kann man Mikrosatelliten in perfekte Mikrosatelliten, nicht perfekte Mikrosatelliten und Compound-Mikrosatelliten unterteilen (WEBER 1990; JOHANSSON et al. 1992; WINTER∅ et al. 1992; COPPIETERS et al. 1993; FREDHOLM et al. 1993):

- Perfekte Mikrosatelliten: ein Sequenzmotiv ohne Unterbrechungen und Umkehrungen, z.B. (CA)19;
- Nicht perfekte Mikrosatelliten: ein Sequenzmotiv mit Unterbrechungen und Umkehrungen, z.B. (CA)11GT(CA)4;
- Compound-Mikrosatelliten: mehrere Sequenzmotive mit bzw. ohne Unterbrechungen oder Umkehrungen, z.B. (AC)16(TC)10 oder (CA)9A(AC)19A(GA)7;

Weiterhin lassen sich Mikrosatelliten aufgrund der Länge ihrer Wiederholungseinheiten in Di-, Tri-, und Tetranukleotidmotive sowie größere Einheiten unterscheiden (VOGT 1990). Mikrosatellitensequenzen sind fast ausschließlich in nicht-kodierenden Regionen des Genoms lokalisiert (EDWARDS et al. 1991; STALLINGS et al. 1991). Es gibt aber auch Mikrosatelliten, die in Exons vorkommen, also in kodierenden Bereichen (MOORE et al. 1991; RIGGINS et al. 1992; BOLT et al. 1993; MOORE 1993; MORAN 1993; ROHRER et al. 1994a). Nicht alle Motive treten im Genom einer Spezies auf und auch die Zahl einzelner Mikrosatellitenmotive ist je nach Spezies sehr unterschiedlich (TAUTZ u. RENZ 1984; WONG et al. 1986; ELLEGREN et al. 1993b; MORAN 1993). Beim Schwein sind (A)n/(T)n-Wiederholungen am häufigsten, durchschnittlich alle 7 kb kommt ein (A)n/(T)n-Motiv im haploiden Genom vor (ELLEGREN et al. 1993b). Mikrosatelliten weisen hohe Mutationsraten auf, die beim Schwein bei 7'10-5 pro Locus und Gamete liegen (ELLEGREN 1995).

Drei Arbeitsgruppen beschrieben 1989, dass die Anzahl der Repeat-Einheiten innerhalb eines Mikrosatellitenlocus bei verschiedenen Individuen variiert (LITT u. LUTY 1989; WEBER u. MAY 1989; TAUTZ 1989). Diese Längenpolymorphismen werden wie codominante Merkmale nach den Mendelschen Regeln vererbt, daher sind Mikrosatelliten als genetische Marker geeignet. Die Markerallele unterscheiden sich in der Regel durch eine Repeat-Einheit, z.B. bei einem Dinucleotid-Repeat um 2 bp. Der Polymorphiegrad, d.h. die Anzahl an Allelen eines Mikrosatellitenlocus wird von zwei Faktoren beeinflusst, nämlich von der Zahl der Wiederholungseinheiten und von der Struktur der Sequenzmotive der Mikrosatelliten. Es besteht eine positive Korrelation zwischen dem Polymorphiegrad und der Zahl der Wiederholungseinheiten (WEBER 1990; ELLEGREN et al. 1992; JOHANSSON et al. 1992; WINTERØ et al. 1992; BUCHANAN et al. 1993), während die Länge der Wiederholungseinheiten keinen Einfluss auf den Polymorphiegrad der Mikrosatelliten zu haben scheint (EDWARDS et al. 1991; SERIKAWA et al. 1992). Perfekte Mikrosatelliten sind normalerweise polymorpher als nicht perfekte und Compound-Mikrosatelliten (WEBER 1990; ELLEGREN et al. 1992; WINTER∅ et al. 1992; FREDHOLM et al. 1993).

Die biologische Funktion der Mikrosatelliten ist nicht vollständig geklärt. Manche Mikrosatelliten besitzen eine transkriptionale Enhancer-Aktivität, was darauf hinweist, dass sie auf die Genregulation wirken können (NAYLOR u. CLARK 1990). Untersuchungen haben weiterhin gezeigt, dass Mikrosatelliten als "Hot-Spots" für Rekombinationsvorgänge angesehen werden (GROSS et al. 1985; FREUND et al. 1989; WAHLS et al. 1990a; WAHLS et al. 1990b) und dass sie möglicherweise währe nd der Bildung der Z-DNA-Struktur die Expression der Gene beeinflussen (HAMADA et al. 1984). Außerdem sind Mikrosatelliten wahrscheinlich an der Replikation beteiligt, wobei sie als Replikationsstart- und Terminationspunkte dienen (WALMSLEY et al. 1983; LAPIDOT et al. 1989). Weiterhin spielen Mikrosatelliten eine Rolle bei der Kondensierung der Chromosomen während der Meiose und Mitose (GROSS u. GARRARD 1986).

Im Wesentlichen werden zwei Theorien zur Entstehung von Mikrosatelliten diskutiert. Die erste Theorie besagt, dass das Auftreten von ungleichmäßigen Crossing over-Ereignissen zwischen homologen DNA-Abschnitten zur Entstehung sich wiederholender Sequenz-Abschnitte im Genom führen kann (SMITH 1976; LEVINSON u. GUTMAN 1987a; BULLOCK et al. 1986). Zweitens könnte auch eine ungenaue Replikation (der sogenannte "Slippage-Effekt") die Entstehung von Mikrosatelliten verursachen (LEVINSON u. GUTMAN 1987b; MORRAL et al. 1991; HARDING et al. 1992; MAHTANI u. WILLARD 1993). Die Synthese repetitiver Einheiten durch Slippage wurde in vitro nachvollzogen (SCHLOETTERER u. TAUTZ 1992).

Über die Darstellung von Multilocus-Mikrosatelliten beim Schwein wurde das erste Mal 1991 berichtet (BUITKAMP et al. 1991; EPPLEN et al. 1991). Es konnten polymorphe Bandenmuster erzeugt werden mit Hilfe der Oligonukleotidsonden (AT)8, (CA)8/(GT)8, (C)5/(GTG)8, (GAA)6/(CTT)6, (TCC)5/(GGAT)4 und (GACA)4 (BUITKAMP et al. 1991; EPPLEN et al. 1991; CHEN 1994). Wegen des hohen Polymorphiegrads und der großen Anzahl im Genom sind Mikrosatelliten geeignete DNA-Marker für die Genkartierung. Nach Einführung der PCR können Mikrosatelliten (MULLIS et al. 1986; SAIKI et al. 1988) nach anschließender gelelektrophoretischer Auftrennung analysiert werden. Aus den flankierenden Sequenzen der Mikrosatellitenloci werden hierzu kurze Sequenzabschnitte (Primer) definiert und in vitro synthetisiert sowie mit einem Isotop oder mit Fluoreszenzfarbstoffen markiert. Anschließend werden dann die Sequenzen des Mikrosatellitenlocus mit Hilfe der PCR amplifiziert und die PCR-Produkte in einem hochauflösenden Polyacrylamidgel oder einem Hydrolinkgel aufgetrennt. Aufgrund der Fragmentlängen der PCR-Produkte kann man auf die Zahl der tandemartig wiede rholten Nukleotidrepeats schließen (z.B. LOVE et al. 1990).

Seit der Einführung von DNA-Sequenzierungsautomaten wird die Fragmentanalyse meist mit Hilfe fluoreszenzmarkierter Oligonukleotide (VOSS et al. 1989) und der lasergestützten Detektion der emittierenden Fluoreszenz in denaturierenden Polyacrylamidgelen durchgeführt (ANSORGE et al. 1986; CARRANO et al. 1989; VOSS et al. 1992).

Nachdem eine Vielzahl von diesen Markern für eine Spezies zur Verfügung steht, können Sets von mehreren Mikrosatellitensequenzen innerhalb desselben Reaktionsansatzes amplifiziert werden. Dieses Verfahren wird als Multiplex-PCR (CHAMBERLAIN et al. 1990) bezeichnet. Sind die Oligonukleotide dabei mit unterschiedlichen Farbstoffen fluoreszenzmarkiert, können sich die Mikrosatellitensequenzen in ihren Fragmentlängen auch überlappen, was die Zahl der Mikrosatelliten, die in einer Reaktion analysiert werden können, erhöht. Damit stehen automatisierte Techniken von der Genotypisierung der Mikrosatelliten (ZIEGLE et al. 1992) bis zur Dateninterpretation (KOBAYASHI et al. 1995) für die Genkartierung zur Verfügung. Mittels solcher leistungsfähiger Hilfsmittel kann eine gezielte Suche nach bestimmten Genen in einem Untersuchungsmaterial durchgeführt werden. Dieser Ansatz zur Genidentifizierung wird als positionelle Klonierung bezeichnet (COLLINS 1992). Zunächst werden geeignete DNA-Proben von Familien gesammelt, in denen das Merkmal, welches durch das Gen bzw. die Gene beeinflusst wird, auftritt. Diese Familien werden in einem Genomscan mit einer Vielzahl von polymorphen Markern, die möglichst gleichmäßig alle Chromosomen abdecken, analysiert, um eine mögliche Kopplung mit einem oder mehreren Markern zu dem Merkmal nachzuweisen (Grobkartierung). Eine Feinkartierung der identifizierten Region erfolgt zumeist mittels zusätzlicher Marker und Meiosen. Eng an die Marker gekoppelte Gene können anhand komparativer Genomkarten identifiziert werden. Unter Einbeziehung von Informationen zur Funktion der Genprodukte, kann der Kreis der Kandidatengene weiter eingegrenzt werden (positionell-funktioneller Ansatz).

Für eine sichere Bestimmung der Fragmentlängen von Mikrosatelliten ist es vorteilhaft, wenn sich die Fragmente, die die jeweiligen Allele repräsentieren, in mehr als einem bp unterscheiden. Analysen von Mikrosatelliten aus Tri- oder Tetranukleotidrepeats sind am effektivsten. Sie besitzen gegenüber Dinukleotidrepeats den Vorteil, dass Artefakte durch Slippage der Taq-Polymerase während der PCR (FOUCAULT et al. 1996), die auch als Schattenbanden bezeichnet werden (HAUGE u. LITT 1993), seltener vorkommen. Dies erleichtert die Auswertung und reduziert damit Fehler bei der Genotypisierung (DUBOVSKY et al. 1995). Der Polymorphiegrad eines Mikrosatelliten kann zwischen Rassen und auch Individuen einer Rasse stark variieren. So kann es vorkommen, dass bei einigen Rassen bzw. bei den untersuchten Tieren der Mikrosatellit monomorph ist, während er bei anderen Tieren mehrere Allele aufweist. Dies hat zur Folge, dass bestimmte Mikrosatelliten in manchen Populationen nicht informativ sind. Daher empfiehlt es sich beispielsweise, die ausgesuchten Mikrosatelliten für den Genomscan zunächst an gepoolten DNA-Proben der Elterntiere auf ihren Informationsgrad hin zu, überprüfen. Bei der Darstellung der Mikrosatelliten zeigt sich auch, dass bei manchen Loci ein Allel nicht amplifiziert wird. Nicht-amplifizierte Allele werden als "Null-Allel" bezeichnet (CALLEN et al. 1993). Der Grund dafür ist zumeist eine Mutation innerhalb der Primerbindestellen (CALLEN et al. 1993; EDE u. CRAWFORD 1995). Bei Familienuntersuchungen wurde festgestellt, dass bei ca. 6,9 % bis 9,5 % der porcinen Mikrosatellitenloci "Null-Allele" vorkommen (COPPIETERS et al. 1993; ROHRER et al. 1994b). Dies kann zur Detektion Falsch-Homozygoter führen.

In dem frei zugänglichen Datenbanksystem des Roslin Institutes (Edinburgh) waren im Mai 2004 rund 1600 porcine Mikrosatelliten verzeichnet (http://iowa.thearkdb.org/browser). Die meisten Mikrosatellitenloci wurden aus DNA-Bibliotheken beim Schwein isoliert und charakterisiert. Durch in silico Analysen gelangen es aber auch aus den Datenbankeinträgen in EMBL und GENBANK porcine Mikrosatelliten darzustellen (MORAN 1993; ELLEGREN et al. 1993a; ELLEGREN et al. 1993b). Mit Hilfe der Durchflusscytometrie und der PARM-PCR (Priming Authoring Random Mismatches Polymerase Chain Reaction) gelang es gezielt Mikrosatelliten auf bestimmten Chromosom zu isolieren (DAVIES et al. 1992; HØYHEIM et al. 1992; MILLER et al. 1992; NAGEL et al. 1993; DEAR u. MILLER 1994; DAVIES et al. 1994; NAGEL et al. 1996). Aus spezifischen Chrom osomenregionen wurden ebenfalls Mikrosatelliten mit Hilfe der Mikrodissektion isoliert und kartiert (AMBADY et al. 1997). Kürzlich gelang es ebenso, Mikrosatelliten durch direkte Sequenzierung von Klonen aus sogenannten large-insert-libraries zu entwickeln (FUJISHIMA-KANAYA et al. 2003; CHEN et al. 2004).

Für die genetische Kartierung beim Schwein wurden spezielle Familien als Referenzfamilien erstellt (ARCHIBALD et al. 1991; ROHRER et al. 1994b). Es handelt sich hierbei um F2-Generationen oder Rückkreuzungsgenerationen. In den Großeltern-Generationen wurden genetisch verschiedene Rassen gekreuzt, um möglichst heterozygote Individuen in den F1-Generationen zu erzeugen. Beispielsweise umfassen die Referenzfamilien des PiGMaP-Programms fünf Familien, die jeweils aus drei Generationen mit insgesamt 164 Tieren bestehen (ARCHIBALD et al. 1995), während die Referenzfamilie des USDA-Programms aus Rückkreuzungsfamilien mit 104 Schweinen besteht (ROHRER et al. 1994b).

Es gibt drei Kopplungskarten für das Schwein, nämlich die PiGMaP-Karte (ARCHIBALD et al. 1995), die USDA-Karte (ROHRER et al. 1996) und die Karte des Nordic-Verbundes (MARKLUND et al. 1996). Die meisten DNA-Marker, die auf den Kopplungskarten platziert wurden, sind Mikrosatelliten des TG-Typs (GROENEN et al. 1995). Bis 1996 waren erst 10 % aller porcinen Mikrosatelliten mehr als einer genetischen Karte zugeordnet (ROHRER et al. 1996), so dass die drei existierenden Genkarten (Karten des PIGMap- USDA- und Nordic-Verbundes) nicht deckungsgleich waren. Diese Karten werden inzwischen zusammengeführt (z.B. ZHANG et al. 1995), um die zur Verfügung stehenden Kartierungswerkzeuge möglichst effizient nutzen zu können (BUETOW et al. 1994). Zusätzlich entstand 1996 durch Genotypisierung der gebräuchlichsten Marker in der USDA-Referenzfamilie eine erweiterte, integrierte genetische und cytogenetische Karte (ROHRER et al. 1996). Außerdem wurden zahlreiche Marker in der skandinavischen Familie neu genotypisiert (MARKLUND et al. 1996). Die geschlechtsneutrale porcine Genkarte ist mit 25 M (MARKLUND et al. 1996; ROHRER et al. 1996) verglichen mit 38 M für den Menschen bemerkenswert kurz, obwohl beide Genome dieselbe Menge an DNA enthalten (MORTON 1991). Dies verdeutlicht, dass die Länge von Kopplungskarten nicht allein von der physikalischen Größe eines Genoms beeinflusst wird (ARCHIBALD u. HALEY 1998), sondern auch von zahlreichen anderen Faktoren (wie z.B. Alter und Geschlecht eines Individuums). Die porcine weibliche Karte ist gemessen in cM-Einheiten um den Faktor 1,3 größer als die männliche Karte (ARCHIBALD et al. 1995; MARKLUND et al. 1996).

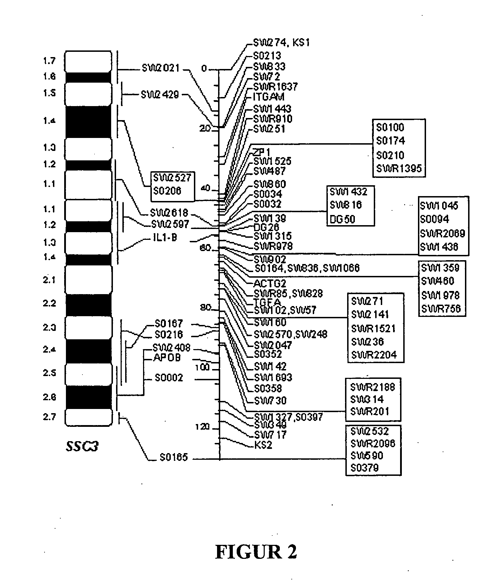

Den Genkarten kommt eine besondere Bedeutung für genomweite DNA-Marker-Analysen zu, da üblicherweise die beschriebenen Abstände zwischen den Markern als Auswahlkriterien genutzt werden. Es zeigte sich, dass die Abstände in verschiedenen Familien unterschiedlich sein können, aber selten die Anordnung der Marker (z.B. GELDERMANN et al. 2003). Figur 2 zeigt beispielhaft die aktuelle Kopplungskarte des porcinen Chromosoms 3 (Stand: Juli 2004).

Mit einer Kopplungsanalyse wird die These überprüft, ob eine gemeinsame Vererbung der Allele zweier Loci wahrscheinlicher ist als eine unabhängige Vererbung (MORTON 1955). Die Grundlage der Kopplungsanalyse ist das Auftreten von Rekombinationsereignissen. Durch Aneinanderlagern homologer Chromosomen bei der Meiose kann es zum Crossing-over kommen. Der Austausch von Chromosomenabschnitten führt zur Rekombination. Aufgrund der beobachteten Rekombinationshäufigkeiten zwischen zwei oder mehreren Genorten bei der Vererbung von Haplotypen innerhalb von Familien werden die Abstände zwischen den Genen oder genetischen Markern bestimmt. Als Haplotyp werden die Allele verschiedener gekoppelter Genorte bezeichnet, die gemeinsam vererbt werden. Die genetische Distanz zwischen zwei Loci ergibt sich dabei aus der Rekombinationsfrequenz und wird in centiMorgan (cM) angegeben. 1 cM entspricht einer Rekombinationsfrequenz von 1 %, also dem Auftreten von einem Crossing-over bezogen auf 100 Meiosen. Ein Abstand von 1 cM auf der genetischen Karte entspricht beim Säuger einem physikalischen Abstand von etwa 1 Mbp, wobei das Verhältnis von genetischem zu physikalischem Abstand (cM/Mbp) im Genom lokusabhängig variiert. Die Wahrscheinlichkeit, dass ein Rekombinationsereignis auftritt, ist umso größer, je weiter zwei Genorte auf einem Chromosom voneinander entfernt liegen. Mit Kartierungsfunktionen, z.B. nach Kosambi, kann die Rekombinationsfrequenz (q) in die genetische Distanz umgerechnet werden (OTT 1991). Bei freier Rekombination zwischen zwei Genorten beträgt die Rekombinationsfrequenz q = 0,5. Zwei Genorte werden umso häufiger gekoppelt vererbt, je geringer der Abstand zwischen ihnen auf einem Chromosom ist, dabei ist $0 \leq \theta < 0,5$. Ist die Rekombinationsfrequenz $\theta > 0,5$ werden die Genorte ungekoppelt vererbt, d.h. sie liegen auf unterschiedlichen Chromosomen, oder sehr weit entfernt auf einem Chromosom.

Bei der Kopplungsanalyse wird die Rekombinationsfrequenz $\theta$ am genotypisierten Familienmaterial geschätzt und getestet, wie groß die Wahrscheinlichkeit für Kopplung ist. Im Lod-Score-Test wird diese Wahrscheinlichkeit (likelihood, L) der Kopplung für bestimmte Rekombinationsraten ($\theta$), also Abstände zwischen den Loci, überprüft. Mit der Lod-Score Methode wird daher gleichzeitig die Rekombinationsrate $\theta$ geschätzt und geprüft, ob diese signifikant von $\theta$ = 0,5 verschieden ist. Die Signifikanzschwelle für den Lod-Score Z liegt für

$$Z(\theta) = \log_{10}(L(\theta) / L(\theta = 0,5))$$

bei $Z \geq 3$. Ein Lod-Score $\leq$ -2 schließt eine Kopplung aus (OTT 1991). Um die Vererbung zweier Loci beobachten zu können, wird ein umfangreiches Familienmaterial benötigt. Einzelne Familien werden separat ausgewertet und die Ergebnisse anschließend addiert (OTT 1991). Die Berechnung wird umso aussagekräftiger, je mehr Generationen, Nachkommen und unabhängige Familien analysiert werden können. Außerdem hat die Anzahl der Allele einen Einfluss. Je polymorpher die Loci sind, desto wahrscheinlicher ist ein Founder an beiden Orten heterozygot, so dass die Weitergabe der Allele (Segregation) an die Nachkommen verfolgt werden kann (FRIES 1993).

Bei der parametrischen Kopplungsanalyse wird ein definierter Vererbungsmodus vorausgesetzt. Es wird bei einem phänotypischen Merkmal, wie z.B. einem Erbdefekt, das auf Kopplung mit einem genetischen Marker getestet wird, neben rezessivem bzw. dominantem Modell mit oder ohne Einfluss des Geschlechtes zusätzlich die Penetranz des Merkmals vorgegeben. Im Gegensatz dazu steht die nichtparametrische Analyse ohne derartige Vorgaben.

Man kann im Allgemeinen zwischen der Singlepoint- und der Multipoint-Analyse unterscheiden. Während bei der Singlepoint-Analyse nur zwei Loci betrachtet werden und die Rekombinationsrate zwischen ihnen variiert, werden bei der Multipoint-Analyse mehrere Loci gleichzeitig berücksichtigt. Der Abstand der weiteren Loci bei der Multipoint-Analyse wird angegeben und bleibt fest, die Anordnung des ersten Locus wird variiert.

ES sind Programme entwickelt worden, die den Lod-Score berechnen. Einen Überblick über vorhandene Programme gibt Bryant (BRYANT 1997). Das Programm GENEHUNTER von Krugylak und Kollegen ermöglicht eine parametrische Multipoint-Analyse mit einer Vielzahl von Loci (KRUGLYAK et al. 1996). Eine Einschränkung liegt jedoch in der Größe der Familie, die gleichzeitig in die Berechnung einbezogen werden kann.

ALLEGRO ist ebenfalls ein Computerprogramm für die parametrische Multipoint-Analyse (GUDBJARTSSON et al. 2000). Das Programm ALLEGRO beruht auf dem Programm GENEHUNTER. Während GENEHUNTER ein Limit von 50 Markern und 16 Non-Founders hat, gibt es bei ALLEGRO weder ein Limit für die Anzahl der Marker, noch für die Anzahl der Non-Founders.

MLINK ist ein Programm zur parametrischen Singlepoint-Analyse aus dem Programmpaket Linkage 5.1 von Lathrop und Lalouel (LATHROP u. LALOUEL 1984). Weitere gängige Programme zur Kopplungsanalyse sind MENDEL von Lange und Kollegen (LANGE et al. 1988) und CRI-MAP von Lander und Green (LANDER u. GREEN 1987). MENDEL benötigt besonders hohe Computerkapazitäten (OTT 1999), während CRI-MAP zur Berechnung von Kopplungskarten bei bekanntem Genotyp geeignet ist (BRYANT 1997). Ein neueres Programm zur Kopplungsanalyse ist MERLIN (ABECASIS et al. 2002).

Die nichtparametrische Kopplungsanalyse wird verwendet, wenn es nicht möglich ist, sich für einen Erbgang zu entscheiden, oder ein Untersuchungsmaterial eine parametrische Analyse nicht zulässt. Auch bei komplexen bzw. ungeklärten Erbgängen mit unterschiedlichen Penetranzen kann sie die vorhandenen Pedigreeinformationen zu einer Analyse nutzen.

GENE HUNTER ist das Programm der Wahl für die nichtparametrische Kopplungsanalyse (OTT 1999). In vergleichenden Studien konnte die Gruppe um Krugylak feststellen, dass die Mächtigkeit ihrer Analyse mit GENEHUNTER der einer Lod-Score-Analyse unter richtigem Modell annährend entsprach (KRUGLYAK et al. 1996). Neben dem Multipoint-Lod-Score berechnet das Programm GENEHUNTER einen NPL-Score (Non Parametric Lod-Wert). Das "identical by descent-sharing" wird geschätzt, d.h. die übereinstimmende Herkunft von Haplotypen unter Individuen einer Familie. Es wird dabei beobachtet, ob betroffene Geschwister einen abstammungsidentischen Genomabschnitt geerbt haben, und es wird berechnet, ob bestimmte Allele häufiger vererbt wurden als es bei zufälliger Segregation zu erwarten wäre (KRUGLYAK et al. 1996). Die Irrtumswahrscheinlichkeit (p), die auf Schätzwerten beruht (perfect data approximation), wird außerdem bestimmt (KRUGLYAK et al. 1996). Dieser p-Wert wird umso exakter geschätzt, je höher der Informationsgehalt der Marker und der des Pedigree ist. Auskunft über die Qualität und Abdeckung der Marker erhält man durch die Bestimmung des prozentualen Informationsgehalts, gemessen an der höchstmöglichen Information des Pedigree (KRUGLYAK et al. 1996). Für, ein monogen bedingtes Merkmal liegt die Signifikanzschwelle, die einem Lod-Score von 3 entspricht, bei p = 0,0001 (CHOTAI 1984). Von suggestiver Kopplung wird ab p = 0,001 (Lod-Score = 2,1) gesprochen. Die Signifikanzschwelle für komplexe Merkmale liegt bei p = 0,00002 (Lod-Score = 3,6), und bei p = 0,0007 (Lod-Score = 2,2) beginnt suggestive Kopplung (LANDER u. KRUGLYAK 1995). Diese Schwellenwerte finden einen weiten Konsens (Editorial Nature Genetics 1 (3), 1995). Die Nachteile des Programms liegen in der begrenzten Familiengröße. Wenn der Informationsgehalt der Marker niedrig ist, berechnet GENEHUNTER den p-Wert zu konservativ, wie Studien von Kong und Cox ergaben (KONG u. COX 1997). Sie entwickelten daher zusammen mit Michael L. Frigge eine modifizierte Version des Programms, die in dieser Hinsicht verbessert wurde, GENEHUNTER-PLUS heißt und allele sharing LOD Scores berechnet.

Alternativ zu GENEHUNTER kann auch ALLEGRO für die nichtparametrische Kopplungsanalyse benutzt werden. ALLEGRO berechnet ebenfalls allele sharing LOD Scores, sowie außerdem in Anlehnung an GENEHUNTER NPL-Scores. Die Berechnungen von ALLEGRO basieren auf den gleichen Modellen wie bei GENEHUNTER, und das Programm benutzt die gleiche Fourier Transformation, um die Zusammenhänge zu berechnen (KRUGLYAK u. LANDER 1998). ALLEGRO hat gegenüber GENEHUNTER allerdings mehrere Vorteile. So war man bemüht, die Input- und Output-Files relativ flexibel zu gestalten. Weiterhin gibt es keine Begrenzung der Marker und der NON-Founders, die in den Berechnungen berücksichtigt werden können. Außerdem kann ALLEGRO mehrere Modelle in einem Rechenvorgang berücksichtigen. SIBPAL aus dem S.A.G.E.-Paket von Elston (ELSTON 1997) und das ANALYZE-Paket von Terwilliger sind andere Programme zur nichtparametrischen Kopplungsanalyse. Eine größere Zahl von Markern für die Multipoint-Analyse können von diesen Programmen nicht berücksichtigt werden (OTT 1999). Almasy und Blangero entwickelten ein weiteres Programm, SOLAR, das die gemeinsame Berechnung von umfangreichem Familienmaterial ermöglichen soll (ALMASY u. BLANGERO 1998). Auch das Programm MERLIN (ABECASIS et al. 2002) ist für die nichtparametrische Kopplungsanalyse geeignet.

Positive Ergebnisse aus der Kopplungsanalyse können mit Hilfe der Assoziationsanalyse bestätigt werden. Es wird getestet, ob zwischen bestimmten Allelen zweier Loci ein Kopplungsungleichgewicht herrscht. Während bei der Kopplungsanalyse die Allelweitergabe innerhalb von Familien beobachtet wird, erlaubt die Assoziationsanalyse, die Allelvererbung über die Familien hinweg mit der übrigen Population zu vergleichen (SCHAID 1995). Ob bei betroffenen Individuen eine signifikante Allelfrequenzabweichung eines genetischen Markers zu der übrigen Population vorliegt, kann im Falle eines Krankheitsmerkmals getestet werden (TERWILLIGER u. OTT 1994). Ein Kopplungsungleichgewicht ist nur zu erwarten, wenn sich der Marker in unmittelbarer Nähe des Krankheitslocus befindet und daher keine freie Rekombination gegeben ist. Wenn eine Population auf einen Founder zurückzuführen ist, ist das Auffinden eines Kopplungsungleichgewichts in dieser Population erleichtert (SCHWAB et al. 1998). Da das Ungleichgewicht jedoch auch über 100 Generationen Bestand haben kann (JORDE 1995), ist der Founder im Nachhinein nicht immer auszumachen.

Die Assoziationsanalyse bietet die Möglichkeit, die mit der Kopplungsanalyse bestinnmten Regionen für eine physikalische Kartierung weiter einzuschränken und hilft bei der Genidentifizierung und Gencharakterisierung (DEVLIN u. RISCH 1995; SCHWAB et al. 1998). Mit einem Chi-Quadrat-Test für Vierfeldertafeln kann auf Assoziation getestet werden (KREYSIG 1979). Zur Bestimmung der Referenz-Allelfrequenzen wurden ursprünglich zufällig ausgewählte Mitglieder der Population herangezogen. Die Auswahl der Referenzen ist jedoch nicht immer repräsentativ. Dieses Problem kann umgangen werden, indem man die nicht-weitergegebenen Allele nicht betroffener Vorfahren aus den betrachteten Familien heranzieht (FALK u. RUBINSTEIN 1987; FLANDERS u. KHOURY 1996). Beispiele für dieses Vorgehen sind der TDT- (transmission/disequilibrium test, SPIELMAN et al. 1993) und der HRR-Test (haplotype relative risk test, KNAPP et al. 1993). Mittels TDT-Test kann nur ein Kopplungsungleichgewicht nachgewiesen werden, wenn der Marker relativ nah am Krankheitslokus liegt, d.h. er sollte nicht weiter als 1 cM entfernt sein.

In einer bevorzugten Ausführungsform der Erfindung wird eine Kombinationen von mindestens zwei oder mehr der genannten ersten Nukleinsäuren zur Bestimmung der Ausprägung oder Vererbung des Phänotypus "Hernia inguinalis/scrotalis" verwendet. Besonders bevorzugt sind Kombinationen von drei, vier, fünf, sechs, sieben, acht oder neun der genannten ersten Nukleinsäuren zur Bestimmung der Prädisposition zur Ausprägung oder Vererbung des Phänotyps "Hernia inguinalis/scrotalis". Diese bevorzugte Ausführungsform ist besonders geeignet, die Zuverlässigkeit des Nachweises zu erhöhen.

In einer weiteren bevorzugten Ausführungsform der Erfindung ist die zweite Nukleinsäure ein SNP, ein Mikrosatellit oder eine den SNP oder Mikrosatelliten flankierende Sequenz, wobei die flankierende Sequenz einen Bereich von bis zu 5 Megabasen stromaufwärts und stromabwärts des Mikrosatelliten umfasst.. Der Begriff "SNP" bedeutet "single nucleotide polymorphism". Die flankierende Sequenz liegt typischerweise außerhalb der für den Mikrosatelliten charakteristischen repititiven Sequenzen und umfasst vorzugsweise einen Bereich von bis zu 5 Megabasen stromaufwärts und/oder stromabwärts, wobei flankierende Bereiche mit einer Länge von bis zu 4 Megabasen, bis zu 3 Megabasen, bis zu 2 Megabasen, bis zu 1,5 Megabasen und bis zu 1 Megabasen besonders bevorzugte Ausführungsformen der erfindungsgemäßen Verwendung darstellen.

In einer weiteren besonders bevorzugten Ausführungsform der erfindungsgemäßen Verwendung wird der Mikrosatellit oder SNP ausgewählt wird aus der Gruppe bestehend aus (a) den auf Chromosom 12 von Sus scrofa gelegenen Mikrosatelliten S0229 im Bereich von 19,3 cM, SW1307 im Bereich von 40,2 cM, ein SNP im Bereich des Gens GH im Bereich von 45 cM, SW874 im Bereich von 64,7 cM, SW168 im Bereich von 70,5 cM, S0090 im Bereich von 80,2 cM; (b) den auf Chromosom 6 von Sus scrofa gelegenen Mikrosatelliten MP35 im Bereich von 0 cM, SW1067 im Bereich von 71,4 cM, ein SNP im Bereich von 75 cM im Bereich des Gens RYR1, SW1376 im Bereich von 76,5 cM; SW122 im Bereich von 83,4 cM, SW617 im Bereich von 86,5 cM, SWR987 im Bereich von 86,6 cM, SW316 im Bereich von 89,3 cM, SW2505 im Bereich von 90,2 cM; (c) den auf Chromosom 3 von Sus scrofa gelegenen Mikrosatelliten SW2021 im Bereich von 12,4 cM, SW2429 im Bereich von 17,2 cM, SW833 im Bereich von 17,3 cM, SW72 im Bereich von 17,8 cM, SE51018 im Bereich von 19 cM; S0002 im Bereich von 102,2 cM; (d) dem auf Chromosom 7 von Sus scrofa gelegenen Mikrosatelliten SW472 im Bereich von 58,9 cM; und (e) den auf Chromosom 15 von Sus scrofa gelegenen Mikrosatelliten S0355 im Bereich von 13,8 cM, SW919 im Bereich von 25,7 cM.

In einer weiteren bevorzugten Ausführungsform der erfindungsgemäßen Verwendung erfolgt der Nachweis von zumindest einem spezifischen Allel. Unter dem Begriff "Allel" versteht der Fachmann eine Serie von zwei und mehr Varianten eines spezifischen chromosomalen Genabschnitts. Der Nachweis eines spezifischen, gemeinsam mit einem Merkmal vererbten Allels erlaubt eine Vorhersage, ob das Merkmal in dem untersuchten Individuum auftreten kann oder ob es ggf. an dessen Nachkommenschaft weitergegeben werden kann. Besonders bevorzugt sind jedoch erfindungsgemäße Verwendungen wobei zwei oder mehr Allele nachgewiesen werden. Typischerweise unterscheiden sich die einzelnen Allele von Mikrosatelliten durch ihre Länge. Dies erlaubt es beispielsweise, durch PCR Amplifikation mit geeigneten Primern, Mikrosatelliten oder Abschnitte von Mikrosatelliten zu amplifizieren und die Länge des Amplifikats einem bestimmten Allel zuzuordnen und somit das vorhandene Allel zu identifizieren.

In einer weiteren bevorzugten Ausführungsform der erfindungsgemäßen Verwendung ist die zweite Nukleinsäure von einem auf Chromosom 12 von Sus scrofa gelegenen Gen oder von einem orthologen Gen eines anderen Säugers abgeleitet.

Der Begriff "von einem anderen Säuger" bezieht sich im wesentlichen auf Säuger die nicht als Sus scrofa bezeichnet werden können, also beispielsweise auf Pferd, Rind, Mensch, Hund etc. "Von einem Gen abgeleitet" bedeutet, dass die Nukleinsäure vorzugsweise die genomische Nukleinsäure ist, aber weiterhin auch die hiervon transkribierten RNAs sowie insbesondere modifizierte RNAs (beispielsweise gespleiste Transkripte) umfasst sind. Hierbei ist bevorzugt, dass es sich bei den Genen um solche handelt, von denen verschieden Varianten oder Allele bekannt sind. Weiterhin bevorzugt sind Varianten die sich durch unterschiedliche Genprodukte voneinander unterscheiden. So ist denkbar, dass die unterschiedlichen Genprodukte, insbesondere die Genprodukte der unten dargestellten Gene, beispielsweise auch durch immunologische Nachweisverfahren oder aufgrund einer biologischen Aktivität nachgewiesen werden können.

In einer besonders bevorzugten Ausführungsform der erfindungsgemäßen Verwendung wird das Gen ausgewählt aus der Gruppe bestehend aus Wachstumshormon-Gen (GH), SRY (sex determining region Y)-box 9 (SOX9), Paired box homeotic Gen 8 (PAX8), Homeobox B-Gencluster (HOXB).

In einer weiteren bevorzugten Ausführungsform der erfindungsgemäßen Verwendung ist die zweite Nukleinsäure von einem auf Chromosom 6 von Sus scrofa gelegenen Gen oder einem orthologen Gen eines anderen Säugers abgeleitet.

In einer weiteren besonders bevorzugten Ausführungsform der erfindungsgemäßen Verwendung wird das Gen ausgewählt aus der Gruppe bestehend aus Cytochrom b light (alpha) chain (*CYBA*), Metallothionein 2A (*MT2A*), Transforming Growth Factor beta 1 (*TGFB1*), α-3-Laminin *(LAMA3),* Extrazelluläres Glycoprotein *(EMILIN2),* Apolipoprotein E 1-Gen (*APOE1*), Alpha 1 B Gylcoprotein (A1BG).

In einer weiteren bevorzugten Ausführungsform der erfindungsgemäßen Verwendung ist die zweite Nukleinsäure von einem auf Chromosom 3 von Sus scrofa gelegenen Gen oder einem orthologen Gen eines anderen Säugers abgeleitet.

In einer besonders bevorzugten Ausführungsform der erfindungsgemäßen Verwendung wird das Gen ausgewählt aus der Gruppe bestehend aus ß-Glucuronidase *(GUSB),* Cytochrom P450 subfamily IIIA (CYP3A), Cytochrom P450 CYP3A29 (CYP3A29), Porcines Homeobox Gen (HBX24), Luteinizing hormone/choriogonadothrophic receptor (LHCGR), Apolipoprotein B *(APOB).*

In einer weiteren bevorzugten Ausführungsform der erfindungsgemäßen Verwendung ist die zweite Nukleinsäure von einem auf Chromosom 7 von Sus scrofa gelegenen Gen oder einem orthologen Gen eines anderen Säugers abgeleitet.

In einer weiteren besonders bevorzugten Ausführungsform der erfindungsgemäßen Verwendung wird das Gen ausgewählt aus der Gruppe bestehend aus Tumor Nekrosefaktor alpha (TNFA), Tumor Nekrosefaktor beta (TNFB), Major Histokompatibilitätskomplex *(SLA),* MHC class I PD14 major transplantation antigen (*MHCTA1*), Prolactin *(PRL).*

In einer weiteren bevorzugten Ausführungsform der erfindungsgemäßen Verwendung ist die zweite Nukleinsäure von einem auf Chromosom 15 von Sus scrofa gelegenen Gen oder einem orthologen Gen eines anderen Säugers abgeleitet ist.

In einer besonders bevorzugten Ausführungsform der erfindungsgemäßen Verwendung wird das Gen ausgewählt aus der Gruppe bestehend aus Integrin α-V(*ITGAV*), Collagen-III-α-1 (*COL3A1*), Homeobox D-Gencluster (*HOXD*), Engrailed 1-Gen (*EN1*).

In eimer weiteren bevorzugten Ausführungsform wird die genannte zweite Nukleinsäure erfindungsgemäß zur Selektion von nicht-menschlichen Säugern ohne Phänotypus "Hernia inguinalis/scrotalis" verwendet. Der Fachmann kennt zahlreiche Selektionsverfahren zur Selektion von Zuchttieren. Verlässliche Verfahren zur Selektion von solchen Tieren das Merkmal "Hernia inguinalis/scrotalis" nicht aufweisen oder an Ihre Nachkommenschaft vererben sind jedoch nicht bekannt. Unter Verwendung der in den erfindungsgemäßen Ausführungsformen genannten ersten und/oder zweiten Nukleinsäure können jedoch verlässliche Selektionsverfahren durchgeführt werden. Beispielsweise könnte mit Hilfe von PCR ein spezifisches Allel nachgewiesen werden, dass gemeinsam mit dem Merkmal "Hernia inguinalis/scrotalis" vererbt wird. Tiere, die Träger eines solchen Allels sind, würden entsprechend von Zuchtprogrammen ausgeschlossen, Tiere, die das Allel nicht tragen, könnten in ein Zuchtprogramm mit aufgenommen werden. Derartige Verwendungen der ersten und/oder zweiten Nukleinsäure sind von den erfindungsgemäßen Verwendungen umfasst.

In einer weiteren bevorzugten Ausführungsform der erfindungsgemäßen Verwendung wird der Säuger ausgewählt aus der Gruppe bestehend aus Sus scrofa, Rind, Pferd, Hund und Mensch. Der Begriff "Sus Scrofa" wird im Zusammenhang mit dieser Erfindung gleichbedeutend mit dem Begriff Schwein verwendet und umfasst alle bekannten Schweinerassen.

In einer weiteren bevorzugten Ausführungsform der erfindungsgemäßen Verwendung wird Sus scrofa ausgewählt aus der Gruppe bestehend aus Deutsche Landrasse, Deutsches Edelschwein, Hampshire, Pietrain, Schwäbisch-Hällisches Schwein, Bunte Bentheimer, Angler Sattelschwein oder hierauf basierenden Hybriden oder Züchtungen. Weiterhin bevorzugt sind Duroc und Leicoma. Von der erfindungsgemäßen Verwendung werden ebenfalls solche Tiere umfasst, die aus der Hybridzucht hervorgegangen sind. Die Hybridzucht kombiniert durch systematische Kreuzung die Vorzüge verschiedener Zuchtlinien und nutzt spezielle Kreuzungseffekte. Aus diesen Zuchtlinienkombinationen entstehen die Elterntiere z.B. Hybrideber, Hybridsau, der Mastschweine.

Es s i e an dieser Stelle erneut darauf hingewiesen, dass sich Längen der spezifischen Allele auf die untersuchten Tiere beziehen. In anderen Populationen, bei anderen Spezies, können die Mikrosatelliten andere Allele aufweisen, die entweder kürzer oder länger sind. Mit der Angabe des Locus der mit der Vererbung und/oder Ausprägung des Merkmals "Hernia inguinalis/scrotalis" korreliert, hat der Fachmann jedoch die Möglichkeit ohne unzumutbaren Aufwand die in einer Population vorhandenen Allele der hier offenbarten Mikrosatellitenmarker zu identifizieren. Darüber hinaus zeigt die hier vorliegende Erfindung auf welchem Wege solche Markerallele identifiziert werden, die mit einer Ausprägung oder Vererbung des Phänotyps "Hernia inguinalis/scrotalis" korrelieren. Diese können dann in Untersuchungen von Tieren aus anderen Populationen als Marker des Phänotyps eingesetzt werden. So kann der Fachmann mit Hilfe von Sequenzanalyse-Programmen solche Nukleinsäure-Abschnitte im Genom von anderen Tierpopulationen identifizieren, die einen Nukleinsäureabschnitt enthält, der ortholog zu der oben genannten zweiten Nukleinsäure ist. Der so identifizierte ortholge Locus kann dann als Grundlage für weitere Untersuchungen dienen, mit deren Hilfe dann, an diesem Lokus gelegene, spezifische Allele identifiziert werden können, die mit dem Phänotyp "Hernia inguinalis/scrotalis" assoziieren, d.h. an diesen Phänotyp gekoppelt vererbt werden.

In einer bevorzugten Ausführungsform der erfindungsgemäßen Verwendung wird zumindest einer der in Tabelle 19 definierten Haplotypen nachgewiesen, also zumindest einer von (a) SSC12A: 13, 14 oder 44; (b) SSC12B: 32, 62, 64 oder 66; (c) SSC6A: 21, 31, 61, 34 oder 64; (d) SSC6B: 12, 16, 23 oder 24; (e) SSC3A: 15, 16, 45, 46 oder 55; (f) SSC3B: 16, 46, 56 oder 59; oder (g) SSC15A: 14, 22 oder 24; (h) SSC7A: 12, 13, 14, 23 oder 24. Diese Haplotypendaten wurden anhand einer gemischten Population bestehend aus Deutsche Landrasse, Deutsches Edelschwein, Hampshire, Pietrain, Schwäbisch-Hällisches Schwein, Bunte Bentheimer, Angler Sattelschwein und hierauf basierenden Hybriden und Züchtungen erhoben.

Nach der technischen Lehre der vorliegenden Erfindung ist es ausreichend, beispielsweise den Haplotypen 13 von SSC12A nachzuweisen. Dieser Haplotyp wird, wie in Tabelle 19 dargestellt ist, durch das Allel 1 (120bp) des Markers SW1307 und durch das Allel 3 (449bp und 156bp) des Markers GH gebildet. Der Nachweis weiterer in Tabelle 19 dargestellten Haplotypen kann zur Absicherung der Testergebnisse oder zur Steigerung der Aussagekraft des Nachweis eingesetzt werden.

Der Fachmann weiß, daß in unterschiedlichen Populationen unterschiedliche Haplotypen gefunden werden. Auf Basis der Kenntnis des Genortes der gemeinsam mit dem Phänotyp vererbt wird, können jedoch mit Hilfe von genetischen Standardverfahren ohne unzumutbaren Aufwand populationsspezifische Haplotypen bestimmt werden. Somit ist die technische Lehre der vorliegenden Erfindung, die im wesentlichen auf der Kenntnis von Chromosomenabschnitten die mit dem Merkmal "Hernia inguinalis/scrotalis" kosegregieren basiert, generalisierbar. In einer weiteren bevorzugten Ausführungsform der erfindungsgemäßen Verwendung wird ein Genomscreen an mehreren Tieren einer Population durchgeführt. Der Begriff mehrere Säuger" umfasst mindestens zwei Tiere einer Population, bevorzugt mindestens 5 Tiere, stärker bevorzugt mindestens 10 Tiere, noch stärker bevorzugt mindestens 20 Tiere, stärker bevorzugt mindestens 50 Tiere, noch stärker bevorzugt mindestens 250 Tiere, am stärksten bevorzugt mindestens 1500 Tiere. Bei dem Genomscreen wird untersucht, ob eine Nukleinsäure von bis zu 5000 Nukleotiden Länge, bevorzugt bis zu 1000 Nukleotiden, mehr bevorzugt bis zu 350 Nukleotiden, noch mehr bevorzugt bis zu 50 Nukleotiden, am meisten bevorzugt von mindestens 8 Nukleotiden Länge gemeinsam mit dem Merkmal "Hernia inguinalis/scrotalis" vererbt wird. Insbesondere wird hierbei geklärt, beispielsweise mit Hilfe von Nukleinsäure-Amplifikationsverfahren oder Hybridisierungsverfahren, welches Markerallel gemeinsam mit dem Merkmal "Hernia inguinalis/scrotalis" vererbt wird. Eine gemeinsame Vererbung von Nukleinsäuren mit der phänotypischen Ausprägung des Merkmals "Hernia inguinalis/scrotalis" impliziert eine genetische Kopplung der Nukleinsäure an das Merkmal. Bevorzugte Marker sind die in den erfindungsgemäßen Ausführungsformen genannten Mikrosatellitenmarker. Neben den genannten Mikrosatelliten können auch andere Nukleinsäuresequenzen als Marker verwendet werden, sofern sie im Sinne der Erfindung identisch oder im wesentlichen identisch sind mit der zweiten, in der Erfindung offenbarten Nukleinsäure oder in einem der oben genannten Nukleinsäurebereiche liegen.

Die Erfindung betrifft weiterhin ein in *vitro* Verfahren zur Bestimmung der Prädisposition zur Ausprägung oder Vererbung des Phänotypus "Hernia inguinalis/scrotalis", wobei man die Säuger, deren befruchtete oder unbefruchtete Eizellen, oder deren Sperma auf die Anwesenheit, Beschaffenheit oder Ausprägung einer der vorgenannten zweiten Nukleinsäure testet. Unterschiedliche ,;Ausprägungen oder Beschaffenheiten" von Nukleinsäuren können z.B. aufgrund von Insertionen, Duplikationen, Deletionen, Substitutionen oder Translokationen hervorgerufen werden. Insertionen oder Deletionen resultieren beispielsweise in einer geänderten Nukleinsäurelänge. Duplikationen sind ein üblicherweise bei der Generierung von Mikrosatelliten beobachtetes Phänomen. Dieser Längenpolymorphismus kann z.B. in einer PCR Reaktion dargestellt werden und schlägt sich bei Verwendung von geeigneten flankierenden Primern z.B. im Falle der Insertion in einem längeren PCR-Produkt nieder. Die unterschiedliche "Ausprägung oder Beschaffenheit" kann weiterhin auch z.B. eine nah verwandte Genvariante sein, die sich im Extremfall lediglich durch einen einzelnen Nukleotidaustausch von der verwandten Gensequenz unterscheidet. Derartige unterschiedliche Ausprägungen oder Beschaffenheiten" der Nukleinsäure können gegebenenfalls mit Hilfe von RFLP-Analysen (Restriktionsfragment-Längenpolymorphismen (RFLPs) oder durch Nukleinsäuresequenzierung dargestellt werden.

In einer bevorzugten Ausführungsform des erfindungsgemäßen Verfahrens wird der Säuger ausgewählt wird aus der Gruppe bestehend aus Sus scrofa, Rind, Pferd, Hund und Mensch.

In einer besonders bevorzugten Ausführungsform des erfindungsgemäßen Verfahrens wird das Sus scrofa ausgewählt aus der Gruppe bestehend aus Deutsche Landrasse, Deutsches Edelschwein, Hampshire, Pietrain, Schwäbisch-Hällisches Schwein, Bunte Bentheimer, Angler Sattelschwein oder hierauf basierenden Hybriden oder Züchtungen.

In bevorzugten Ausführungsform des erfindungsgemäßen Verfahrens wird (a) eine PCR Amplifikation mit komplementären Primern mit einer Länge von mindestens 8 Nukleotiden durchführt, wobei ein Primer an den + Strang und ein weiterer Primer in entgegengesetzter Orientierung den - Strang der in den erfindungsgemäßen Ausführungsformen genannten zweiten Nukleinsäure bindet; oder wird (b) eine Hybridisierung durchgeführt, wobei eine Hybridisierungssonde mit einer Länge von mindestens 8 Nukleotiden an die in den erfindungsgemäßen Ausführungsformen genannte zweite Nukleinsäure bindet; oder wird (c) eine Sequenzierung der in den erfindungsgemäßen Ausführungsformen genannten zweiten Nukleinsäure durchführt; oder wird (d) eine Detektion mit einem spezifischen Antikörper oder Antikörperfragment oder Antikörperderivat oder Aptamer durchführt, wobei der Antikörper oder das Antiköperfragment oder das Antikörperderivat oder das Aptamer spezifisch gegen eine der in den erfindungsgemäßen Ausführungsformen genannten ersten oder zweiten Nukleinsäure gerichtet ist. Im letztgenannten Fall wird ein Antikörper verwendet, der gegen eine spezifische, erste Nukleinsäuresequenz gerichtet ist, eine zweite Nukleinsäuresequenz die sich von der ersten lediglich durch mindestens einen Sequenzunterschied (Mutation) unterscheidet aber nicht bindet. Die spezifische, erste Nukleinsäure kann also, z.B. nach Transfer auf eine Nitrozellulosemembran, mit Hilfe dieses Antikörpers nachgewiesen werden indem dieser Antikörper entweder selbst markiert ist oder mit Hilfe eines zweiten, markierten Antikörpers auf der Membran sichtbar gemacht wird. Eine ausbleibende Bindung an die Membran würde auf die Abwesenheit der ersten Nukleinsäuresequenz hinweisen.

In der PCR Reaktion bindet ein Primer an den + Strang und ein weiterer Primer in entgegengesetzter Orientierung an den - Strang der oben offenbarten zweiten Nukleinsäure. Im Reaktionsgemisch sind neben der oben genannten Nukleinsäure und einem Überschuss an Primern weiterhin vorhanden ein Überschuss an Desoxynukleosidtriphosphaten sowie eine DNA Polymerase, z.B. Taq Polymerase. Bei geeigneten Puffer- und Reaktionsbedingungen binden die Primer an die Nukleinsäure und die DNA Polymerase verlängert die Primer anhand der in der Nukleinsäure vorgegebenen Nukleotidsequenz. Durch Anheben und Absenken der Reaktionstemperatur lösen sich die Polymerisationsprodukte von der Nukleinsäure, so dass andere Nukleinsäuren, meist die im Überschuss vorhandenen Primer, an die Nukleinsäure binden können. Eine zyklische Wiederholung dieser Temperaturbedingungen resultiert konsequenterweise in einer Amplifikation der Nukleinsäuresequenz. Die Annealingtemperatur eines Primers wird von seinem Adenin + Tymin sowie Cytosin + Guanin Gehalt beeinflusst. Für jedes Adenin und Tymin werden 2 °C gerechnet, während für jedes Cytosin und Guanin 4 °C berechnet wird. Die Qualität einer PCR-Reaktion wird von der Primer-Konzentration, der wechselnden dNTP-Menge im PCR-Mix und der Qualität der Taq-DNA-Polymerase direkt beeinflusst. Ein typisches Reaktionsgemisch von 12,5μl ist z.B. wie folgt zusammengesetzt: 0,20μM Primer, 200μM dNTPs, 0,50 U Taq-Polymerase, 1,25μl 10 × Buffer, 1,50 μl DNA (50ng/μl) und mit H2O auf 12,5 μl aufgefüllt. Bevorzugt werden die im Methodenteil dieser Anmeldung aufgeführten Reaktionsbedingungen gewählt.

Als Primer werden solche Nukleinsäuren verstanden, die mindestens eine Länge von 8 Nukleotiden aufweisen und an eine der oben offenbarten zweite Nukleinsäuren binden. Bevorzugte Primer haben eine Länge von mindestens 8 Nukleotiden, bevorzugt mindestens 15 Nukleotiden, stärker bevorzugt mindestens 20 Nukleotiden, noch stärker bevorzugt eine Länge von mindestens 30 Nukleotiden, am stärksten bevorzugt eine Länge von mindestens 50 Nukleotiden. Die Nukleotidsequenzen der Primer können beliebig aus den oben offenbarten zweiten Nukleinsäuresequenzen zusammengestellt werden, soweit sie zumindest 8 aufeinanderfolgende Nukleotide aufweisen. Alternativ dazu kann es (bei Identität) bei der Hybridisierung der Primer mit der Zielsequenz innerhalb der zweiten Nukleinsäure auch zu Basenfehlpaarungen kommen, sofern unter den gewählten Reaktionsbedingungen eine Hybridisierung zustande kommt, die zu einer Elongationsreaktion führen kann. Ein Primer sollte innerhalb von 8 benachbarten Nukleotiden 7 identische Nukleotide aufweisen. Allerdings schließt die Erfindung auch solche Ausführungsformen ein, bei denen 4, 5 oder 6 der 8 Nukleotide mit der entsprechenden Sequenz der zweiten Nukleinsäure identisch sind. Selbstverständlich sind die Grundprinzipien der PCR-Methodik einzuhalten, deren Verfahrensschritte und Reaktionsbedingungen Stand der Technik sind. Im Einzelnen können die Verfahrensschritte aber dennoch einer Anpassung durch den Fachmann bedürfen. Insbesondere ist sicherzustellen, dass die Spezifität der Reaktion erhalten bleibt, was im Einzelnen gegebenenfalls durch Vorversuche ausgetestet werden muss. Nur beispielsweise sei hier auf dem Fachmann bekannte Laborbücher verwiesen, die diese Methodik beschreiben, beispielsweise auf Sambrook et al., 1989, Molecular Cloning, A Laboratory Manual, Cold Spring Harbor und alle nachfolgenden Auflagen. PCR-Verfahren sind z.B. beschrieben in Newton, PCR, BIOS Scientific Publishers Limited, 1994, und nachfolgende Auflagen. Neben der PCR-Amplifikation ist aber auch die Reverse Polymerase Kettenreaktion (RT-PCR) ein bevorzugtes Nachweisverfahren. Neben den genannten Amplifikationsmethoden sind in den vergangenen Jahren weitere Amplifikationsverfahren entwickelt worden, die ebenfalls bevorzugte Ausführungsformen der Erfindung darstellen. Weitere Amplifikationsverfahren sind beispielsweise die "Ligase Chain Reaction" (LCR, EP-A 320308), "Cyclic Probe Reaction" (CPR,), "Strand Displacement Amplification" (SDA, Walker et al., Nukleic Acids Res. 1992 (7):1691-6.) oder "Transciption-based amplification systems" (TAS, Kwoh et al Proc. Nat. Acad Sci. USA 86:1173 (1989), Gingeras et al., PCT Application WO 88/10315).

Ein anderes bevorzugtes Nachweisverfahren zur Bestimmung der Prädisppsition zur Ausprägung des Phänotyps "Hernia inguinalis/scrotalis" umfasst die Hybridisierung mit einer Hybridisierungssonde. Hierbei versteht man unter der Hybridisierungssonde eine Nukleinsäure mit einer Länge von bis zu 50 Nukleotiden, stärker bevorzugt bis zu 100 Nukleotiden, noch stärker bevorzugt bis zu 200, 300, 400, 500 oder 1000 Nukleotiden und am meisten bevorzugt bis zu 5000 Nukleotiden, die an eine der oben offenbarten zweiten Nukleinsäuren bindet. Hierbei muss die Spezifität des Nachweises in Vorversuchen ausgetestet werden, was eine für den Fachmann gängige Praxis darstellt. Vorzugsweise ist die erste Nukleinsäure mit einer detektierbaren Markierung, wie einer radioaktiven oder fluoreszierenden Markierung versehen. Beispiele für Hybridisierungsverfahren sind Dot Blot, Northern Blot, Reverse Northern Blot, in situ Hybridisierung oder Southern Blot (Sambrook et al., 1989, Molecular Cloning, A Laboratory Manual, Cold Spring Harbor und alle nachfolgenden Auflagen).

Ein anderes bevorzugtes Nachweisverfahren zur Bestimmung der Prädisposition zur Ausprägung des Phänotyps "Hernia inguinalis/scrotalis" ist die Sequenzierung einer der oben offenbarten zweiten Nukleinsäure. Sequenzierverfahren sind aus dern Stand der Technik bekannt und bedürfen für den Fachmann keiner weiteren Erläuterung. Beispielsweise sei hier auf Sambrook et al., 1989, Molecular Cloning, A Laboratory Manual, Cold Spring Harbor (und alle nachfolgenden Auflagen verwiesen), worin die Verfahren nach Sanger und Maxam/Gilbert dargestellt sind. Als Primer werden erfindungsgemäß solche Nukleinsäuren verstanden die eine Länge von mindestens 8 Nukleotiden, bevorzugt mindestens 15 Nukleotiden, stärker bevorzugt mindestens 18 Nukleotiden, noch stärker bevorzugt mindestens 21 Nukleotiden, am stärksten bevorzugt eine Länge von mindestens 25 Nukleotiden aufweisen. Für Sequenzierprimer gelten die für die PCR-Primer dargestellten Eigenschaften entsprechend.

Ausgehend von den vorstehend offenbarten Nukleinsäuren kann der Fachmann ohne unzumutbaren Aufwand durch Austesten feststellen, welche Primer oder Hybridisierungssonden, die aus den oben genannte zweiten Nukleinsäuren abgeleitet sind, zu speziellen Nachweisverfahren, beispielsweise PCR-Verfahren, Sequenzierung oder Hypridisierungsverfahren geeignet und welche nicht, oder weniger geeignet sind. Die Primer oder Hybridisierungssonden zum Einsatz in der Erfindung können auch beispielsweise in größeren DNA- oder RNA-Sequenzen vorliegen, z.B. flankiert von Restriktionsschnittstellen, darüber hinaus können Nukleinsäuren, Hybridisierungssonden und Primer auch aus Basenderivaten aufgebaut sein. Eine Reihe von Modifikationen ändern die Chemie des Phosphdiester-Rückrats der DNA bzw. der RNA, der Zucker oder heterocyklischen Basen. Untern den nützlichen Modifikationen befinden sich unter anderem Phosphorthioate; Phosphordithioate, bei denen beide, nicht an der Wasserstoffbrückenbindung beteiligten Sauerstoff-Atome durch Schwefel, Phosphoramide, Alkylphosphotriester und/oder Boranophosphate ersetzt sind. Achirale Phosphat-Derivate umfassen 3'-O'-5'-S-Phosphorthioate, 3'-S-5'-O-Phosphorthioate, 3'-CH2-5'-O-Phosphonate and 3'-NH-5'-O-Phosphoroamidate. Bei Peptid-Nukleinsäuren kann das gesamte Phosphodiester-Rückrat durch peptidische Bindungen ersetzt sein. Zucker-Modifikationen werden verwendet, um die Stabilität oder Affinität zu verändern. Das A-Anomer von Desoxyribose kann verwendet werden, wobei die Base in Bezug auf das natürliche B-Anomer invertiert ist. Die 2'-OH Gruppe der Ribose kann zum entsprechenden 2'-O-Methyl oder 2'-O-Allyl Zuck.er verändert werden, wodurch ein Stabilitätsgewinn erzielt wird, ohne Beeinträchtigung der Bindungsaffinität. Einige weitere nützliche Substitutionen umfassen Desoxyuridin anstatt von Desoxythymidin; 5-methyl-2'-Desoxycytidin und 5-Bromo-2'-Desoxycytidin anstatt von Desoxycytidin. 5-Propyyl-2'-Desoxyuridin und 5-Propyyl-2'-Desoxycytidin können Desoxythymidin und Desoxycytidin ersetzen und somit die Affinität und biologische Aktivität steigern.

Zur Detektion können die Nukleinsäuren eine Markierung aufweisen. Beispiele hierfür sind eine radioaktive Markierung z.B. mit 35S, 32P oder 3H, Fluoreszenzmarkierung, Biotinmarkierung, Digoxigeninmarkierung, Peroxidasemarkierung oder Markierung mit einer alkalischen Phosphatase. Die in der Hybridisations- oder Amplifikationsreaktion verwendeten Nukleinsäuren können weiterhin mit verschiedenen geeigneten Markern versehen sein. Geeignete Marker umfassen Fluorochrome, z.B. Fluorescein Isothiocyanat (FITC), Rhodamin, Texas Red, Phycoerythrin, Allophycocyanin, 6-Carboxyfluorescein (6-FAM), 2',7'-Dimethoxy-4',5'-Dichloro-6-Carboxyfluorescein (JOE), 6-Carboxy-X-Rhodamine (ROX), 6-Carboxy-2',4',7',4,7-Hexachlorofluorescein (HEX), 5-Carboxyfluorescein (5-FAM) oder N,N,N',N'-Tetramethyl-6-Carboxyrhodamine (TAMRA). Die Markierung kann weiterhin Teil eines mehrstufigen Systems sein, wobei die Nukleinsäure mit Biotin konjugiert ist, oder mit einem Hapten oder einem ähnlichen Stoff der einen hochaffinen Bindungspartner hat, z.B. Avidin, spezifische Antikörper etc., wobei in diesem Fall der Bindungspartner mit einer detektierbaren Verbindung konjugiert ist. Im Fall der Amplifikationsreaktion kann die Markierung mit einem Primer konjugiert und/oder die Nukleotide im Pool der Amplifikationsreaktion mit einer geeigneten Markierung versehen sein, so dass die Markierung in das neu entstehende Amplifikationsprodukt eingebaut wird. Alternativ können Doppelstränge, die in einer Hybridisierungsreaktion neu entstanden sind, aber auch durch DNA-Doppelstrang spezifische Antikörper nachgewiesen werden. Besagte Antikörper sind dadurch charakterisiert, dass sie lediglich an doppelsträngige DNA binden, nicht aber an einzelsträngige DNA.

Ein anderes bevorzugtes Nachweisverfahren ist die Detektion der ersten oder zweiten Nukleinsäure mit einem spezifischen Antikörper oder Antiköperfragment oder Antikörperderivat oder einem Aptamer. Bei diesem Verfahren werden spezifische Antiköper generiert, die die ersten oder zweiten Nukleinsäuren erkennen. Fragmente von Antikörpern sind z.B. Fv, Fab- oder F(ab')2 - Fragmente Derivate schließen scFvs, chimäre oder humanisierte Antikörper mit ein. Aptamere sind Nukleinsäuren, die aufgrund ihrer dreidimensionalen Struktur spezifisch an ein Zielmolekül binden. Verfahren zur Generierung spezifischer Antikörper sind aus dem Stand der Technik bekannt. Die Spezifität der Bindung an die genomische Nukleinsäure kann z.B. durch Kompetitionsexperimente mit radioaktiv markierter gewünschter Zielnukleinsäure und nicht gewünschter, z.B. zufällig ausgewählter Nukleinsäure getestet werden. Übliche Nachweisverfahren, in denen Antikörper Verwendung finden, sind z.B. ELISA oder RIPA aber auch Immunfluoreszenz und andere Nachweisverfahren. Die Antikörper binden hierbei spezifisch die ersten oder zweiten Nukleinsäuren. Die Antikörper-Bindung kann z.B. durch Markierung der primären Antikörper sichtbar gemacht werden oder wird mit Hilfe von Antiköper-bindenden zweiten Antikörper detektiert, die dann ihrerseits markiert sind. Die Antikörper können z.B. mit fluoreszierenden Substanzen, durch radioaktive Markierung oder eine enzymatische Markierung modifiziert sein. Immunologische Nachweisverfahren unter Verwendung von spezifischen Antikörpern, wie auch die Generierung von Antikörpern und Fragmenten oder Derivaten davon sind, wie bereits erwähnt, aus dem Stand der Technik bekannt. Beispielsweise sei hier genannt Harlow et al., 1988, Antibodies: A Laboratory Manual, Cold Spring Harbor Laboratory Press und alle nachfolgenden Auflagen.

Eine andere bevorzugte Ausführungsform betrifft das erfindungsgemäße Verfahren, wobei ein Genomscreen an mehreren Säugern einer Population durchgeführt wird. Der Begriff "mehrere Säuger" umfasst in diesem Zusammenhang mindestens zwei Tiere einer Population, bevorzugt mindestens 5 Tiere, stärker bevorzugt mindestens 10 Tiere, noch stärker bevorzugt mindestens 20 Tiere, stärker bevorzugt mindestens 50 Tiere, noch stärker bevorzugt mindestens 250 Tiere, am stärksten bevorzugt mindestens 1500 Tiere. Bei dem Genomscreen wird untersucht, ob eine Nukleinsäure von bis zu 5000 Nukleotiden Länge, bevorzugt bis zu 1000 Nukleotiden, mehr bevorzugt bis zu 350 Nukleotiden, noch mehr bevorzugt bis zu 50 Nukleotiden, am meisten bevorzugt von mindestens 8 Nukleotiden Länge gemeinsam mit dem Merkmal "Hernia inguinalis/scrotalis" vererbt wird. Insbesondere wird hierbei geklärt, beispielsweise mit Hilfe von Nukleinsäure-Amplifikationsverfahren oder Hybridisierungsverfahren, welches Markerallel gemeinsam mit dem Merkmal "Hernia inguinalis/scrotalis" vererbt wird. Eine gemeinsame Vererbung von Nukleinsäuren mit der phänotypischen Ausprägung des Merkmals "Hernia inguinalis/scrotalis" impliziert eine genetische Kopplung der Nukleinsäure an das Merkmal. Bevorzugte Marker sind die in den erfindungsgemäßen Ausführungsformen genannten Mikrosatellitenmarker. Neben den genannten Mikrosatelliten können auch andere Nukleinsäuresequenzen als Marker verwendet werden, sofern sie im Sinne der Erfindung identisch oder im wesentlichen identisch sind mit der zweiten, in der Erfindung offenbarten Nukleinsäure oder in einem der oben genannten Nukleinsäurebereiche liegen.

In einer bevorzugten Ausführungsform des erfindungsgemäßen Verfahrens wird zumindest einer der in Tabelle 19 definierten Haplotypen (a) SSC12A: 13, 14 oder 44; (b) SSC12B: 32, 62, 64 oder 66; (c) SSC6A: 21, 31, 61, 34 oder 64; (d) SSC6B: 12, 16, 23 oder 24; (e) SSC3A: 15, 16, 45, 46 oder 55; (f) SSC3B: 16, 46, 56 oder 59; (g) SSC15A: 14, 22 oder 24; (h) SSC7A: 12, 13, 14, 23 oder 24 nachgewiesen. Um beispielsweise die Aussagekraft des Nachweises zu erhöhen können zwei, drei, vier fünf oder mehr der erfindungsgemäßen Haplotypen bestimmt werden.

Die Erfindung betrifft weiterhin ein Kit mindestens enthaltend (a) ein Primerpaar zur Amplifikation der in den erfindungsgemäßen Ausführungsformen genannten zweiten Nukleinsäure, wobei jeweils ein Primer an den + Strang und ein weiterer Primer an den - Strang dieser Nukleinsäure bindet; oder (b) eine Hybridisierungssonde mit einer Länge von mindestens 8 Nukleotiden die an die in den erfindungsgemäßen Ausführungsformen genannte zweite Nukleinsäure bindet; oder (c) einen spezifischen Antikörper oder ein Antikörperfragment oder ein Antikörperderivat oder ein Aptamer das an die in den erfindungsgemäßen Ausführungsformen genannte erste oder zweite Nukleinsäure bindet; in einem oder mehreren Behältern.

PCR basierende Kits enthalten ein Primerpaar zur Amplifikation einer der oben genannten zweiten Nukleinsäure. Als Primer werden solche Nukleinsäuren verstanden, die mindestens eine Länge von 8 Nukleotiden aufweisen und an eine der oben offenbarten zweite Nukleinsäuren binden. Bevorzugte Primer haben eine Länge von mindestens 8 Nukleotiden, bevorzugt mindestens 15 Nukleotiden, stärker bevorzugt mindestens 20 Nukleotiden, noch stärker bevorzugt eine Länge von mindestens 30 Nukleotiden, am stärksten bevorzugt eine Länge von mindestens 50 Nukleotiden. Die Nukleotidsequenzen der Primer können beliebig aus den oben offenbarten zweiten Nukleinsäuresequenzen zusammengestellt werden, soweit sie zumindest 8 aufeinanderfolgende Nukleotide aufweisen. Ein Primer sollte innerhalb von 8 benachbarten Nukleotiden mindestens 7 mit der Zielsequenz identische Nukleotide aufweisen. Allerdings schließt die Erfindung auch solche Ausführungsformen ein, bei denen 4, 5 oder 6 der 8 Nukleotide mit der entsprechenden Sequenz der zweiten Nukleinsäure identisch sind.

Auf Hybridisierungverfahren basierende Kits enthalten eine Hybridisierungssonde. Die Hybridisierungssonde kann bis zu 50 Nukleotide lang sein, stärker bevorzugt bis zu 100 Nukleotide, noch stärker bevorzugt bis zu 1000 Nukleotide und am meisten bevorzugt bis zu 5000 Nukleotide oder länger sein. Vorzugsweise ist die Hybridisierungssonde eine radioaktiv-markierte Nukleinsäure oder sie enthält modifizierte Nukleotide.

Kits zum Nachweis von Nukleinsäuren auf ELISA, RIA, RIPA oder ähnlicher Basis enthalten einen spezifischen Antikörper oder ein Antikörperfragment oder ein Antikörperderivat oder ein Aptamer. Antikörper oder Antikörperfragment oder Antikörperderivat oder Aptamer sind spezifisch gegen die erste oder zweite Nukleinsäure gerichtet. Übliche Nachweisverfahren in denen die Kits Verwendung finden sind z.B. ELISA oder RIPA aber auch Immunfluoreszenz und andere Nachweisverfahren. Immunologische Nachweisverfahren und Verfahren zur Generierung von spezifischen Antikörpern sind aus dem Stand der Technik bekannt. Die Komponenten des Kits können in Behältern verpackt sein wie z.B. Fläschchen, optional auch in Puffern und /oder Lösungen. Gegebenenfalls können einer oder mehrere der Komponenten in demselben Behälter verpackt sein.

Zusätzlich oder alternativ können eine oder mehrere Komponenten an einen festen Träger absorbiert sein, wie z.B. an Nitrozellulosefilter, Nylonmembranen, oder an die Vertiefung einer Mikrotiterplatte.

In der Beschreibung sind eine Reihe von Dokumenten zitiert. Der Offenbarungsgehalt dieser Dokumente inklusive von Gebrauchsanweisungen von Herstellern ist hiermit per Referenz inkorporiert.

Die Figuren zeigen:

Figur 1: Descensus testis beim Schwein (nach WENSING 1986).

(A) Entwicklung am Tag 65 post conceptionem (p.c.); (B) 75 Tage p.c.; (C) 85-90 Tage p.c.; (D) bei der Geburt. 1. Hoden (*Testis*); 2. Nebenhoden (*Epididymis*); 3. parietales Blatt des Bauchfells (*parietales Peritoneum*); 4. innerer schiefer Bauchmuskel (*Musculus obliquus intemus abdominis*); 5. äußerer schiefer Bauchmuskel (*Musculus obliquus externus abdominis*); 6. Scheidenhautfortsatz (*Processus vaginalis*); 7a. Gubernaculum; 7b. vaginaler Teil (*pars vaginalis*); 7c. infravaginaler Teil (*pars infravaginalis*); 8. Hodenheber (*Musculus cremaster*); 9. *Fascia spermatica*; 10. *Tunica vaginalis.*

Figur 2: Kopplungskarte des porcinen Chromosoms 3.
Figur 3: Multipoint-Analyse für SSC3.
Figur 4: Multipoint-Analyse für SSC6.
Figur 5: Multipoint-Analyse für SSC7.
Figur 6: Multipoint-Analyse für SSC12.
Figur 7: Multipoint-Analyse für SSC15.
Figur 8: Multipoint-Analyse für SSC3 nach Pedigree-Erweiterung und Feinkartierung.
Figur 9: Multipoint-Analyse für SSC6 nach Pedigree-Erweiterung und Feinkartierung.
Figur 10: Multipoint-Analyse für SSC7 nach Pedigree-Erweiterung und Feinkartierung.
Figur 11: Multipoint-Analyse für SSC12 nach Pedigree-Erweiterung und Feinkartierung.
Figur 12: Multipoint-Analyse für SSC15 nach Pedigree-Erweiterung und Feinkartierung.

Die nachfolgenden Beispiele erläutern die Erfindung:

**Beispiel 1: Material, experimentelle Methoden**

Tiere

Blut-, Gewebe- und Spermaproben wurden seit 1997 von vier Zucht- und Besamungsorganisationen (BHZP Lüneburg, Schweinezuchtverband Nord-West Münster, GFS Ascheberg und SWE Oldenburg) von Schweinen mit Phänotyp Bruch sowie deren Eltern gesammelt. Es gingen insgesamt 1629 Proben mit 756 betroffenen Ferkeln ein. Die Informationen auf den Probeneinsendungen waren Tieridentifikation, Elternangabe, Bruchstatus, Wurfnummer der Sau, Abferkeldatum, Bruchlokalisation und der Einsendebetrieb bzw. die Organisation.
Parallel zu dieser Sammlung wurden Eber mit einer hohen Frequenz von Nachkommen, die unter einer *Hernia inguinalis* bzw. *scrotalis* litten; an ausgewählte Sauen angepaart. Ziel war es, Würfe zu generieren, in denen die Anzahl an Brüchlingen erhöht war. Von allen Tieren aus diesen gezielten Paarungen wurden Blut-, Gewebe- oder Spermaproben genommen. Die isolierte DNA wurde im Weiteren für das Genomic mismatch scanning verwendet.

**EINTEILUNG VON FAMILIEN FÜR DEN GENOMSCAN**

Für den Genomscan mit polymorphen Markern wurden zunächst 71 Halbgeschwisterfamilien (sogenannte Eberfamilien) verwendet (Pedigree I), für die zu Beginn des Projekts Probenmaterial vorlag. Dieses Pedigree I wurde im Laufe des Versuchs auf 84 Halbgeschwisterfamilien erweitert (Pedigree II). Das Kriterium einer Eberfamilie ist, dass von dem Eber mindestens ein Vollgeschwisterpaar an Bruchferkeln vorliegt. Die kleinste im Versuch vorliegende Eberfamilie besteht aus dem Eber, der Mutter und einem Bruchferkelpaar, die größte nach Probensammlung aus einem Eber, vier Paaren an Vollgeschwisterbruchferkeln, zwei Halbgeschwisterbruchferkeln und den jeweiligen Müttern. Es gibt kein weibliches Tier im gesammelten Material, das mehr als eine Abferklung mit Bruchferkeln aufwies. Typisiert wurden im Folgenden alle Eltern und alle Brüchlinge. Die Genotypen der Mütter von Halbgeschwistern wurden zur Verminderung des Typisierungsaufwandes und, da kein Informationsgewinn damit verbunden war, nicht analysiert.

**Enzyme, Oligonukleotide, Größenstandards, Chemikalien und Kits**

**ENZYME**

Enzyme wurden von folgenden Firmen verwendet:

Amersham Pharmacia Biotech, Freiburg
Boehringer-Mannheim, Mannheim
New England Biolabs, Schwalbach

Qiagen, Düsseldorf

**OLIGONUKLEOTIDE**

MWG-Biotech, Ebersberg: unmarkierte und fluoreszenzmarkierte Oligonukleotide Applied Biosystems, Weiterstadt: fluoreszenzmarkierte Oligonukleotide

**GRÖßENSTANDARDS**

Applied Biosystems, Weiterstadt: GeneScan™-500 ROX™ Size Standard

**CHEMIKALIEN**

Die verwendeten Chemikalien wurden von folgenden Firmen bezogen:

Amersham Pharmacia Biotech, Freiburg
Applied Biosystems, Weiterstadt
Bio-Rad Laboratories, München
Biozym Diagnostik, Hessisch Oldendorf
Fluka Chemie, Buchs
Gibco BRL Life Technologies, Heidelberg
ICN Biomedicals, Eschwege
MBI Fermentas, St. Leon-Rot
Merck, Darmstadt
Qiagen, Düsseldorf
Roth, Karlsruhe
Sigma Chemie, München

**Verbrauchsmaterialien, Glaswaren und Laborgeräte**

**VERBRAUCHSMATERIALIEN**

Verbrauchsmaterialien wurden von den folgenden Firmen geliefert:

Eppendorf, Hamburg
Greiner Labortechnik, Solingen
Kisker, Steinfurt
Kranich, Göttingen
Sarstedt, Nürmbrecht
Schleicher und Schuell, Dassel
Schütt Labortechnik, Göttingen
Stratagene, Heidelberg

**GLASWAREN**

Glaswaren stammen von den Firmen:
Schütt Labortechnik, Göttingen
Kranich, Göttingen

**LABORGERÄTE**

Agfa-Gevaert AG, Leverkusen: Entwicklermaschine CURIX 60
Amersham Pharmacia Biotech, Freiburg: Hoefer DyNA Quant 200 Fluorometer;GeneQuant-proRNA/DNA-Calculator; Electrophoresis Power Supply EPS 600
Applied Biosytems, Weiterstadt: 3100 Genetic Analyzer
Bachofer Laborgeräte GmbH, Reutlingen: Transilluminatoren mit 312 nm und 366 nm;Heizblock

BDK Luft- und Reinraumtechnik GmbH, Sonnenbühl- Genkingen: Abzug

Bender & Hobein, Zürich: Vortex Genie 2

Biometra, Göttingen: PCR-Maschine Biometra T-Gradient

Bio-Rad Laboratories, München: Elektrophoresekammern für Agarosegele;Fluorometer Smart Spec$^{TM}$ 3000

Du Pont und Servall Instruments, Bad Homburg: Sorvall RC 5B Refrigerated Superspeed Centrifuge

Eppendorf, Hamburg: Tischzentrifugen 5415 C und 5415 R; Kühlzentrifuge 5417 R; Thermoblock Thermostat 5320; Research-Pipetten

Heraeus Instruments, Hannover: Inkubator; Zentrifugen (Megafuge 1.0R; Biofuge 28RS)

Infors, Bottmingen: Schüttelinkubator CH-4103

Invitrogen, Karlsruhe: Kammern für Polyacrylamidgelelektrophorese Novex Mini-Cell (X Cell Sure Lock)

Janke & Kunkel, Staufen: Magnetrührer

Millipore, Eschborn: Reinstwasseranlage

MWG-Biotech, Ebersberg: Hybaid Omni Gene Thermocycler; Kammern für Agarosegelelektrophorese

Qiagen, Düsseldorf: Zentrifuge 4-15C

Schütt-Labortechnik, Göttingen: Bioclav; Kühlschränke und Gefrierschränke; Haake Wasserbad DC3; pH-Meter 761 Calimatic; Waagen

Stratagene, Heidelberg: Eagle Eye II; RoboCycler Gradient 96

## METHODEN

### Isolierung von DNA

ISOLIERUNG VON GENOMISCHER DNA AUS VOLLBLUT

Es wurden 3 ml Blut mit 7 ml bidestilliertem Wasser versetzt, um die Zellen zum Platzen zu bringen und um die Zellkerne freizusetzen. Der Ansatz wurde 2-3 min gemischt und anschließend bei 5000 rpm für 10 min bei Raumtemperatur zentrifugiert. Der Überstand wurde abgegossen, und das Pellet, welches die Zellkerne enthält, wurde in 1 ml Nonidet (0,1 %) resuspendiert. Es wurden weitere 9 ml Nonidet hinzugegeben und bei 5000 rpm für 10 min bei Raumtemperatur zentrifugiert. Der Überstand wurde abgegossen und das Pellet in 1 ml Proteinase K-Puffer resuspendiert. Hiernach wurden 70 μl 10 % SDS und 100 μl Proteinase K hinzugegeben und der Ansatz für mindestens 2 h im Wasserbad bei einer Temperatur von 56 °C inkubiert. Nach der Inkubation wurden dem Ansatz 350 μl 6 M NaCl zugegeben und für 2 min kräftig geschüttelt bis die Lösung schaumig war. Es folgte eine Zentrifugation für 10 min bei 5000 rpm und 4 °C. Der Überstand wurde anschließend zum Fällen der DNA in 5 ml 100%igen Ethanol gegeben. Die gefällte DNA wurde mit Hilfe einer Impföse gefischt und in einer adäquaten Menge 1 x TE-Puffer gelöst.

| Proteinase K-Puffer pH 7,4: | 10 mM | Tris |
|---|---|---|
| | 25 mM | EDTA |
| | 75 mM | NaCl |

Proteinase K-Konzentration: 10 mg/ml

| 1x TE-Puffer: | 10 mM | Tris/HCl pH 8.0 |
|---|---|---|
| | 1 mM | EDTA |

ISOLIERUNG VON GENOMISCHER DNA AUS GEWEBE

Ein bis zwei Gramm Gewebe wurden manuell zerkleinert und mit 350 μl Gewebelysispuffer und 100 μl Proteinase K versetzt. Anschließend wurde der Ansatz zur vollständigen Lyse des Gewebes über Nacht bei 56 °C inkubiert. Zu dem gelösten Gewebe wurden 500 μl Phenol gegeben, der Ansatz gemischt und 5 min bei 14000 rpm und Raumtemperatur zentrifugiert. Von den zwei entstandenen Phasen wurde die obere in 500 μl Chloroform gegeben, der Ansatz gemischt und erneut bei Raumtemperatur 5 min bei 14000 rpm zentrifugiert. Es bildeten sich wiederum zwei Phasen, von denen die obere in 100%igen Ethanol überführt wurde, um die DNA zu fällen. Die gefällte DNA wurde mit einer Impföse gefischt und in einem angemessenen Volumen 1 x TE-Puffer gelöst.

| Gewebelysispuffer: | 50 mM | Tris/HCl pH 7,5 |
|---|---|---|
| | 100 mM | EDTA |
| | 100 mM | NaCl |
| | 1 % | SDS |
| | 1 $\mu$g/ml | Proteinase K |

## ISOLIERUNG VON GENOMISCHER DNA AUS SPERMA

Zu 200 $\mu$l Ebersperma wurde 1 ml Spermawaschpuffer gegeben, der Ansatz gemischt und 10 min bei 5000 rpm und Raumtemperatur zentrifugiert. Der Überstand wurde abgegossen, und das Pellet in 1 ml Spermawaschpuffer resuspendiert. Es folgte erneut eine Zentrifugation für 10 min bei 5000 rpm und Raumtemperatur. Zum in 2 ml Spermawaschpuffer resuspendierten Pellet wurden 150 $\mu$l Proteinase K gegeben und 6 h bei 65 °C inkubiert. Nach der Inkubation folgte eine Zentrifugation für 10 min bei 5000 rpm und Raumtemperatur. Das Pellet wurde in 2 ml Sperma-S-Puffer resuspendiert, 150 $\mu$l Proteinase K wurden zugegeben, und der Ansatz wurde über Nacht im Schüttelwasserbad bei 37 °C inkubiert. 2 ml Phenol wurden zugegeben, gemischt und 5 min bei 5000 rpm und Raumtemperatur zentrifugiert. Von den zwei entstandenen Phasen wurde die obere in 2 ml Chloroform gegeben, gemischt und wiederum bei Raumtemperatur 5 min bei 5000 rpm zentrifugiert. Es bildeten sich erneut zwei Phasen, von denen die obere in 100%igen Ethanol überführt wurde, um die DNA zu fällen. Die gefällte DNA wurde mit einer Impföse gefischt und in einem angemessenen Volumen 1 $\times$ TE-Puffer gelöst.

| Spermawaschpuffer pH 6,4: | 130 mM | Tris |
|---|---|---|
| | 200 mM | EDTA |
| | 40 mM | Trizma Pre-Set Crystals |
| | | (Sigma-Aldrich Chemie, Steinheim) |
| | 2 % | SDS |

| Sperma-S-Puffer pH 8,5: | 65 mM | Tris |
|---|---|---|
| | 25 mM | EDTA |
| | 10 mM | DTT |
| | 2 % | N-Lauryl-Sarcosinat |

## POLYMERASE-KETTENREAKTION

Bei der Entwicklung geeigneter Primerpaare für die Polymerase-Kettenreaktion (PCR) wurden folgende Parameter beachtet: Schmelztemperatur, optimale Annealingtemperatur, Nukleotidverteilung (GC-Gehalt gegenüber AT-Gehalt) und mögliche Primer/Primer-Wechselwirkungen. Mit einer PCR in der ein Temperaturgradient für die Annealingtemperatur verwendet wurde, wurde für jedes Primerpaar die spezifische Annealingtemperatur bestimmt.

Standard-PCR-Ansatz:

| ca. 100 ng | DNA |
|---|---|
| 0,5-2,5 Units | Taq-Polymerase |
| 1x | 10x PCR-Reaktionspuffer des Herstellers |
| 2,5-5 $\mu$l | 5x Q-Solution |
| 1,5 mM | $MgCl_2$ |
| 10-50 pmol | Primer 1 |
| 10-50 pmol | Primer 2 |
| 0,1-0,5 mM | Desoxyribonukleosidtriphosphate |

PCR-Ansatz mit *puReTaq Ready-To-Go PCR Beads*[1]:

| | |
|---|---|
| ca. 100 ng | DNA |
| 0,5 bzw. 1 | Bead |
| 10-50 pmol | Primer 1 |
| 10-50 pmol | Primer 2 |
| ad 12,5 bzw. 25 $\mu$l | ddH$_2$O |

[1] *puReTaq Ready-To-Go PCR Bead* gelöst in einem Volumen von 25 $\mu$l entspricht: 200 $\mu$M dATP, 200 $\mu$M dCTP, 200 $\mu$M dGTP, 200 $\mu$M dTTP, 10 mM Tris-HCl (pH 9.0), 50 mM KCl, 1,5 mM MgCl$_2$ und 2,5 Units puReTaq DNA Polymerase

Die Komponenten des Ansatzes wurden auf Eis pipettiert, gemischt, kurz zentrifugiert und die Reaktionsgefäße in einen Thermocycler überführt. Nach 1-5 min Predenaturierung bei 92-95 °C, um zu gewährleisten, dass die DNA-Moleküle einzelsträngig in der Lösung vorliegen, wurden 30-35 Zyklen mit 30-60 s Denaturierung bei 92 °C, 30-60 s Annealing bei der jeweiligen Annealingtemperatur des Primerpaares und 30-60 s Elongation bei 72 °C durchgeführt. Nach dem letzten Zyklus erfolgte eine finale Elongation bei 72 °C für 10 min.

**Mikrosatelliten**

**MIKROSATELLITENAUSWAHL**

Die Auswahl der Mikrosatelliten aus den bekannten Karten erfolgte unter den Gesichtspunkten Polymorphiegrad, Abstand der Marker zueinander, Position auf dem Chromosomen und Standardisierung der PCR-Bedingungen. Für den Genomscan wurden 133 porcine Mikrosatellitenmarker ausgewählt (ROHRER et al. 1994b). Der Markerabstand sowie die Länge der Chromosomen wurde den Angaben unter http://www.marc.usda.gov/ entnommen. Lagen zwei Marker an der gleichen Position, wurden sie in den statistischen Auswertungen mit einem Abstand von 0,1 cM berücksichtigt.

Zunächst wurden die Marker so gewählt, dass sie möglichst gleichmäßig das gesamte Genom abdeckten (Genomewide Scan). Chromosomen, für die eine Assoziation zwischen Markern und Phänotyp gefunden wurde (Grobkartierung), wurden in einem zweiten Schritt dichter mit Markern abgedeckt (Feinkartierung).

**MIKROSATELLITENANALYSE**

Für die ausgewählten Mikrosatelliten wurde zunächst eine Gradienten-PCR zur Optimierung der Annealingtemperatur und zur Kontrolle der chromatographischen Auftrennung durchgeführt. Tabelle 3 zeigt die chromosomale Lokalisation, die Primersequenzen und die Annealing-Temperatur der ausgewählten Marker.

Tab. 3: Genetische Marker (chromosomale Lokalisation, Primersequenzen und Annealing-Temperatur) des Genomscans

| SSC | Marker | Primer forward | Primer reverse | $T_A$ in °C |
|---|---|---|---|---|
| 1 | SW1515 | CTCCGGTTTCCATTTGTGG | GATCCCTGCCCCCAACAC | 51 |
| 1 | SW1851 | GGCTTGGACATTCTCATTGG | GGTTGAGGAACCCTGATGTG | 54 |
| 1 | SW2185 | CCCAACCCATTATGATGCAG | AGGATGTTATTCTTAACTCATCTGG | 52 |
| 1 | S0155 | TGTTCTCTGTTTCTCCTCTGTTTG | AAAGTGGAAAGAGTCAATGGCTAT | 53 |
| 1 | S0320 | CCCACAATCCCTGACTACCTG | GATCCAAATACCAAAATCTTG | 54 |
| 1 | SW1301 | TGGATAAGCAATGAGGTCCC | TAGTGGATTTATAATGTGCTAACCC | 54 |
| 2 | SW2443 | GAGCACAGAAGATTTTTAGGGC | TTAGTTTTCTCCTGGGCTGTG | 59 |
| 2 | SWR783 | CATACCTGCACATCTCTTCAGC | GCAGCTATAGCTCCGATTGG | 62 |
| 2 | S0141 | GATCTGGTCTGTCTTGTGTCCT | AGACCCCAACTCTTGGTCTCAT | 57 |
| 2 | SW1201 | CCAACCAACCAACAGAAAAC | CGGCACTGGTAACTCCAATT | 56 |
| 2 | SW1026 | TGGAGAGGCAATGCTGTATG | GTATTTCACCTGCAGCTCCC | 60 |
| 2 | INSL-3 A | GTCTACTCTTGTATAGATGA | AGAGCATTCCCAAAGGAC | 52 |
| 2 | INSL-3 B [1] | GTCTACTCTTGTATAGATGA | AGAGCATTCCCAAAGGAC | 52 |
| 2 | SW1408 | CAGCCCTGTCACTTGAGTAGC | TTCTGCTCTACAGCAAAGCG | 60 |
| 2 | S0036 | AGTGACGTGAGGGTCTGCTCCTC | ATGGACGGTGGATTCACAGCC | 51 |
| 3 | SW274 | CGCACAGCGACATCTTTTA | AAGTGCAGCCCTAAAAAGACA | 55 |
| 3 | SW2021 | GCGACACATGAGATAAAACTGC | AATCCACAGGCTTACTCAGATG | 56 |
| 3 | SW2429 | TCTTTTTAGGGTGGAGGATGG | CATGTCCCCTATGAACTCTGTG | 58 |
| 3 | SW833 | CTGACTGTTTTGCTGCAGTG | TCCACTGAGGTCTCTCACTCTC | 58 |
| 3 | SW72 | ATCAGAACAGTGCGCCGT | TTTGAAAATGGGGTGTTTCC | 50 |
| 3 | SE51018 | TCTAAACAAAGGGCAGGTGG | GGACTTCCATATGCTGTGGG | 51 |
| 3 | S0701 | GCAGAGTGATTCAGTTATAC | TCATCTTCCCTACCACC | 60 |
| 3 | S0206 | TGGGTGTGGTCAACAACCAA | ACGTGCCTGCCTCTACCATC | 57 |
| 3 | SW2408 | AGACACTTGTAGTCGCTCCTCC | AGACAAAAGGGGATGCCAC | 57 |
| 3 | S0002 | GAAGCCAAAGAGACAACTGC | GTTCTTTACCCACTGAGCCA | 56 |
| 3 | S0397 | GGCTAATTGGAGCTGAGAAGCA | AGTGCAGCATTTCCGATAACTCTG | 54 |
| 3 | SW1327 | AGCTCTTGAAGCTCAAAGACG | CATACTTCTCCAGGAGGAAAGC | 54 |
| 3 | SW349 | CCTGTTGTAGGCTCCATGAG | CTAGGAGTCGGCCCTGAAC | 53 |
| 4 | SW489 | CAAGTGTGAAATTTGTGCGG | CGAAGTGCTAACTATAAGCAGCA | 52 |
| 4 | S0301 | CCGTCTTACTTAGGATGTTT | TGATGTGTTTATGTGTTTGA | 53 |
| 4 | SW871 | ATCCCTGTTTCCTCCACCTC | AATTAAAGCCATTCACTGGGG | 57 |
| 4 | S0073 | ACTGAAACAGGAATTCAGATCC | TGAAGTATTATGGCATCATGGA | 51 |
| 4 | S0097 | GACCTATCTAATGTCATTATAGT | TTCCTCCTAGAGTTGACAAACTT | 53 |
| 5 | SW413 | CAGACACACACCCCAGTGTC | AGGTCCAACCCTCCTGATG | 66 |
| 5 | SW1482 | ATTGCAGACTACAGTTCTTGCC | ACTTACGGGCTGATGCTGTC | 50 |
| 5 | SW2425 | ATCTCCATAGGTCAGAGGCTC | ACTCTGTGAGACATTCCTGTATTCC | 56 |
| 5 | SW2 | TGCCAATGGTGTGGCTATAA | CCCTGAAGGCTCAGATGGT | 55 |
| 5 | SW1468 | CTCCAGATGTTACACACATGTG | GAATATTCAGAATGTGGCGTG | 55 |
| 5 | SW378 | ATTATGCACCCCTACTCCCC | GATTTCTTCTTTGTTTGTGCCC | 60 |
| 6 | MP35 | CTGCCAAGAGAGGCTCTTCTCAA | CATCCGCCTGGTTCCCTCCCTAT | 69 |
| 6 | SW2406 | AATGTCACCTTTAAGACGTGGG | AATGCGAAACTCCTGAATTAGC | 53 |
| 6 | SW1841 | TTCTCGAATCTGACCATGACAC | AGCTTCACTGATAAGGAAGTCACTG | 54 |
| 6 | SW1067 | TGCTGGCCAGTGACTCTG | CCGGGGGATTAAACAAAAAG | 54 |
| 6 | RYR1 | GTGCTGGATGTCCTGTGTTCCCT | CTGGTGACATAGTTGATGAGGTTTG | 68 |

## EP 1 630 236 A2

Tab. 3: Genetische Marker (chromosomale Lokalisation, Primersequenzen und Annealing-Temperatur) des Genomscans (Fortsetzung)

| SSC | Marker | Primer forward | Primer reverse | $T_A$ in °C |
|---|---|---|---|---|
| 6 | SW1376 | TGATTTACAATGTTGGGTCG | GATCATGTGAGAAAAAGAATG | 56 |
| 6 | SW1129 | GATCATATGAGGAAAAGAATGTGT | CACAGGGGGAACACCTTAAT | 54 |
| 6 | SW122 | TTGTCTTTTTATTTTGCTTTTGG | CAAAAAAGGCAAAAGATTGACA | 54 |
| 6 | SW617 | CTGGGTTTACAGTGTTCTGCC | TGTGATGGAGCCTGATAGAGG | 68 |
| 6 | SWR987 | TTGTTATGCCTACCTGTGTTGG | CTCCATATGCCACAGGTGTG | 53 |
| 6 | SW316 | TTCTCCAGCCATCATGAGTG | AATGACCATTCCTGAGGCTG | 57 |
| 6 | SW2505 | AGGTAACCTGGGCTTGGG | ACCAATATGCTGGGCACC | 64 |
| 6 | S0003 | GAAGTGTTAAGGAAAGCCTT | AGCCTCAGTTTCTCTACCTA | 51 |
| 6 | SW2098 | ACAGAAGGAGCTACTTACTGTTTGG | ACTAGCTCTAAAATTCTATGTTGCATG | 54 |
| 6 | SW1881 | ATATGACCCAGCAAGTCTTCTG | CGGAAATACATTTTTATGGCTG | 56 |
| 6 | SW322 | CATTCAACCTGGAATCTGGG | TCCCTGGAAAGGCTACACC | 58 |
| 6 | SW2419 | AGGGCGTGCTCTTCTAACTG | TGACTCAGCATCTCCTGCC | 53 |
| 7 | SW2564 | TGACTTAGCCTCATGTGTCCC | GCTGTGTACAGTTACCTGCAGG | 58 |
| 7 | SW1354 | GAGCCAGATTAATGCAGTTGC | CCTAGTCCCGAGCGGTAATC | 52 |
| 7 | SW1369 | AGCCTTCTCTGGCTCATGG | TCAAATGGAATCATCTCTTCCC | 54 |
| 7 | SY33 | AGGGCTTCCATCGAGGTC | TCTTCCCTTTTCTTTTTTATAGTTGC | 52 |
| 7 | TNFB | CTGGTCAGCCACCAAGATTT | GGAAATGAGAAGTGTGGAGACC | 56 |
| 7 | SW472 | AAAATGAACCCTCTCCAGTTTC | TCTGAACACTACAGCCCGC | 56 |
| 7 | SW2019 | ATGATGCGAACCTGGAACTC | TATGTGTAACTTGGTCCCATGC | 58 |
| 7 | SW1856 | TCATTCCAAACACACAGAGTCC | TTGTATGGTATCCTGTGATGCC | 58 |
| 7 | SW352 | GCCCCCATTCTCAATTCAC | GATCAAGCTCCCCTCTTCG | 51 |
| 7 | SWR773 | GTGGCTGGGGTATAGGCC | TGCTGAAGCATCCACTTCAC | 55 |
| 7 | S0212 | CCACGACTCAAACCTTAG | TCTTTCTTAGAATATCTCACAT | 55 |
| 8 | SW2410 | ATTTGCCCCCAAGGTATTTC | CAGGGTGTGGAGGGTAGAAG | 55 |
| 8 | SW2521 | GTGCGCTGGTGTGTGTTC | GAGGTGACTTCTGCAGGTCTG | 56 |
| 8 | S0225 | GCTAATGCCAGAGAAATGCAGA | CAGGTGGAAAGAATGGAATGAA | 53 |
| 8 | SW1551 | TTTACTTGGGGAAACCCTCC | GATCAACCCAAATTCTTGGC | 51 |
| 8 | S0178 | TAGCCTGGGAACCTCCACACGCTG | GGCACCAGGAATCTGCAATCCAGT | 61 |
| 9 | SW983 | GCAGTCCCACTCTTAGGTATATATCC | ATAATGCTGCTATGAACACTGTAGTG | 52 |
| 9 | SW911 | CTCAGTTCTTTGGGACTGAACC | CATCTGTGGAAAAAAAAAGCC | 55 |
| 9 | S0019 | TTCTTATTTCTCTGTGTCTT | ATTGTTTCCCTTTCTTCTGA | 55 |
| 9 | S0295 | GCCTAAAAAGACCAAAGAA | TACTGCTGAGGCAAAGGA | 58 |
| 9 | SW749 | TTCCCAAACCAACCAAAGAG | AGGAACTTGCCAAAATCACG | 58 |
| 10 | SW1894 | CTCACTGCAAAAACAGGTCTTG | CCTAGGTCTTAGGCTTCTAGGTTG | 58 |
| 10 | SW173 | CTGGGAACCTCCATGTGC | GTCCTGGGCCTTTAGGTAGG | 58 |
| 10 | S0070 | GGCGAGCATTTCATTCACAG | GAGCAAACAGCATCGTGAGC | 59 |
| 10 | SW920 | CATGGAGCTGAACTTGCAAA | ATCAAGCCCAACTTAAGAATACA | 54 |
| 10 | SW2067 | GAAGAAATTAAATGCACGTCCC | TTGCTGCTTGTGCCTTTG | 55 |
| 11 | SW2008 | CAGGCCAGAGTAGCGTGC | CAGTCCTCCCAAAAATAACATG | 60 |
| 11 | S0071 | GACATGGAATCAGGTTGCTCAA | CCAGAAGCAGGTTTTGAGATGA | 65 |
| 11 | S0230 | AACAGCCCAAGTGCCCATT | TCCCCCTCCACTTCCTTTC | 58 |
| 11 | SW703 | AAGATGAAGCAGGAACTCAAGG | CTTGATGGCTTTACTGTTCACC | 56 |
| 11 | SW1135 | TAAGTTTAGGTGCCTCATTTGATTT | GAAAACTCTCTTAGTTTCTTTATGCAA | 60 |
| 12 | SW2490 | TGTTTGTCTGTCTGTCTCTCTTCC | TGTGCTTTTCAGAGGCAGG | 56 |
| 12 | S0229 | TTGGCATTTACTGTCTTTAGTGACGA | GGCCATATCTGGTATTGGGTGTCT | 54 |

Tab. 3: Genetische Marker (chromosomale Lokalisation, Primersequenzen und Annealing-Temperatur) des Genomscans (Fortsetzung)

| SSC | Marker | Primer forward | Primer reverse | $T_A$ in °C |
|---|---|---|---|---|
| 12 | SW1307 | TCATCCTTCCTTTTCTTTATTTCTT | TCTGGCTCGGATGCAATC | 57 |
| 12 | GH | CTGGGGAGCTTACAAACTCCTT | TTATCCATTAGCACATGCCTGCCA | 59 |
| 12 | SW874 | AAAAGAACCCAACTACAGCAGC | TTTATGAGGGTATCCTGACACC | 55 |
| 12 | SW168 | GCACTTTCTTCCCTTACCCC | CAGTGTAAAGCATGGAAGATGC | 54 |
| 12 | S0090 | CCAAGACTGCCTTGTAGGTGAATA | GCTATCAAGTATTGTACCATTAGG | 54 |
| 12 | S0147 | AGCTGCAGCTCCAGATCATCT | GCTGTAAGCAGAGATTAACAC | 57 |
| 12 | LOX12A | CTTCAATGGTGCAGTGGGTTG | ACTTTAATCAGATACAGAAGTTGG | 53 |
| 12 | SW605 | AGCCTTCTGTGCAGAAAAGC | CCCCAGGTTCTCTGCTCTC | 54 |
| 12 | SWR1021 | CGCCACAAGTGAACTCCC | CCGCGGGTCCAGCTATAG | 58 |
| 13 | SWR1941 | AGAAAGCAATTTGATTTGCATAATC | ACAAGGACCTACTGTATAGCACAGG | 62 |
| 13 | SW344 | AGCTTCGTGTGTGCAGGAG | GTAGTGGTCCAAAGAGAGTGCC | 53 |
| 13 | SWR1008 | ACAGCCACCAACAGTGTTTG | GAACTTCCATATGCTGCAAGTG | 62 |
| 13 | S0068 | AGTGGTCTCTCTCCCTCTTGCT | CCTTCAACCTTTGAGCAAGAAC | 59 |
| 13 | SW1056 | GGTGGTTGGTTCTCAAAAACA | TTTCTGGTGTACAGCAAAGTGA | 57 |
| 13 | S0289 | AGGAGCATTTGGCCACGTCTG | TGTTGACCTTCTGTGATGGGGC | 58 |
| 14 | SW857 | TGAGAGGTCAGTTACAGAAGACC | GATCCTCCTCCAAATCCCAT | 53 |
| 14 | SW295 | ACCTGCCAGAGTTGTGGC | AAGAGTTTCATTTCTCCCATCC | 62 |
| 14 | SW2439 | GTTTCTTTGTTGCCAACTTGC | AGCACTAGAGCTCAGGAGATGG | 58 |
| 14 | SW342 | GGGTTCTGTGGTAGTGACTGC | TCATCCACAACAGCAGAACC | 55 |
| 14 | S0007 | TTACTTCTTTGGATCATGTC | GTCCCTCCTCATAATTTCTG | 50 |
| 14 | SW1557 | TGCTCTAATCTACCCGGGTC | CCACCCCACTCCCTTCTG | 53 |
| 14 | SWC27 | CTGAGACTGTGCTGCTCACTG | CCATTTTCCAAAAACATGGG | 53 |
| 15 | SW2072 | AACAAGCACGGAGTATGATGG | AAGATGAACCCCAAAGTCCC | 50 |
| 15 | S0355 | TCTGGCTCCTACACTCCTTCTTGATG | TTGGGTGGGTGCTGAAAAATAGGA | 61 |
| 15 | SW919 | TCCAAAGTCATGAAGATTTATTC | TCACAGACCTAAATGTAAGAGCT | 55 |
| 15 | S0148 | TGGTGTAGGCCTGCAGTTGA | CCATCCATTGCTACTGGCAC | 61 |
| 15 | SW1111 | AGGTCCTACTGTCCATCACAGG | GAAGCAGAGTTGGCTTACAGTG | 66 |
| 15 | SW964 | GTGGTTCCTCTATGCAGAGTCC | ATGTGATGAAACATGATGGAGG | 60 |
| 15 | S0369 | GAGAGGAAGGAGAAGGAAACAAG | AGGTCTATGTGTCAGCATAAAGAA | 55 |
| 15 | S0118 | TGAACTTTATATGTACATGTATGC | CCTTTATTTACAGACCACAATGGG | 52 |
| 15 | SWR1533 | ATTTCTTCTTCTTCTTCTTTGTGTG | CTGGGAAGCTCCATATTCCA | 50 |
| 15 | SW120 | TTTTAAGATGTGGCTGTGTTGG | GATCACCTGCTAAGTGAAAGTCA | 60 |
| 15 | SW906 | GAGGACAATGTGAGAAAAAGAATG | TTTTTTCCTGTGATTAGAACTCTTAGG | 55 |
| 15 | SW1983 | GCAGGTTCGGCCTTAAAAG | CCACATAGCTCCCTGCTACC | 64 |
| 15 | SW1119 | CAACCTCAAAAATGGAGAAAGG | GTTCTTGCGGTGTTTGGC | 55 |
| 16 | S0111 | TCAGTTATTTCTGGCTATCATCTC | TTGATGTAGACCACCCAGCTAGTG | 46 |
| 16 | SW813 | AGTTGATTTAAAATGTTGTGCCA | AATATTTCAAAAAAAGGAATGCG | 52 |
| 16 | SW742 | AATTCTACTTCTGGGGAGAGGG | CTTTTGGGAACATTTCTGCC | 51 |
| 16 | S0298 | ACATAACATCGTAAATCAGC | CTCCATCACAGGTCTCACA | 55 |
| 16 | SW81 | GATCTGGTCCTGCACAGGG | GGGGCTCTCAGGAAGGAG | 55 |
| 16 | SW1897 | GTGCCGTGGCAGGAACTC | ACTGCCATTTGTTTTCAAAGTG | 55 |
| 17 | SWR1004 | TGGGAACACCTGCTTCATTC | TCCATATGCCCCAAGTGTG | 53 |
| 17 | S0296 | TGAAAAATAACAAGAACCAC | AAAAGCAAATAATGATAATAG | 50 |
| 17 | SW1031 | ATCACCCAGACAAAACAATCTC | TATGTCAACCCCAAACCCC | 58 |
| 17 | SW2431 | CTTCCCAGGATGTTGTCTAAAC | CATGGTGCACACTTAGTGGG | 55 |
| 18 | SW1808 | CCAAAAAAGTGGACTGTAAGCC | TACGGATGGATGGAGACAGG | 51 |

| 18 | LEP | TGCAGTCTGTCTCCTCCAAA | CGATAATTGGATCACATTTCTG | 55 |

Tab. 3: Genetische Marker (chromosomale Lokalisation, Primersequenzen und Annealing-Temperatur) des Genomscans (Fortsetzung)

| SSC | Marker | Primer forward | Primer reverse | $T_A$ in °C |
|---|---|---|---|---|
| 18 | SW787 | CTGGAGCAGGAGAAAGTAAGTTC | GGACAGTTACAGACAGAAGAAGG | 46 |
| 18 | HOXA10 | ATGTCAGCCAGAAAGGGCTATC | TCATTGCGCTTGCCTCCCAGA | 60 |
| 18 | SWR414 | GATTTGACCCCATGCCTG | AAGGCAAACCCCTTGAGTTC | 51 |
| X | SW2470 | TAGTACCTAGGCTTCCCCAGG | CTTTGTCTCTCCCTCTCCATAC | 58 |
| X | SW2588 | TGTCTTCTTCCCCCTCCC | AAAGCCTGGTGAGGACCC | 62 |

1) Snap-shot PCR: AAGCTTGTCAGCACTCATAT (50 °C)

Die selektierten Mikrosatelliten wurden anschließend mittels PCR mit Primern, von denen einer mit Fluorophoren (Hex, Ned, Fam, Joe) markiert wurde, amplifiziert (vergleiche Kapitel 4.3.6). Folgendes Temperaturprofil wurde für die PCR verwendet:

|  | Predenaturierung | 92 °C | 2 min |
|---|---|---|---|
| 35 Zyklen: | Denaturierung | 92 °C | 30 s |
|  | Annealing | primerspezifisch | 30 s |
|  | Elongation | 72 °C | 30 s |
|  | Finale Elongation | 72 °C | 10 min |

Die PCR-Produkte wurden mit Hilfe eines *ABI PRISM 3100 Analyzer* elektrophoretisch aufgetrennt. Dazu wurden je nach Farbmarkierung des Primers 0,2-0,7 $\mu$l der PCR-Produkte mit 12 $\mu$l Formamid und 0,5 $\mu$l *GenScan$^{TM}$-500 ROX$^{TM}$ Size Standard* gemischt. Die Auswertung der Mikrosatellitendaten erfolgte mit den Programmen Genescan$^{TM}$3.7 und Genotyper$^{TM}$3.6 (Applied Biosystems, Weiterstadt).

**Single Nucleotide Polymorphisms (SNPs)**

Neben den Mikrosatelliten wurden auch folgende sechs SNPs am Tiermaterial untersucht:

Leptin-Gen *(LEP):* STRATIL et al. 1997
Homeobox A10-Gen *(HOXA10):* UIBELEISEN 2001
Ryanodin-Rezeptor-Gen (*RYR1*): BREM u. BRENIG 1992
Wachstumshormon-Gen (*GH*): NIELSEN u. LARSEN 1991
Insulin-like factor 3-Gen (*INSL3*): KNORR et al. 2004

**Statistische Auswertungen**

**ALLELFREQUENZSCHÄTZUNG UND BERECHNUNG DES INFORMATIONSGEHALTES DER MARKER**

Die Allelfrequenzen der Marker wurden anhand aller genotypisierten Tiere geschätzt. Der Informationsgehalt der Marker wurde mit Hilfe des Programms ALLEGRO (GUDBJARTSSON et al. 2000) berechnet. Der prozentuale Informationsgehalt an einer Markerposition stellt die tatsächliche Information im Verhältnis zur maximal möglichen Information im Rahmen des vorhandenen Pedigrees dar.

**NICHTPARAMETRISCHE KOPPLUNGSANALYSE**

Die Auswertung der Daten erfolgte mit dem Programm ALLEGRO. Es wurde eine nichtparametrische Kopplungsanalyse durchgeführt, d.h. es wurde eine Kopplungswahrscheinlichkeit mit dem Krankheitslocus bestimmt ohne einen Erbgang vorauszusetzen. Allele wurden dabei in aufsteigender Folge fortlaufend nummeriert. Es wurde sowohl eine Singlepoint-Analyse wie auch eine Multipoint-Analyse durchgeführt. NPL-Werte wurden zusammen mit einer zugehörigen Irrtumswahrscheinlichkeit (p-Wert) für alle Chromosomen berechnet. Es konnten alle typisierten Marker in die Berechnungen einbezogen werden. Bei der Multipoint-Analyse wurde der NPL-Wert an den Markerpositionen sowie an Positionen zwischen den Markern bestimmt.

Die Irrtumswahrscheinlichkeit p basiert auf Schätzwerten, die Genauigkeit dieser Schätzung ist abhängig vom Informationsgehalt des Pedigrees und der verwendeten Marker. Dieser Informationsgehalt wird prozentual anhand der höchstmöglichen Information des Materials bestimmt. Als Signifikanzschwelle für Kopplung wurde ein NPL-Wert von 1,96 gewählt, bei einer gleichzeitigen Irrtumswahrscheinlichkeit von kleiner als 0,05.

**TRANSMISSION-DISEQUILIBRIUM-TEST (TDT-TEST)**

Der Transmission-Disequilibrium-Test wurde mit dem Programm GASSOC (Genetic Association analysis software for cases and parent) Version 1.06 durchgeführt (SCHAID 1996).

**Beispiel 2: Charakterisierung des Markersets anhand der vorliegenden Tierfamilien**

Die verwendeten Mikrosatellitenmarker hatten einen durchschnittlichen Informationsgehalt von 38 % mit einem Minimum von 3,6 % und einem Maximum von 69,7 %. Die spezifischen Allelfrequenzen für die Marker, die im Rahmen dieser Arbeit typisiert wurden, wurden anhand aller typisierten Tiere geschätzt und sind der Tabelle 4 zu entnehmen. Die Anzahl der Allele der einzelnen Marker schwankt zwischen 2 und 18. Die Verteilung der Marker über das Genom und ihre Abstände zueinander zeigt Tabelle 5.

Tab. 4: Allelfrequenzen der typisierten genetischen Marker

| Marker | SSC | Allele | Allelfrequenzen (kleinstes Allel – gößtes Allel) |
|--------|-----|--------|--------------------------------------------------|
| SW1515 | 1 | 6 | 0.36 0.01 0.51 0.08 0.02 0.12 |
| SW1851 | 1 | 6 | 0.09 0.03 0.13 0.03 0.58 0.14 |
| SW2185 | 1 | 6 | 0.11 0.29 0.04 0.15 0.14 0.27 |
| S0155 | 1 | 7 | 0.20 0.02 0.03 0.20 0.41 0.11 0.03 |
| S0320 | 1 | 6 | 0.02 0.04 0.10 0.11 0.70 0.03 |
| SW1301 | 1 | 9 | 0.05 0.18 0.38 0.03 0.02 0.01 0.29 0.02 0.02 |
| SW2443 | 2 | 9 | 0.006 0.06 0.004 0.35 0.15 0.12 0.19 0.03 0.09 |
| SWR783 | 2 | 3 | 0.12 0.63 0.25 |
| S0141 | 2 | 12 | 0.07 0.24 0.01 0.025 0.124 0.141 0.32 0.01 0.03 0.01 0.01 0.01 |

| | | | |
|---|---|---|---|
| SW1201 | 2 | 5 | 0.014 0.008 0.035 0.811 0.132 |
| SW1026 | 2 | 6 | 0.059 0.015 0.605 0.186 0.013 0.122 |
| INSL-3 A | 2 | 2 | 0.039 0.096 |
| INSL-3 | 2 | 2 | 0.535 0.465 |
| SW1408 | 2 | 8 | 0.004 0.038 0.026 0.078 0.06 0.054 0.65 0.09 |
| S0036 | 2 | 8 | 0.193 0.11 0.02 0.262 0.159 0.009 0.202 0.045 |
| SW274 | 3 | 9 | 0.129 0.002 0.066 0.643 0.071 0.017 0.019 0.050 0.003 |
| SW2021 | 3 | 14 | 0.053 0.012 0.033 0.130 0.006 0.100 0.312 0.102 0.005 0.079 0.005 0.114 0.002 0.047 |
| SW2429 | 3 | 7 | 0.002 0.382 0.132 0.007 0.166 0.324 0.037 |
| SW833 | 3 | 5 | 0.039 0.080 0.379 0.386 0.116 |
| SW72 | 3 | 6 | 0.348 0.028 0.007 0.347 0.215 0.055 |
| SE51018 | 3 | 10 | 0.016 0.006 0.017 0.018 0.510 0.290 0.048 0.008 0.082 0.005 |
| S0701 | 3 | 3 | 0.200 0.772 0.028 |
| S0206 | 3 | 7 | 0.267 0.014 0.015 0.259 0.312 0.077 0.056 |
| SW2408 | 3 | 13 | 0.138 0.178 0.115 0.024 0.001 0.260 0.001 0.102 0.154 0.017 0.002 0.004 0.004 |
| S0002 | 3 | 13 | 0.069 0.058 0.079 0.004 0.329 0.004 0.031 0.206 0.180 0.014 0.014 0.002 0.010 |
| S0397 | 3 | 12 | 0.014 0.110 0.052 0.012 0.099 0.001 0.224 0.381 0.007 0.059 0.017 0.024 |
| SW1327 | 3 | 8 | 0.050 0.008 0.499 0.316 0.070 0.008 0.040 0.009 |
| SW349 | 3 | 11 | 0.001 0.008 0.216 0.097 0.004 0.023 0.009 0.211 0.248 0.023 0.160 |
| SW489 | 4 | 4 | 0.374 0.382 0.118 0.126 |
| S0301 | 4 | 7 | 0.113 0.043 0.093 0.286 0.099 0.312 0.054 |
| SW871 | 4 | 8 | 0.004 0.153 0.006 0.065 0.401 0.114 0.149 0.108 |

Tab. 4: Allelfrequenzen der typisierten genetischen Marker (Fortsetzung)

| Marker | SSC | Allele | Allelfrequenzen (kleinstes Allel – gößtes Allel) |
|---|---|---|---|
| S0073 | 4 | 9 | 0.022 0.146 0.103 0.031 0.554 0.025 0.087 0.018 0.014 |
| S0097 | 4 | 10 | 0.301 0.018 0.002 0.249 0.002 0.041 0.167 0.021 0.007 0.183 |
| SW413 | 5 | 6 | 0.068 0.195 0.227 0.004 0.342 0.164 |
| SW1482 | 5 | 8 | 0.181 0.008 0.088 0.119 0.056 0.062 0.14 0.346 |
| SW2425 | 5 | 8 | 0.084 0.013 0.076 0.186 0.079 0.010 0.526 0.026 |
| SW2 | 5 | 10 | 0.004 0.060 0.010 0.036 0.105 0.175 0.175 0.181 0.067 0.187 |
| SW1468 | 5 | 4 | 0.664 0.309 0.002 0.025 |
| SW378 | 5 | 3 | 0.028 0.677 0.295 |
| MP35 | 6 | 6 | 0.033 0.220 0.308 0.028 0.012 0.399 |
| SW2406 | 6 | 8 | 0.470 0.079 0.022 0.275 0.003 0.079 0.048 0.024 |
| SW1841 | 6 | 17 | 0.168 0.064 0.018 0.135 0.088 0.220 0.005 0.002 0.013 0.011 0.049 0.070 0.088 0.044 0.003 0.018 0.005 |
| SW1067 | 6 | 10 | 0.007 0.004 0.275 0.012 0.145 0.011 0.432 0.021 0.007 0.086 |
| RYR1 | 6 | 2 | 0.915 0.085 |
| SW1376 | 6 | 6 | 0.435 0.060 0.140 0.013 0.038 0.314 |
| SW1129 | 6 | 8 | 0.081 0.012 0.293 0.019 0.096 0.157 0.325 0.017 |
| SW122 | 6 | 9 | 0.037 0.003 0.094 0.052 0.070 0.436 0.141 0.006 0.161 |
| SW617 | 6 | 2 | 0.506 0.494 |
| SWR987 | 6 | 7 | 0.027 0.302 0.304 0.142 0.001 0.208 0.015 |

| SW316 | 6 | 10 | 0.037 0.064 0.057 0.005 0.296 0.166 0.083 0.131 0.008 0.153 |
| SW2505 | 6 | 7 | 0.196 0.006 0.278 0.281 0.171 0.064 0.004 |
| S0003 | 6 | 9 | 0.036 0.510 0.002 0.147 0.011 0.002 0.165 0.065 0.062 |
| SW2098 | 6 | 6 | 0.010 0.399 0.011 0.341 0.182 0.057 |
| SW1881 | 6 | 7 | 0.100 0.317 0.073 0.110 0.090 0.153 0.157 |
| SW322 | 6 | 9 | 0.031 0.079 0.001 0.071 0.327 0.161 0.022 0.108 0.200 |
| SW2419 | 6 | 9 | 0.048 0.065 0.018 0.168 0.180 0.148 0.066 0.295 0.012 |
| SW2564 | 7 | 5 | 0.09 0.83 0.03 0.03 0.02 |
| SW1354 | 7 | 6 | 0.36 0.002 0.08 0.504 0.048 0.006 |
| SW1369 | 7 | 6 | 0.013 0.533 0.236 0.123 0.007 0.088 |
| SY33 | 7 | 7 | 0.067 0.001 0.050 0.461 0.077 0.220 0.124 |
| TNFB | 7 | 11 | 0.255 0.167 0.045 0.054 0.049 0.051 0.045 0.162 0.086 0.001 0.085 |
| SW472 | 7 | 3 | 0.358 0.614 0.028 |
| SW2019 | 7 | 7 | 0.054 0.222 0.277 0.225 0.093 0.097 0.032 |
| SW1856 | 7 | 7 | 0.015 0.018 0.044 0.086 0.153 0.657 0.027 |
| SW352 | 7 | 6 | 0.002 0.008 0.714 0.034 0.234 0.008 |
| SWR773 | 7 | 3 | 0.638 0.027 0.335 |
| S0212 | 7 | 6 | 0.058 0.138 0.069 0.466 0.024 0.245 |
| SW2410 | 8 | 7 | 0.784 0.018 0.028 0.003 0.008 0.149 0.010 |
| SW2521 | 8 | 3 | 0.144 0.579 0.277 |
| S0225 | 8 | 4 | 0.154 0.056 0.709 0.081 |
| SW1551 | 8 | 7 | 0.258 0.197 0.010 0.179 0.290 0.053 0.013 |
| S0178 | 8 | 8 | 0.504 0.024 0.009 0.028 0.061 0.068 0.100 0.206 |
| SW983 | 9 | 8 | 0.120 0.014 0.391 0.085 0.285 0.066 0.021 0.018 |

Tab. 4: Allelfrequenzen der typisierten genetischen Marker (Fortsetzung)

| Marker | SSC | Allele | Allelfrequenzen (kleinstes Allel – gößtes Allel) |
|---|---|---|---|
| SW911 | 9 | 4 | 0.366 0.493 0.093 0.048 |
| S0019 | 9 | 8 | 0.021 0.206 0.080 0.181 0.046 0.385 0.062 0.019 |
| S0295 | 9 | 7 | 0.045 0.406 0.445 0.043 0.006 0.002 0.053 |
| SW749 | 9 | 3 | 0.625 0.032 0.343 |
| SW1894 | 10 | 4 | 0.534 0.275 0.172 0.019 |
| SW173 | 10 | 4 | 0.010 0.181 0.788 0.021 |
| S0070 | 10 | 12 | 0.029 0.255 0.002 0.099 0.236 0.008 0.162 0.072 0.006 0.042 0.013 0.076 |
| SW920 | 10 | 2 | 0.325 0.675 |
| SW2067 | 10 | 7 | 0.021 0.193 0.241 0.075 0.087 0.187 0.196 |
| SW2008 | 11 | 6 | 0.082 0.066 0.042 0.504 0.046 0.260 |
| S0071 | 11 | 5 | 0.015 0.077 0.223 0.142 0.543 |
| S0230 | 11 | 11 | 0.002 0.002 0.002 0.046 0.046 0.076 0.054 0.254 0.486 0.024 0.008 |
| SW703 | 11 | 6 | 0.439 0.047 0.014 0.038 0.446 0.016 |
| SW1135 | 11 | 5 | 0.516 0.381 0.008 0.037 0.058 |
| SW2490 | 12 | 12 | 0.065 0.081 0.012 0.041 0.008 0.007 0.066 0.455 0.018 0.025 0.042 0.180 |
| S0229 | 12 | 11 | 0.004 0.009 0.276 0.035 0.092 0.441 0.023 0.006 0.001 0.056 0.057 |
| SW1307 | 12 | 4 | 0.367 0.016 0.171 0.446 |

| Marker | SSC | Allele | Allelfrequenzen (kleinstes Allel – gößtes Allel) |
|--------|-----|--------|------------------------------------------------|
| GH | 12 | 4 | 0.270 0.055 0.227 0.448 |
| SW874 | 12 | 7 | 0.057 0.278 0.088 0.431 0.010 0.121 0.015 |
| SW168 | 12 | 6 | 0.007 0.141 0.284 0.117 0.001 0.450 |
| S0090 | 12 | 6 | 0.096 0.468 0.082 0.191 0.001 0.162 |
| S0147 | 12 | 7 | 0.021 0.451 0.066 0.457 0.001 0.002 0.002 |
| LOX12A | 12 | 3 | 0.479 0.035 0.485 |
| SW605 | 12 | 8 | 0.006 0.085 0.007 0.010 0.777 0.054 0.057 0.004 |
| SWR1021 | 12 | 7 | 0.644 0.144 0.026 0.031 0.071 0.066 0.019 |
| SWR1941 | 13 | 7 | 0.107 0.115 0.004 0.320 0.182 0.228 0.044 |
| SW344 | 13 | 9 | 0.008 0.062 0.015 0.131 0.445 0.054 0.200 0.077 0.008 |
| SWR1008 | 13 | 11 | 0.170 0.029 0.033 0.184 0.016 0.072 0.364 0.055 0.053 0.020 0.004 |
| S0068 | 13 | 15 | 0.002 0.164 0.267 0.002 0.002 0.005 0.026 0.101 0.085 0.277 0.023 0.009 0.009 0.023 0.005 |
| SW1056 | 13 | 4 | 0.097 0.198 0.455 0.250 |
| S0289 | 13 | 8 | 0.042 0.505 0.020 0.020 0.285 0.014 0.105 0.029 |
| SW857 | 14 | 8 | 0.374 0.004 0.008 0.263 0.261 0.021 0.019 0.050 |
| SW295 | 14 | 5 | 0.352 0.004 0.436 0.168 0.040 |
| SW2439 | 14 | 3 | 0.014 0.964 0.022 |
| SW342 | 14 | 12 | 0.041 0.009 0.005 0.324 0.034 0.007 0.028 0.266 0.188 0.048 0.041 0.009 |
| S0007 | 14 | 10 | 0.003 0.329 0.045 0.020 0.075 0.126 0.035 0.048 0.286 0.033 |
| SW1557 | 14 | 5 | 0.124 0.744 0.067 0.006 0.059 |
| SWC27 | 14 | 6 | 0.177 0.052 0.008 0.598 0.151 0.014 |
| SW2072 | 15 | 7 | 0.064 0.379 0.245 0.204 0.030 0.038 0.040 |
| S0355 | 15 | 9 | 0.480 0.297 0.020 0.001 0.001 0.002 0.113 0.010 0.072 |
| SW919 | 15 | 18 | 0.078 0.007 0.135 0.102 0.330 0.031 0.004 0.001 0.007 0.018 0.111 0.043 0.025 0.004 0.004 0.083 0.013 0.004 |
| S0148 | 15 | 9 | 0.040 0.324 0.003 0.258 0.101 0.039 0.018 0.188 0.029 |

Tab. 4: Allelfrequenzen der typisierten genetischen Marker (Fortsetzung)

| Marker | SSC | Allele | Allelfrequenzen (kleinstes Allel – gößtes Allel) |
|--------|-----|--------|------------------------------------------------|
| SW1111 | 15 | 9 | 0.076 0.010 0.011 0.458 0.017 0.290 0.011 0.001 0.126 |
| SW964 | 15 | 9 | 0.015 0.328 0.057 0.040 0.117 0.034 0.171 0.004 0.234 |
| S0369 | 15 | 5 | 0.044 0.946 0.006 0.003 0.001 |
| S0118 | 15 | 6 | 0.004 0.883 0.101 0.006 0.005 0.001 |
| SWR1533 | 15 | 6 | 0.017 0.148 0.161 0.667 0.004 0.003 |
| SW120 | 15 | 9 | 0.009 0.470 0.127 0.059 0.073 0.144 0.006 0.110 0.002 |
| SW906 | 15 | 6 | 0.041 0.356 0.091 0.168 0.001 0.343 |
| SW1983 | 15 | 13 | 0.102 0.002 0.151 0.084 0.075 0.003 0.350 0.005 0.041 0.002 0.003 0.182 0.005 |
| SW1119 | 15 | 7 | 0.015 0.175 0.261 0.194 0.284 0.017 0.054 |
| S0111 | 16 | 8 | 0.287 0.193 0.073 0.116 0.247 0.052 0.009 0.023 |
| SW813 | 16 | 4 | 0.326 0.016 0.371 0.287 |
| SW742 | 16 | 13 | 0.312 0.016 0.132 0.002 0.012 0.020 0.008 0.208 0.032 0.172 0.082 0.002 0.002 |
| S0298 | 16 | 3 | 0.396 0.341 0.236 |
| SW81 | 16 | 10 | 0.017 0.004 0.006 0.566 0.026 0.123 0.002 0.238 0.013 0.005 |

| Marker | SSC | Allele | Allelfrequenzen (kleinstes Allel – gößtes Allel) |
|--------|-----|--------|--------------------------------------------------|
| SW1897 | 16 | 3 | 0.891 0.104 0.005 |
| SWR1004 | 17 | 6 | 0.004 0.323 0.14 0.395 0.025 0.113 |
| S0296 | 17 | 9 | 0.009 0.006 0.196 0.535 0.032 0.009 0.112 0.019 0.082 |
| SW1031 | 17 | 4 | 0.758 0.049 0.047 0.146 |
| SW2431 | 17 | 7 | 0.015 0.003 0.216 0.460 0.128 0.118 0.060 |
| SW1808 | 18 | 11 | 0.275 0.138 0.150 0.013 0.037 0.042 0.008 0.017 0.237 0.008 0.075 |
| LEP | 18 | 2 | 0.908 0.092 |
| SW787 | 18 | 7 | 0.096 0.240 0.268 0.143 0.129 0.113 0.011 |
| HOXA10 | 18 | 2 | 0.709 0.291 |
| SWR414 | 18 | 6 | 0.011 0.651 0.230 0.004 0.089 0.015 |

## Tab. 5: Verteilung der Marker auf den Chromosomen

| SSC 1 | 153 cM | SSC 2 | 132 cM | SSC 3 | 129 cM | SSC 4 | 131 cM | SSC 5 | 145 cM | SSC 6 | 200 cM |
|---|---|---|---|---|---|---|---|---|---|---|---|
| SW1515 | 16,4 | SW2443 | 0 | SW274 | 0 | SW489 | 8 | SW413 | 8,4 | MP3S | 0 |
| SW1851 | 44,6 | SWR783 | 23,7 | SW2021 | 12,4 | S0301 | 27,1 | SW1482 | 39,9 | SW2406 | 21,4 |
| SW2185 | 67,6 | S0141 | 31,2 | SW2429 | 17,3 | SW871 | 47,8 | SW2425 | 72,3 | SW1841 | 41,5 |
| S0155 | 93,9 | SW1201 | 44,8 | SW833 | 17,3 | S0073 | 74,4 | SW2 | 78,7 | SW1067 | 71,4 |
| S0320 | 112,5 | SW1026 | 60,6 | SW72 | 17,8 | S0097 | 120 | SW1468 | 97,5 | RYR1 | 75 |
| SW1301 | 140,5 | INSL-3A | 75 | SE51018 | 19 | | | SW378 | 134,4 | SW1376 | 76,5 |
| | | INSL-3B | 76 | S0701 | 31,46 | | | | | SW1129 | 80,2 |
| | | SW1408 | 88,5 | S0206 | 42,3 | | | | | SW122 | 83,3 |
| | | S0036 | 132,1 | SW2408 | 94,2 | | | | | SW617 | 86,5 |
| | | | | S0002 | 102,2 | | | | | SWR987 | 86,5 |
| | | | | S0397 | 109 | | | | | SW316 | 89,3 |
| | | | | SW1327 | 109,6 | | | | | SW2505 | 90,2 |
| | | | | SW349 | 112,6 | | | | | S0003 | 102 |
| | | | | | | | | | | SW2098 | 106,1 |
| | | | | | | | | | | SW1881 | 121,1 |
| | | | | | | | | | | SW322 | 149,8 |
| | | | | | | | | | | SW2419 | 161,4 |

| SSC 7 | 157 cM | SSC 8 | 127 cM | SSC 9 | 142 cM | SSC 10 | 128 cM | SSC 11 | 84 cM | SSC 12 | 113 cM |
|---|---|---|---|---|---|---|---|---|---|---|---|
| SW2564 | 0 | SW2410 | -1,3 | SW983 | 4 | SW1894 | 23,2 | SW2008 | 14,1 | SW2490 | 0 |
| SW1354 | 22,3 | SW2521 | 23,1 | SW911 | 36,8 | SW173 | 56,1 | S0071 | 43,7 | S0229 | 19,3 |
| SW1369 | 48,2 | S0225 | 82,8 | S0019 | 86,4 | S0070 | 62,3 | S0230 | 56,4 | SW1307 | 40,2 |
| SY33 | 57,7 | SW1551 | 105,9 | S0295 | 100,5 | SW920 | 86,3 | SW703 | 76,2 | GH | 45 |
| TNFB | 58,1 | S0178 | 127,7 | SW749 | 139,4 | SW2067 | 128 | SW1135 | 83,7 | SW874 | 64,7 |
| SW472 | 58,9 | | | | | | | | | SW168 | 70,5 |
| SW2019 | 60,5 | | | | | | | | | S0090 | 80,2 |
| SW1856 | 61,5 | | | | | | | | | S0147 | 89,9 |
| SW352 | 87,7 | | | | | | | | | LOX12A | 102,5 |
| SWR773 | 117,3 | | | | | | | | | SW605 | 108,3 |
| S0212 | 141,2 | | | | | | | | | SWR1021 | 113,1 |

| SSC 13 | 126 cM | SSC 14 | 111 cM | SSC 15 | 124 cM | SSC 16 | 93 cM | SSC 17 | 97 cM | SSC 18 | 63 cM |
|---|---|---|---|---|---|---|---|---|---|---|---|
| SWR1941 | 14,1 | SW857 | 7,4 | SW2072 | 12,5 | S0111 | 0 | SWR1004 | 17,8 | SW1808 | 0 |
| SW344 | 35,4 | SW295 | 35,7 | S0355 | 13,8 | SW813 | 6 | S0296 | 32 | LEP | 25 |
| SWR1008 | 53 | SW2439 | 41,5 | SW919 | 25,7 | SW742 | 9,3 | SW1031 | 63,4 | SW787 | 31,6 |

| | | | | | | | | | | | |
|---|---|---|---|---|---|---|---|---|---|---|---|
| S0068 | 62,2 | SW342 | 53,2 | S0148 | 34,6 | S0298 | 33,2 | SW2431 | 94 | HOXA10 | 49,6 |
| SW1056 | 96,1 | S0007 | 60 | SW1111 | 39,8 | SW81 | 40,1 | | | SWR414 | 57,6 |
| S0289 | 112,1 | SW1557 | 87,9 | SW964 | 50,7 | SW1897 | 86,2 | | | | |
| | | SWC27 | 111,5 | S0369 | 56 | | | | | | |
| | | | | S0118 | 60 | | | | | | |
| | | | | SWR1533 | 70,4 | | | | | | |
| | | | | SW120 | 79,3 | | | | | | |
| | | | | SW906 | 89,3 | | | | | | |
| | | | | SW1983 | 101,5 | | | | | | |
| | | | | SW1119 | 119,9 | | | | | | |
| SSC X | 200 cM | | | | | | | | | | |
| SW2470 | 45,8 | | | | | | | | | | |
| SW2588 | 128,4 | | | | | | | | | | |

**Beispiel 3: Kopplungsanalyse**

Der Genomscan mit polymorphen Markern, zur Bestimmung von Chromosomenregionen, die mit dem Merkmal Hernien assoziiert sind, erfolgte in zwei Schritten. Zunächst wurde ein 71 Familien (n = 437) umfassendes Pedigree (Pedigree I) mit 108 DNA-Markern typisiert. Einbezogen in die Analyse wurden alle Autosomen. Zwei Marker, die auf dem X-Chromosom lagen (SW2470, SW2588) wurden zur Absicherung des angegebenen Geschlechts ebenso typisiert.

Für die chromosomalen Regionen, für die die höchsten abgesicherten Assoziationen zu dem Merkmal Hernien gefunden wurden, wurde daraufhin die Feinkartierung mit mehr Markern und Meiosen durchgeführt. Es wurden die Chromosomen 3, 6, 7, 12 und 15 an dem 81 Familien umfassenden Pedigree II (n = 512) mit 31 ergänzenden Markern (Tabelle 5 orange markierte Marker) feinkartiert.

Statistisches Auswahlkriterium war der NPL-Wert ($\geq$ 1,96) bei einer gleichzeitig zugrunde liegende Irrtumswahrscheinlichkeit von $p \leq 0,05$ (Exact NPL-p-value).

Unter den insgesamt 139 DNA-Markern, die an den Pedigrees I und II untersucht wurden, befanden sich auch sechs SNPs (INSL-3 A, INSL-3 B, RYR1, GH, LEP, HOXA10), in Genen gelegen, die zu Beginn der Untersuchung als funktionelle Kandidatengene genannt wurden.

An den Pedigrees I und II wurden nichtparametrische Single- und Multipoint-Kopplungsanalysen durchgeführt. Während die Singlepoint-Analyse die Statistik lediglich am untersuchten Marker berechnet, wird bei der Multipoint-Analyse auch der Verlauf der Teststatistik zwischen den Loci kalkuliert. Dadurch erfolgt eine feinere Abdeckung der Chromosomenbereiche, und somit stellt die Multipoint-Analyse eine statistische Feinkartierungsprozedur dar. Eine Kopplung eines Chromosomenbereichs zum Merkmal liegt in vorliegender Arbeit nur dann vor, wenn diese nach Singlepoint- und Multipoint-Analyse (Ausnahme SSC 15) statistisch abgesichert ist. Räumlich definiert ist der Bereich des Effekts durch die Marker, die nach Multipoint-Analyse die statistischen Kriterien erfüllen.

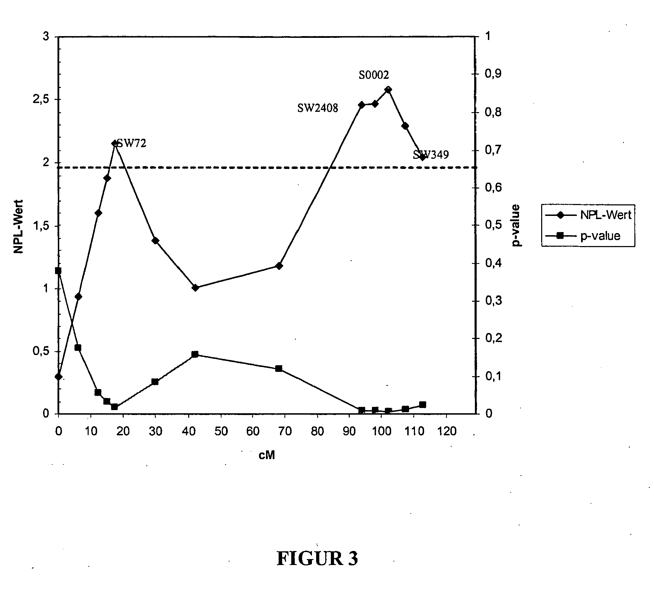

Die NPL-Teststatistik der Multipoint-Analyse und die p-Werte sind graphisch dargestellt (Figuren 3 bis 12). Aufgetragen sind die Distanzen in cM (Abszisse) und die Höhe der Teststatistik mit der Signifikanzgrenze von 1,96 (Ordinate). Der gekoppelte Bereich ist damit der Bereich, welcher oberhalb der Schnittpunkte der NPL-Wertekurve mit der Signifikanzgrenze liegt. Das jeweilige Maximum der TestStatistik gibt die wahrscheinlichste Position des Effekts an. In allen Tabellen sind die Marker, für die Kopplung besteht, fett gedruckt. Zusätzlich typisierte Marker der Feinkartierung sind orange hervorgehoben.

Mittels TDT-Test wurde ferner auf den interessierenden Chromosomen auf Assoziation zwischen den einzelnen Markerallelen und dem Krankheitslokus geprüft.

**NICHTPARAMETRISCHE KOPPLUNGSANALYSE ANHAND PEDIGREE I (GROBKARTIERUNG)**

*Singlepoint-Analyse*

Die Ergebnisse der Singlepoint-Analyse mit dem Programm ALLEGRO sind in der Tabelle 6 dargestellt. In fünf Fällen lagen NPL-Werte von über 1,96 für Chromosomenbereiche vor: Auf SSC 3 erreicht der Marker SW2408 an der Position 94,2 cM einen NPL-Wert von 2,05 bei einer Irrtumswahrscheinlichkeit von 0,0220. Auf SSC 6 ergaben sich für die Marker SW122 (83,3 cM) und SW316 (89,3 cM) NPL-Werte von 2,06 bzw. 2,23 und Irrtumswahrscheinlichkeiten von 0,0215 bzw. 0,0146. Die Singlepoint-Analyse für den Marker SW472 (58,9 cM) auf SSC 7 errechnete einen NPL-Wert von 2,42 bei einer Irrtumswahrscheinlichkeit von 0,0093. Für die Marker GH an der Position 45 cM und SW874 an der Position 64,7 cM auf <u>SSC 12</u> ergaben sich NPL-Werte von 2,99 bzw. 2,14 und Irrtumswahrscheinlichkeiten von 0,0019 bzw. 0,0181.

## Tab. 6: Singlepoint-Analyse mit 108 Markern

| Chromosom Marker | cM | NPL-Wert | Exact NPL p-value | ChromosomMarker | cM | NPL-Wert | Exact NPL p-value |
|---|---|---|---|---|---|---|---|
| SSC 1 | 153 | | | SSC 2 | 132 | | |
| SW1515 | 16,4 | -0,38 | 0,6447 | SW2443 | 0 | 0,97 | 0,1668 |
| SW1851 | 44,6 | 0,15 | 0,2662 | SWR783 | 23,7 | 1,08 | 0,1410 |
| SW2185 | 67,6 | 0,32 | 0,1077 | S0141 | 31,2 | 0.63 | 0,2631 |
| S0155 | 93,9 | 0,26 | 0,1184 | SW1201 | 44,8 | 0,17 | 0,4260 |
| S0320 | 112,5 | 0,009 | 0,4838 | SW1026 | 60,6 | 0,48 | 0,3116 |
| SW1301 | 140,5 | 0,12 | 0,3261 | INSL-3 A | 75 | -0,03 | 0,5065 |
| | | | | INSL-3 B | 76 | 0,90 | 0,1839 |
| | | | | SW1408 | 88,5 | 0,32 | 0,3693 |
| | | | | S0036 | 132,1 | 1,46 | 0,0748 |
| SSC 3 | 129 | | | SSC 4 | 131 | | |
| SW274 | 0 | -0,04 | 0,5099 | SW489 | 8 | 0,09 | 0,4582 |
| SW2021 | 12,4 | 1,034 | 0,1507 | S0301 | 27,1 | 0,03 | 0,4813 |
| SW72 | 17,8 | 1,63 | 0,0536 | SW871 | 47,8 | 0,26 | 0,3918 |
| S0206 | 42,3 | 0,39 | 0,3437 | S0073 | 74,4 | -0,27 | 0,6022 |
| SW2408 | 94,2 | 2,05 | 0,0220 | S0097 | 120 | 0,77 | 0,2178 |
| S0002 | 102,2 | 1,85 | 0,0343 | | | | |
| SW349 | 112,6 | 0,86 | 0,1948 | | | | |
| SSC 5 | 145 | | | SSC 6 | 200 | | |
| SW413 | 8,4 | -1,48 | 0,9329 | SW2406 | 21,4 | 0,74 | 0,2268 |
| SW1482 | 39,9 | -0,92 | 0,8210 | SW1841 | 41,5 | 1,40 | 0,0830 |
| SW2425 | 72,3 | 0,50 | 0,3041 | RYR1 | 75 | 0,19 | 0,4220 |
| SW2 | 78,7 | 0,03 | 0,4841 | SW122 | 83,3 | 2,06 | 0,0215 |
| SW1468 | 97,5 | 0,45 | 0,3234 | SW316 | 89,3 | 2,23 | 0,0146 |
| SW378 | 134,4 | 0,46 | 0,3202 | S0003 | 102 | 0,97 | 0,1669 |
| | | | | SW2098 | 106,1 | 1,01 | 0,1570 |
| | | | | SW1881 | 121,1 | 0,38 | 0,3469 |
| | | | | SW2419 | 161,4 | 1,14 | 0,1273 |

## Tab. 6: Singlepoint-Analyse mit 108 Markern (Fortsetzung)

| ChromosomMarker | cM | NPL-Wert | Exact NPL p-value | ChromosomMarker | cM | NPL-Wert | Exact NPL p-value |
|---|---|---|---|---|---|---|---|
| SSC 7 | 157 | | | SSC 8 | 127 | | |
| SW2564 | 0 | 1,56 | 0,0575 | SW2410 | -1,3 | -0,32 | 0,6194 |
| SW1354 | 22,3 | -0,02 | 0,5026 | SW2521 | 23,1 | 0,77 | 0,2200 |
| SW472 | 58,9 | 2,42 | 0,0093 | S0225 | 82,8 | -0,33 | 0,6251 |
| SW352 | 87,7 | 0,80 | 0,2105 | SW1551 | 105,9 | -0,19 | 0,5692 |
| SWR773 | 117,3 | 0,93 | 0,1750 | S0178 | 127,7 | 1,63 | 0,0542 |
| S0212 | 141,2 | -0,51 | 0,6891 | | | | |
| SSC 9 | 142 | | | SSC 10 | 128 | | |
| SW983 | 4 | 1,45 | 0,0753 | SW1894 | 23,2 | 0,08 | 0,4621 |
| SW911 | 36,8 | -0,70 | 0,7554 | SW173 | 56,1 | 0,03 | 0,4824 |
| S0019 | 86,4 | 0,48 | 0,3130 | S0070 | 62,3 | 0,08 | 0,4641 |
| S0295 | 100,5 | 0,65 | 0,2558 | SW920 | 86,3 | 0,54 | 0,2904 |
| SW749 | 139,4 | 0,64 | 0,2590 | SW2067 | 128 | 0,85 | 0,1973 |
| SSC 11 | 84 | | | SSC 12 | 113 | | |
| SW2008 | 14,1 | -0,0006 | 0,4949 | SW2490 | 0 | 0,97 | 0,1669 |
| S0071 | 43,7 | -0,31 | 0,6154 | GH | 45 | 2,99 | 0,0019 |

| ChromosomMarker | cM | NPL-Wert | Exact NPL p-value | ChromosomMarker | cM | NPL-Wert | Exact NPL p-value |
|---|---|---|---|---|---|---|---|
| S0230 | 56,4 | -0,95 | 0,8284 | SW874 | 64,7 | 2,14 | 0,0181 |
| SW703 | 76,2 | 1,30 | 0,0990 | S0090 | 80,2 | 1,89 | 0,0316 |
| SW1135 | 83,7 | -0,56 | 0,7107 | SWR1021 | 113,1 | -0,07 | 0,5216 |
| SSC 13 | 126 | | | SSC 14 | 111 | | |
| SWR1941 | 14,1 | -0,22 | 0,5824 | SW857 | 7,4 | -0,03 | 0,5056 |
| SW344 | 35,4 | 0,50 | 0,3067 | SW295 | 35,7 | -0,03 | 0,5040 |
| SWR1008 | 53 | 0,86 | 0,1950 | SW2439 | 41,5 | 0,17 | 0,4276 |
| S0068 | 62,2 | 0,90 | 0,1833 | SW342 | 53,2 | 0,64 | 0,2589 |
| SW1056 | 96,1 | -0,53 | 0,6994 | S0007 | 60 | 0,45 | 0,3226 |
| S0289 | 112,1 | -0,82 | 0,7929 | SW1557 | 87,9 | 0,17 | 0,4262 |
| | | | | SWC27 | 111,5 | 1,63 | 0,0547 |

Tab. 6: Singlepoint-Analyse mit 108 Markern (Fortsetzung)

| Chromosom Marker | cM | NPL-Wert | Exact NPL p-value | Chromosom Marker | cM | NPL-Wert | Exact NPL p-value |
|---|---|---|---|---|---|---|---|
| SSC 15 | 124 | | | SSC 16 | 93 | | |
| S0355 | 13,8 | 1,61 | 0,0563 | S0111 | 0 | 1,23 | 0,1107 |
| S0148 | 34,6 | 1,41 | 0,0812 | SW813 | 6 | 0,50 | 0,3052 |
| SW964 | 50,7 | 1,06 | 0,1448 | SW742 | 9,3 | 0,72 | 0,2330 |
| S0118 | 60 | 0,52 | 0,2994 | S0298 | 33,2 | 0,28 | 0,3870 |
| SW120 | 79,3 | 1,40 | 0,0835 | SW81 | 40,1 | 0,92 | 0,1778 |
| SW1983 | 101,5 | 0,13 | 0,4442 | SW1897 | 86,2 | 0,26 | 0,3922 |
| SW1119 | 119,9 | 0,42 | 0,3346 | | | | |
| SSC 17 | 100 | | | SSC 18 | 63 | | |
| SWR1004 | 17,8 | 0,32 | 0,3700 | SW1808 | 0 | 0,25 | 0,3984 |
| S0296 | 32 | 0,74 | 0,2274 | LEP | 25 | -0,28 | 0,6051 |
| SW1031 | 63,4 | 0,87 | 0,1900 | SW787 | 31,6 | -0,37 | 0,6394 |
| SW2431 | 94 | 0,47 | 0,3159 | HOXA10 | 49,6 | 0,87 | 0,1904 |
| | | | | SWR414 | 57,6 | 0,003 | 0,4934 |

*Multipoint-Analyse*

Die Multipoint-Analyse mit dem Programm ALLEGRO ergab für fünf Chromosomen (3, 6, 7, 12 und 15) NPL-Werte von über 1,96. Die Ergebnisse der Multipoint-Analyse für diese Chromosomen sind in den Tabellen 7 bis 11 sowie in den Figuren 3 bis 7 dargestellt. Die Marker, die in die Multipoint-Analyse eingingen, waren die selben wie für die Singlepoint-Analyse (siehe Tabelle 6).

Auf SSC 3 konnten für die Marker SW72 (17,8 cM), SW2408 (94,2 cM), S0002 (102,2 cM) und SW349 (112,6 cM) NPL-Werte von über 1,96 errechnet werden (Tabelle 7). Es wird vermutet, dass von zwei Chromosomenbereichen Effekte auf das Merkmal ausgehen (Figur 3).

Tab. 7: Multipoint-Analyse für SSC 3

| Marker | cM | NPL-Wert | Exact NPL p-value |
|---|---|---|---|
| SW274 | 0 | 0,30 | 0,3796 |
| | 6,2 | 0,94 | 0,1737 |
| SW2021 | 12,4 | 1,60 | 0,0567 |
| | 15,1 | 1,88 | 0,0323 |
| **SW72** | **17,8** | **2,15** | **0,0177** |
| | 30,05 | 1,38 | 0,0849 |
| S0206 | 42,3 | 1,01 | 0,1572 |
| | 68,25 | 1,18 | 0,1194 |
| **SW2408** | **94,2** | **2,46** | **0,0081** |
| | **98,2** | **2,47** | **0,0080** |
| **S0002** | **102,2** | **2,58** | **0,0060** |
| | **107,4** | **2,29** | **0,0126** |
| **SW349** | **112,6** | **2,05** | **0,0221** |

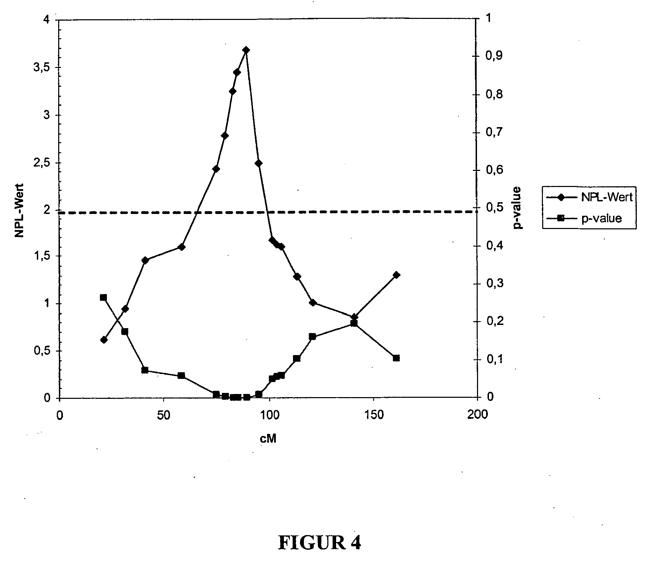

Auf <u>SSC 6</u> ergaben sich für die Marker RYR1 (75 cM), SW122 (83,3 cM) und SW316 (89,3 cM) NPL-Werte von über 1,96 (Tabelle 8). Der eingipfelige Verlauf der Kurve lässt vermuten, dass lediglich ein Chromosombereich mit dem Phänotyp gekoppelt ist (Figur 4).

### Tab. 8: Multipoint-Analyse für SSC 6

| Marker | cM | NPL-Wert | Exact NPL p-value |
|--------|------|----------|-------------------|
| SW2406 | 21,4 | 0,62 | 0,2666 |
|        | 31,45 | 0,94 | 0,1741 |
| SW1841 | 41,5 | 1,46 | 0,0734 |
|        | 58,25 | 1,60 | 0,0570 |
| **RYR1** | **75** | **2,43** | **0,0088** |
|        | **79,15** | **2,78** | **0,0034** |
| **SW122** | **83,3** | **3,24** | **0,0009** |
|        | **85,3** | **3,44** | **0,0005** |
| **SW316** | **89,3** | **3,67** | **0,0002** |
|        | **95,65** | **2,48** | **0,0078** |
| S0003 | 102 | 1,67 | 0,0498 |
|        | 104,05 | 1,62 | 0,0547 |
| SW2098 | 106,1 | 1,60 | 0,0573 |
|        | 113,6 | 1,28 | 0,1018 |
| SW1881 | 121,1 | 1,00 | 0,1595 |
|        | 141,25 | 0,85 | 0,1962 |
| SW2419 | 161,4 | 1,29 | 0,1008 |

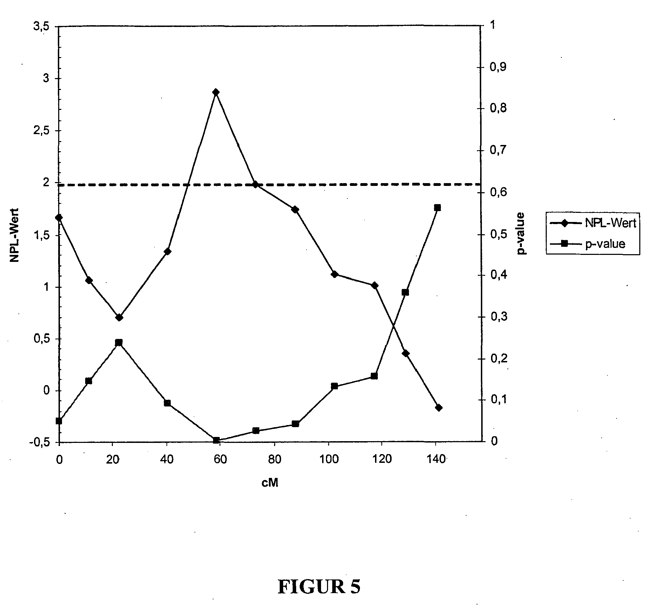

Für den Marker SW472 an der Position 58,9 cM auf <u>SSC 7</u> ergab sich in der Multipoint-Analyse ein NPL-Wert von 2,86 (Tabelle 9). In den benachbarten Regionen sinken die NPL-Werte stark ab (Figur 5), es liegt ein eingipfliger Verlauf der Teststatistik vor.

### Tab. 9: Multipoint-Analyse für SSC 7

| Marker | cM | NPL-Wert | Exact NPL p-value |
|--------|------|----------|-------------------|
| SW2564 | 0 | 1,66 | 0,0515 |
|        | 11,15 | 1,06 | 0,1447 |
| SW1354 | 22,3 | 0,7 | 0,2384 |
|        | 40,6 | 1,34 | 0,0926 |
| **SW472** | **58,9** | **2,86** | **0,0029** |
|        | **73,3** | **1,98** | **0,0265** |

| Marker | cM | NPL-Wert | Exact NPL p-value |
|--------|------|----------|-------------------|
| SW352 | 87,7 | 1,74 | 0,0437 |
|        | 102,5 | 1,11 | 0,1338 |
| SWR773 | 117,3 | 1,01 | 0,1555 |
|        | 129,25 | 0,35 | 0,357 |
| S0212 | 141,2 | -0,17 | 0,5632 |

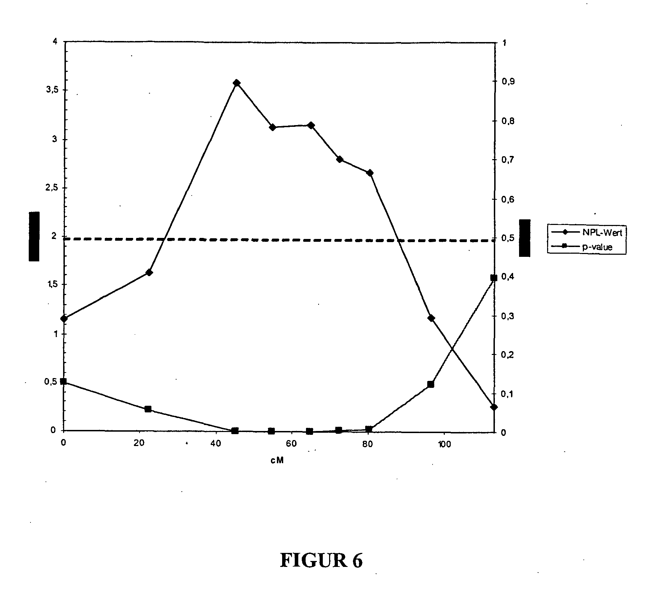

In der Multipoint-Analyse für <u>SSC 12</u> konnten für die Marker GH (45 cM), SW874 (64,7 cM) und S0090 (80,2 cM) NPL-Werte von über 1,96 errechnet werden (Tabelle 10). Die Statistik der NPL-Werte über 1,96 überspannt einen Bereich von ca. 25 cM bis 90 cM (Figur 6).

Tab. 10: Multipoint-Analyse für SSC 12

| Marker | cM | NPL-Wert | Exact NPL p-value |
|---|---|---|---|
| SW2490 | 0 | 1,15 | 0,1254 |
| | 22,5 | 1,63 | 0,0542 |
| **GH** | **45** | **3,58** | **0,0003** |
| | **54,85** | **3,13** | **0,0013** |
| **SW874** | **64,7** | **3,15** | **0,0011** |
| | **72,45** | **2,8** | **0,0033** |
| **S0090** | **80,2** | **2,67** | **0,0047** |
| | 96,65 | 1,17 | 0,1225 |
| SWR1021 | 113,1 | 0,26 | 0,3946 |

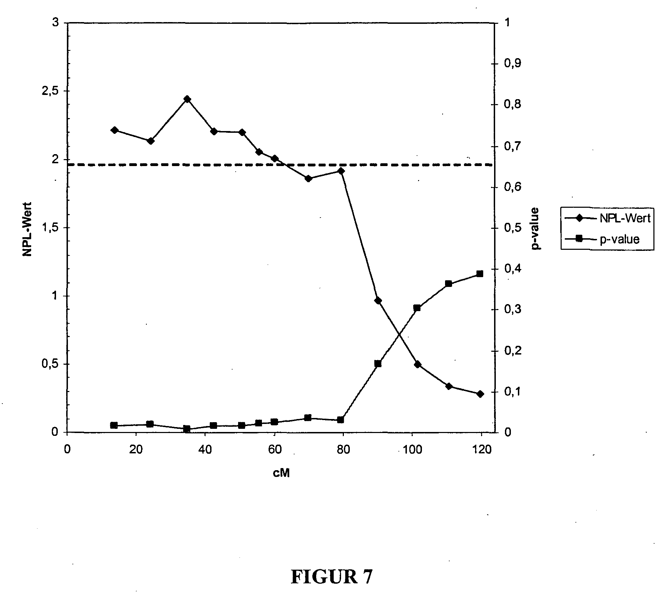

In der Multipoint-Analyse erreichten vier Marker (S0355, S0148, SW964 und S0118) auf <u>SSC 15</u> NPL-Werte von über 1,96 in dem Bereich von 13,8 cM bis 60 cM (Tabelle 11 und Figur 7).

Tab. 11: Multipoint-Analyse für SSC 15

| Marker | cM | NPL-Wert | Exact NPL p-value |
|---|---|---|---|
| S0355 | 13,8 | 2,22 | 0,0152 |
| | 24,2 | 2,14 | 0,0182 |
| S0148 | 34,6 | 2,44 | 0,0088 |
| | 42,65 | 2,21 | 0,0157 |
| SW964 | 50,7 | 2,20 | 0,0158 |
| | 55,35 | 2,06 | 0,0221 |
| S0118 | 60 | 2,01 | 0,0243 |
| | 69,65 | 1,86 | 0,0341 |

Tab. 11: Multipoint-Analyse für SSC 15 (Fortsetzung)

| Marker | cM | NPL-Wert | Exact NPL p-value |
|---|---|---|---|
| SW120 | 79,3 | 1,92 | 0,0301 |
| | 90,4 | 0,97 | 0,1664 |
| SW1983 | 101,5 | 0,50 | 0,3036 |
| | 110,7 | 0,34 | 0,3628 |
| SW1119 | 119,9 | 0,28 | 0,3863 |
| | 13,8 | 2,22 | 0,0152 |

## NICHTPARAMETRISCHE KOPPLUNGSANALYSE ANHAND PEDIGREE II (FEINKARTIERUNG)

*Singlepoint-Analyse*

Die Ergebnisse der Typisierung weiterer Marker auf den Chromosomen 3, 6, 7, 12 und 15 am Pedigree II sind in Tabelle 12 dargestellt (die zusätzlichen Marker sind ebenso wie in Tabelle 5 orange markiert). Die zusätzlichen Loci waren so platziert, dass sie die Markerdichte in den gekoppelten Chromosomenbereichen erhöhten, oder aber in wenigen Fällen auch noch vorhandene Lücken auf den Chromosomen nach der Grobkartierung schlossen.

Der Marker SW72 an der Position 17,8 erreichte nach Hinzufügen von drei Markern, zwischen 17,2 cM und 19 cM gelegen, einen NPL-Wert von 2,29 bei einer Irrtumswahrscheinlichkeit von 0,0125. Durch die zusätzlichen Typisierungen verschob sich die zweite Position eines Effekts auf <u>SSC 3,</u> von Position 94,2 cM (SW2408) zu Position 102,2 cM (S0002). Der NPL-Wert stieg auf 2,31 an, ebenso halbierte sich die Irrtumswahrscheinlichkeit auf p = 0,0119. Somit liegen auf <u>SSC 3</u> auch nach Analyse von Pedigree II zwei Regionen, die mit dem Merkmal Hernien gekoppelt sind. Der NPL-Wert des Markers SW122 an Position 83,3 cM auf <u>SSC 6</u> sank am Pedigree II geringfügig auf 2,04 (p = 0,0221), während der NPL-Wert des Markers SW316 (89,3 cM) deutlich unter 1,96 (1,72) sank. Der zusätzliche Marker MP35 an Position 0 cM erreichte einen NPL-Wert von 2,06 bei einer Irrtumswahrscheinlichkeit von p = 0,0212. Auf <u>SSC 7</u> sank der NPL-Wert des Markers SW472 von 2,42 (p = 0,0093) auf 2,12 (p = 0,0187). Der neue Marker SW2019 distal zu SW472 an Position 60,5 cM wies einen NPL-Wert von 1,50 (p = 0,0684) auf, während der proximale zusätzliche Marker TNFB an Position 58,1 cM einen NPL-Wert von 0,30 (p = 0,3782) erreichte. Durch die Feinkartierung auf <u>SSC 12</u> mit den Markern S0229 und SW1307 erstreckt sich nun der erste Effekt auf den Bereich 19,3 cM bis 45 cM. Der höchste NPL-Wert findet sich an der Position des GH-Gens. Zusätzliche Marker und Meiosen ließen außerdem den NPL-Wert an Position 80,2 cM (S0090) auf 2,55 ansteigen (p = 0,0063), womit sich nach Singlepoint-Analyse die Position eines zweiten Effekts ergab. Auf SSC 15 erreichte weiterhin kein Marker einen NPL-Wert von über 1,96 nach der Singlepoint-Analyse.

### Tab. 12: Singlepoint-Analyse nach Pedigree-Erweiterung und Feinkartierung

| Chromosom Marker | cM | NPL-Wert | Exact NPL p-value | Chromosom Marker | cM | NPL-Wert | Exact NPL p-value |
|---|---|---|---|---|---|---|---|
| SSC 3 | 129 | | | SSC 6 | 200 | | |
| SW274 | 0 | -0,03 | 0,5091 | MP35 | 0 | 2,06 | 0,0212 |
| SW2021 | 12,4 | 1,37 | 0,0873 | SW2406 | 21,4 | 0,67 | 0,2487 |
| SW2429 | 17,2 | 1,58 | 0,0587 | SW1841 | 41,5 | 1,26 | 0,1045 |
| SW833 | 17,3 | 0,53 | 0,2969 | SW1067 | 71,4 | 1,28 | 0,1021 |
| SW72 | 17,8 | 2,29 | 0,0125 | RYR1 | 75 | 0,29 | 0,3826 |
| SE5101S | 19 | 1,49 | 0,0694 | SW1376 | 76,5 | 1,38 | 0,0857 |
| S0701 | 31,46 | 0,46 | 0,3188 | SW1129 | 80,2 | 1,31 | 0,0960 |
| S0206 | 42,3 | 0,26 | 0,3949 | SW122 | 83,3 | 2,04 | 0,0221 |
| SW2408 | 94,2 | 1,48 | 0,0707 | SW617 | 86,5 | 0,48 | 0,3121 |
| S0002 | 102,2 | 2,31 | 0,0119 | SWR987 | 86,6 | 1,57 | 0,0603 |
| S0397 | 109 | 0,05 | 0,4758 | SW316 | 89,3 | 1,72 | 0,0453 |
| SW1327 | 109,6 | 1,73 | 0,0441 | SW2505 | 90,2 | 1,83 | 0,0355 |
| SW349 | 112,6 | 1,38 | 0,0852 | S0003 | 102 | 1,20 | 0,1163 |
| | | | | SW2098 | 106,1 | 0,91 | 0,1806 |
| | | | | SW1881 | 121,1 | 0,35 | 0,3609 |
| | | | | SW322 | 149,8 | 0,02 | 0,4863 |
| | | | | SW2419 | 161,4 | 1,03 | 0,1508 |
| SSC 7 | 157 | | | SSC 12 | 113 | | |
| SW2564 | 0 | 1,29 | 0,0999 | SW2490 | 0 | 0,14 | 0,4406 |
| SW1354 | 22,3 | -0,02 | 0,5023 | S0229 | 19,3 | 2,48 | 0,0076 |
| SW1369 | 48,2 | -0,05 | 0,5153 | SW1307 | 40,2 | 2,02 | 0,0236 |
| SY33 | 57,7 | 0,99 | 0,1617 | GH | 45 | 3,06 | 0,0015 |
| TNFB | 58,1 | 0,30 | 0,3782 | SW874 | 64,7 | 1,30 | 0,0984 |
| SW472 | 58,9 | 2,12 | 0,0187 | SW168 | 70,5 | 0,85 | 0,1959 |
| SW2019 | 60,5 | 1,50 | 0,0684 | S0090 | 80,2 | 2,55 | 0,0063 |
| SW1856 | 61,5 | 0,77 | 0,2190 | S0147 | 89,9 | 0,68 | 0,2455 |
| SW352 | 87,7 | 0,64 | 0,2584 | LOX12A | 102,5 | 0,70 | 0,2394 |
| SWR773 | 117,3 | 0,75 | 0,2255 | SW605 | 108,3 | 1,03 | 0,1513 |
| S0212 | 141,2 | -0,41 | 0,6548 | SWR1021 | 113,1 | 0,69 | 0,2435 |

### Tab. 12: Singlepoint-Analyse nach Pedigree-Erweiterung und Feinkartierung (Fortsetzung)

| Chromosom Marker | cM | NPL-Wert | Exact NPL p-value |
|---|---|---|---|
| SSC 15 | 124 | | |
| SW2072 | 12,5 | 0,35 | 0,3615 |
| S0355 | 13,8 | 1,17 | 0,1210 |
| SW919 | 25,7 | 1,35 | 0,0896 |
| S0148 | 34,6 | 0,92 | 0,1778 |
| SW1111 | 39,8 | 0,74 | 0,2294 |
| SW964 | 50,7 | 0,22 | 0,4090 |
| S0369 | 56 | 0,27 | 0,3913 |
| S0118 | 60 | 0,004 | 0,4942 |
| SWR1533 | 70,4 | 0,68 | 0,2465 |
| SW120 | 79,3 | 1,05 | 0,1482 |
| SW906 | 89,3 | 0,62 | 0,2667 |
| SW1983 | 101,5 | 0,17 | 0,4303 |
| SW1119 | 119,9 | 0,35 | 0,3553 |

*Multipoint-Analyse*

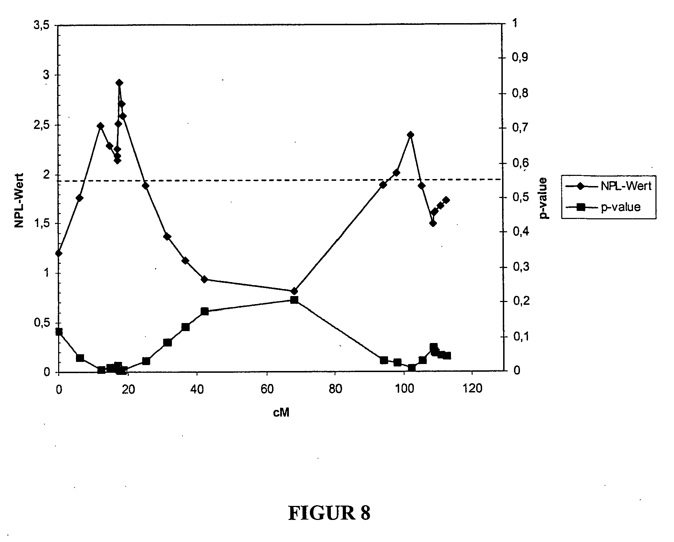

Unter Einbeziehung der zusätzlich typisierten Marker am Pedigree II stieg in der Multipoint-Analyse der NPL-Wert des Markers SW72 auf SSC 3 von 2,15 auf 2,92 bei einer Irrtumswahrscheinlichkeit von 0,0022. Auch die umgebenden Marker von den Positionen 12,4 cM bis 19 cM erreichten NPL-Werte, die über 2 lagen. Die NPL-Werte der distalen Marker auf SSC 3 sanken bis auf den NPL-Wert von S0002 (NPL-Wert = 2,38; p = 0,0097) unter 1,96 (Tabelle 13 und Figur 8). Die Multipoint-Ergebnisse am Pedigree II bestätigen damit, wie die Singlepoint-Analyse, das Vorhandensein von zwei chromosomalen Regionen, die mit dem Effekt assoziiert sind.

Tab. 13: Multipoint-Analyse nach Pedigree-Erweiterung und Feinkartierung für SSC 3

| Marker | cM | NPL-Wert | Exact NPL p-value |
|---|---|---|---|
| SW274 | 0 | 1,19 | 0,1170 |
| | 6,2 | 1,75 | 0,0423 |
| SW2021 | 12,4 | 2,49 | 0,0074 |
| | 14,8 | 2,29 | 0,0123 |
| SW2429 | 17,2 | 2,14 | 0,0177 |
| | 17,25 | 2,19 | 0,0156 |
| SW833 | 17,3 | 2,24 | 0,0137 |
| | 17,55 | 2,51 | 0,0070 |
| SW72 | 17,8 | 2,92 | 0,0022 |
| | 18,4 | 2,71 | 0,0041 |
| SE51018 | 19 | 2,58 | 0,0057 |
| | 25,23 | 1,87 | 0,0322 |
| S0701 | 31,46 | 1,37 | 0,0866 |
| | 36,88 | 1,13 | 0,1302 |
| S0206 | 42,3 | 0,93 | 0,1740 |
| | 68,25 | 0,81 | 0,2075 |
| SW2408 | 94,2 | 1,88 | 0,0318 |
| | 98,2 | 2,01 | 0,0241 |
| S0002 | 102,2 | 2,38 | 0,0097 |
| | 105,6 | 1,87 | 0,0326 |
| S0397 | 109 | 1,49 | 0,0697 |
| | 109,3 | 1,61 | 0,0560 |
| SW1327 | 109,6 | 1,62 | 0,0543 |
| | 111,1 | 1,67 | 0,0490 |
| SW349 | 112,6 | 1,73 | 0,0434 |

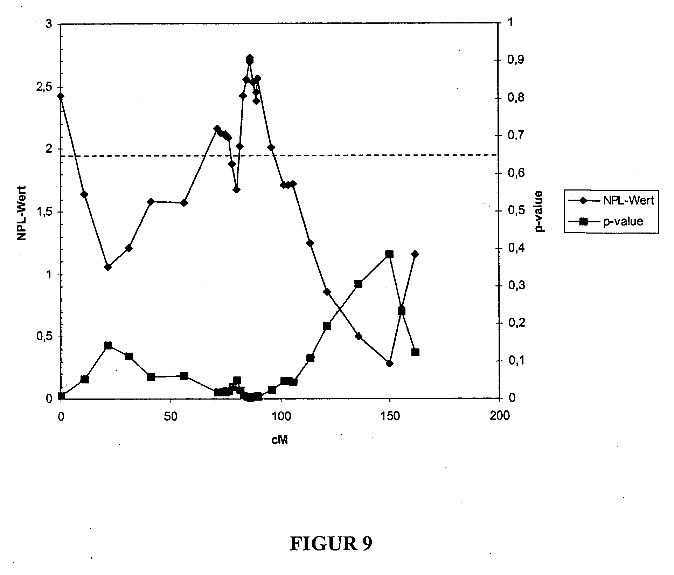

Auf <u>SSC 6</u> hatten drei Bereiche NPL-Werte über 1,96 (Tabelle 14 und Figur 9). Der Marker MP35 an der Position 0 cM hatte einen NPL-Wert von 2,43 (p = 0,0086). Die beiden anderen Bereiche lagen zwischen 71,4 und 76,5 cM sowie zwischen 81,8 und 96,1 cM. Den höchsten NPL-Wert auf diesem Chromosom erreichte SW617 an Position 86,5 cM mit 2,73 (p = 0,0039).

Tab. 14: Multipoint-Analyse nach Pedigree-Erweiterung und Feinkartierung für SSC 6

| Marker | cM | NPL-Wert | Exact NPL p-value |
|---|---|---|---|
| MP35 | 0 | 2,43 | 0,0086 |
| | 10,7 | 1,64 | 0,0527 |
| SW2406 | 21,4 | 1,06 | 0,1452 |
| | 31,45 | 1,21 | 0,1134 |
| SW1841 | 41,5 | 1,58 | 0,0587 |
| | 56,45 | 1,57 | 0,0603 |
| SW1067 | 71,4 | 2,16 | 0,0171 |
| | 73,2 | 2,13 | 0,0181 |
| RYR1 | 75 | 2,12 | 0,0187 |
| | 75,75 | 2,10 | 0,0194 |
| SW1376 | 76,5 | 2,09 | 0,0200 |
| | 78,35 | 1,88 | 0,0321 |
| SW1129 | 80,2 | 1,68 | 0,0489 |

| | | | |
|---|---|---|---|
| | 81,8 | 2,02 | 0,0233 |
| SW122 | 83,4 | 2,43 | 0,0086 |
| | 85 | 2,55 | 0,0063 |
| SW617 | 86,5 | 2,73 | 0,0039 |
| | 86,55 | 2,71 | 0,0041 |
| SWR987 | 86,6 | 2,69 | 0,0043 |
| | 87,95 | 2,53 | 0,0066 |
| SW316 | 89,3 | 2,38 | 0,0099 |
| | 89,75 | 2,45 | 0,0082 |
| SW2505 | 90,2 | 2,56 | 0,0062 |
| | 96,1 | 2,01 | 0,0238 |
| S0003 | 102 | 1,71 | 0,0456 |
| | 104,05 | 1,71 | 0,046 |
| SW2098 | 106,1 | 1,72 | 0,0447 |
| | 113,6 | 1,24 | 0,1094 |
| SW1881 | 121,1 | 0,86 | 0,1929 |
| | 135,45 | 0,50 | 0,3068 |
| SW322 | 149,8 | 0,28 | 0,3849 |
| | 155,6 | 0,72 | 0,2337 |
| SW2419 | 161,4 | 1,16 | 0,124 |

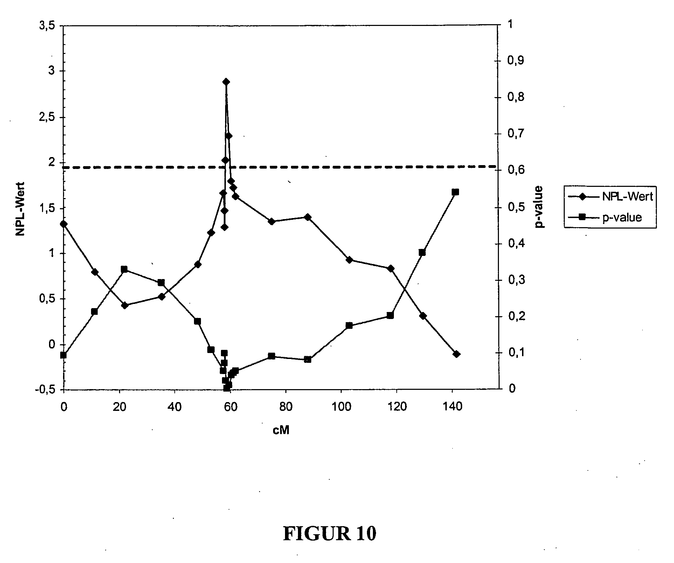

Der NPL-Wert des Markers SW472 auf SSC 7 veränderte sich in der Mulitpoint-Analyse von 2,86 (p = 0,0029), am Pedigree I berechnet, durch die weiteren Marker und Meiosen nur geringfügig auf 2,88 (p = 0,0025) am Pedigree II (Tabelle 15 und Figur 10).

## Tab. 15: Multipoint-Analyse nach Pedigree-Erweiterung und Feinkartierung für SSC 7

| Marker | cM | NPL-Wert | Exact NPL p-value |
|---|---|---|---|
| SW2564 | 0 | 1,33 | 0,0928 |
| | 11,15 | 0,79 | 0,2143 |
| SW1354 | 22,3 | 0,43 | 0,3306 |
| | 35,25 | 0,53 | 0,2944 |
| SW1369 | 48,2 | 0,88 | 0,1883 |
| | 52,95 | 1,23 | 0,1102 |
| SY33 | 57,7 | 1,66 | 0,0504 |
| | 57,9 | 1,47 | 0,0718 |
| TNFB | 58,1 | 1,29 | 0,0997 |
| | 58,5 | 2,02 | 0,0236 |
| SW472 | 58,9 | 2,88 | 0,0025 |
| | 59,7 | 2,29 | 0,0123 |
| SW2019 | 60,5 | 1,8 | 0,0382 |
| | 61,25 | 1,72 | 0,0451 |
| SW1856 | 62 | 1,63 | 0,0528 |
| | 75,1 | 1,35 | 0,0902 |
| SW352 | 88,2 | 1,4 | 0,0828 |
| | 103 | 0,93 | 0,1762 |
| SWR773 | 117,8 | 0,83 | 0,2029 |
| | 129,75 | 0,31 | 0,3757 |
| S0212 | 141,7 | -0,11 | 0,5395 |

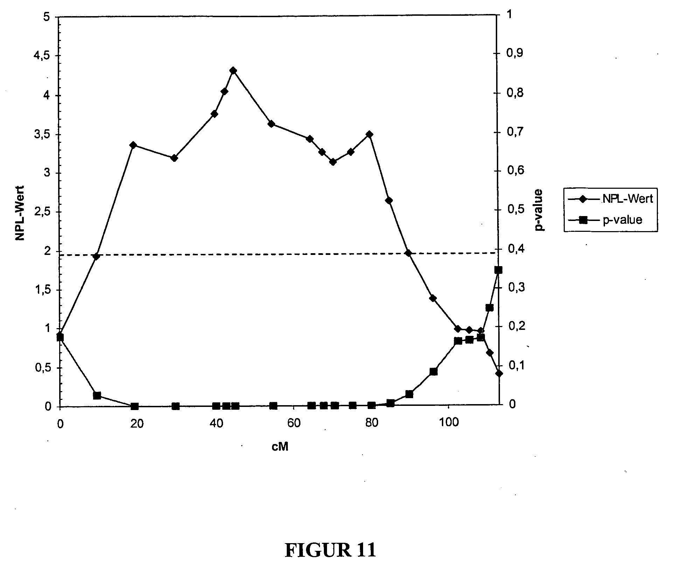

Auf SSC 12 stiegen nach Feinkartierung die NPL-Werte für die Marker im Chromosomenbereich zwischen 19,3 cM (S0229) und 80,2 cM (S0090), die schon an Pedigree I untersucht wurden, deutlich an. Die zusätzlich typisierten Marker wiesen NPL-Werte von über 3 auf. Den höchsten NPL-Wert erreichte der Marker GH mit einem NPL-Wert von 4,30 bei einer Irrtumswahrscheinlichkeit von p = 0,000018 (Tabelle 16 und Figur 11).

Tab. 16: Multipoint-Analyse nach Pedigree-Erweiterung und Feinkartierung für SSC 12

| Marker | cM | NPL-Wert | Exact NPL p-value |
|---|---|---|---|
| SW2490 | 0 | 0,92 | 0,1796 |
| | 9,65 | 1,93 | 0,0286 |
| S0229 | 19,3 | 3,35 | 0,000582 |
| | 29,75 | 3,18 | 0,0010 |
| SW1307 | 40,2 | 3,76 | 0,00014 |
| | 42,6 | 4,04 | 0,0000504 |
| GH | 45 | 4,30 | 0,0000178 |
| | 54,85 | 3,62 | 0,000235 |
| SW874 | 64,7 | 3,43 | 0,000451 |
| | 67,6 | 3,26 | 0,000789 |
| SW168 | 70,5 | 3,13 | 0,0012 |
| | 75,35 | 3,26 | 0,000783 |
| S0090 | 80,2 | 3,48 | 0,000377 |
| | 85,05 | 2,63 | 0,005 |
| S0147 | 89,9 | 1,95 | 0,0272 |
| | 96,2 | 1,37 | 0,0864 |
| LOX12A | 102,5 | 0,98 | 0,1645 |
| | 105,4 | 0,96 | 0,1681 |
| SW605 | 108,3 | 0,95 | 0,1721 |
| | 110,7 | 0,67 | 0,2506 |
| SWR1021 | 113,1 | 0,39 | 0,3449 |

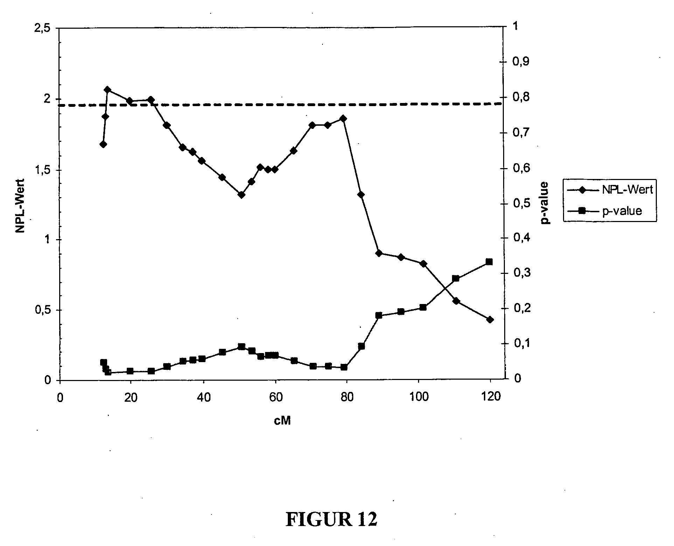

Durch die zusätzlichen Marker und Meiosen sanken die NPL-Werte im Vergleich zur Grobkartierung auf SSC 15 im Bereich zwischen 13,8 cM (S0355) und 60 cM (S0118). Nur noch der Marker S0355 an der Position 13,8 cM und der zusätzlich typisierte Marker SW919 an der Position 25,7 cM weisen NPL-Werte von über 1,96 auf (Tabelle 17 und Figur 12).

Tab. 17: Multipoint-Analyse nach Pedigree-Erweiterung und Feinkartierung für SSC 15

| Marker | cM | NPL-Wert | Exact NPL p-value |
|---|---|---|---|
| SW2072 | 12,5 | 1,68 | 0,0488 |
| | 13,15 | 1,87 | 0,0327 |
| S0355 | 13,8 | 2,06 | 0,0213 |
| | 19,75 | 1,98 | 0,0255 |
| SW919 | 25,7 | 1,99 | 0,0251 |
| | 30,15 | 1,81 | 0,0371 |
| S0148 | 34,6 | 1,65 | 0,0518 |
| | 37,2 | 1,62 | 0,0551 |
| SW1111 | 39,8 | 1,56 | 0,0611 |
| | 45,25 | 1,44 | 0,077 |
| SW964 | 50,7 | 1,32 | 0,094 |
| | 53,35 | 1,41 | 0,081 |
| S0369 | 56 | 1,51 | 0,0672 |
| | 58 | 1,5 | 0,0687 |
| S0118 | 60 | 1,5 | 0,0689 |
| | 65,2 | 1,63 | 0,0539 |
| SWR1533 | 70,4 | 1,81 | 0,0368 |
| | 74,85 | 1,81 | 0,03722 |
| SW120 | 79,3 | 1,86 | 0,0344 |
| | 84,3 | 1,32 | 0,0943 |
| SW906 | 89,3 | 0,9 | 0,1826 |

| Marker | cM | NPL-Wert | Exact NPL p-value |
|---|---|---|---|
| | 95,4 | 0,87 | 0,1909 |
| SW1983 | 101,5 | 0,82 | 0,2042 |
| | 110,7 | 0,56 | 0,2846 |
| SW1119 | 119,9 | 0,42 | 0,3327 |

**Beispiel 4: Assoziationsanalyse**

Um die Ergebnisse aus der Kopplungsanalyse abzusichern und zu bestätigen, wurde mit Hilfe des Programms GASSOC 1.06 für die Marker der Chromosomen 3, 6, 7, 12 und 15 ein TDT-Test durchgeführt. Der TDT-Score mit den dazugehörigen Irrtumswahrscheinlichkeiten ist in Tabelle 18 dargestellt. Liegt die Irrtumswahrscheinlichkeit für einen Marker unter 5 %, kann davon ausgegangen werden, dass für diesen Marker ein Kopplungsungleichgewicht vorliegt.

Tab. 18: TDT-Tests

| Chromosom Marker | cM | TDT-Score | TDT-p-value | Chromosom Marker | cM | TDT-Score | TDT-p-value |
|---|---|---|---|---|---|---|---|
| SSC 3 | 129 | | | SSC 6 | 200 | | |
| SW274 | 0 | 4,9 | 0,555 | MP35 | 0 | 12,34 | 0,0304 |
| SW2021 | 12,4 | 16,55 | 0,220 | SW2406 | 21,4 | 8,92 | 0,1782 |
| SW2429 | 17,2 | 13,3 | 0,038 | SW1841 | 41,5 | 15,07 | 0,3029 |
| SW833 | 17,3 | 11,55 | 0,021 | SW1067 | 71,4 | 7,09 | 0,6278 |
| SW72 | 17,8 | 11,4 | 0,043 | RYR1 | 75 | 1 | 0,3173 |
| SE51018 | 19 | 9,72 | 0,373 | SW1376 | 76,5 | 18,46 | 0,0024 |
| S0701 | 31,46 | 3,33 | 0,188 | SW1129 | 80,2 | 9,26 | 0,2347 |
| S0206 | 42,3 | 4,69 | 0,584 | SW122 | 83,3 | 19,76 | 0,0113 |
| SW2408 | 94,2 | 9,21 | 0,602 | SW617 | 86,5 | 1,89 | 0,1693 |
| S0002 | 102,2 | 19,00 | 0,088 | SWR987 | 86,6 | 14,31 | 0,0264 |
| S0397 | 109 | 10,85 | 0,456 | SW316 | 89,3 | 11,38 | 0,2506 |
| SW1327 | 109,6 | 14,03 | 0,051 | SW2505 | 90,2 | 9,89 | 0,1295 |
| SW349 | 112,6 | 13,64 | 0,136 | S0003 | 102 | 4,79 | 0,7796 |
| | | | | SW2098 | 106,1 | 3,16 | 0,6752 |
| | | | | SW1881 | 121,1 | 5,62 | 0,4673 |
| | | | | SW322 | 149,8 | 6,94 | 0,5430 |
| | | | | SW2419 | 161,4 | 22,49 | 0,0041 |
| SSC 7 | 157 | | | SSC 12 | 113 | | |
| SW2564 | 0 | 4,56 | 0,3360 | SW2490 | 0 | 13,84 | 0,2418 |
| SW1354 | 22,3 | 2,51 | 0,7749 | S0229 | 19,3 | 8,92 | 0,5397 |
| SW1369 | 48,2 | 9,35 | 0,0958 | SW1307 | 40,2 | 7,42 | 0,0596 |
| SY33 | 57,7 | 3,91 | 0,6888 | GH | 45 | 8,77 | 0,0325 |
| TNFB | 58,1 | 12,19 | 0,2726 | SW874 | 64,7 | 3,64 | 0,6026 |
| SW472 | 58,9 | 0,15 | 0,9266 | SW168 | 70,5 | 1,76 | 0,7805 |
| SW2019 | 60,5 | 7,68 | 0,2623 | S0090 | 80,2 | 6,77 | 0,2384 |
| SW1856 | 61,5 | 8,62 | 0,1958 | S0147 | 89,9 | 3,93 | 0,5591 |
| SW352 | 87,7 | 5,67 | 0,2252 | LOX12A | 102,5 | 3,60 | 0,1654 |
| SWR773 | 117,3 | 0,54 | 0,7636 | SW605 | 108,3 | 7,50 | 0,3787 |
| S0212 | 141,2 | 2,25 | 0,8131 | SWR1021 | 113,1 | 12,46 | 0,0524 |

Tab. 18: Ergebnisse des TDT-Tests (Fortsetzung)

| Chromosom Marker | cM | TDT-Score | TDT-p-value |
|---|---|---|---|
| SSC 15 | 124 | | |
| SW2072 | 12,5 | 7,00 | 0,3210 |
| S0355 | 13,8 | 6,14 | 0,6310 |
| SW919 | 25,7 | 15,44 | 0,3489 |
| S0148 | 34,6 | 9,82 | 0,2782 |
| SW1111 | 39,8 | 10,74 | 0,1504 |

| Chromosom Marker | cM | TDT-Score | TDT-p-value |
|---|---|---|---|
| SW964 | 50,7 | 12,35 | 0,1363 |
| S0369 | 56 | 1,33 | 0,7212 |
| S0118 | 60 | 3,88 | 0,4222 |
| SWR1533 | 70,4 | 5,14 | 0,1621 |
| SW120 | 79,3 | 6,68 | 0,3515 |
| SW906 | 89,3 | 2,36 | 0,6703 |
| SW1983 | 101,5 | 14,85 | 0,1893 |
| SW1119 | 119,9 | 3,36 | 0,7626 |

Auf SSC 3 lag die Irrtumswahrscheinlichkeit für die Marker SW2429, SW833 und SW72 unter 5 %. Die TDT-Ergebnisse bestätigen damit für diese Marker die Ergbnisse der nichtparametrischen Kopplunsanalyse. Die Irrtumswahrscheinlichkeiten für die Marker S0002 und SW1327 lagen unter 10 %. Für den Marker S0002 wurde damit Assoziation zum Merkmal nachgewiesen und die Ergebnisse der Kopplungsanalyse wurden bestätigt, während für den Marker SW1327 mit einem NPL-Wert von 1,77 keine Kopplung nach nichtparametrischer Kopplungsanalyse aufgezeigt wurde.

Fünf Marker auf SSC 6 (MP35, SW1376, SW122, SWR987 und SW2419) erreichten im TDT-Test signifikante Testergebnisse mit Irrtumswahrscheinlichkeiten von unter 5 %. Bis auf den Marker SW2490 (161,4 cM) stimmen somit die Bereiche überein, die in der Kopplungsanalyse NPL-Werte von über 1,96 und im TDT-Test signifikante Ergebnisse zeigen.

Auf SSC 7 konnte keine Assoziation zwischen Marker und Merkmal festgestellt werden. Die höchste Wahrscheinlichkeit einer Assoziation besteht für den Marker SW1369 mit einem TDT-p-value von 0,0958.

Für den Marker GH auf SSC 12 wurde der Hinweis auf Kopplung mit dem Krankheitslokus nach der nichtparametrischen Kopplungsanalyse bestätigt. Die Irrtumswahrscheinlichkeit im TDT-Test lag bei 3,25 %. Der distal gelegene Marker SW1307 erreichte eine Irrtumswahrscheinlichkeit von 5,96 %, was den NPL-Wert von 2,02 in der Singlepoint-Analyse und von 3,76 in der Multipoint-Analyse bestätigt. Im TDT-Test wurde für den Marker SWR1021 eine Assoziation bei einer Irrtumswahrscheinlichkeit von 5,24 % errechnet. Dieses Ergebnis kann nicht durch die Kopplungsanalyse unterstützt werden. Auf SSC 15 konnte für keinen Marker eine Assoziation zu *Hernia inguinalis/scrotalis* bestimmt werden. Die Irrtumswahrscheinlichkeiten lagen für alle Marker über 13,5 %.

**Beispiel 5: Bestimmung von Haplotypen, die mit dem Phänotyp *Hernia inguinalis/scrotalis* assoziiert sind**

Im Folgenden werden Haplotypendaten präsentiert, wobei unter dem Begriff "Haplotypen" die gemeinsame Vererbung von Allelen zweier benachbarter Marker an assoziierten Chromosomenregionen verstanden wird, die die höchsten Signifikanzwerte aufzeigen. Weiterhin wird zudem das prozentuale Vorkommen der einzelnen Haplotypen bei den betroffenen Tieren angegeben. Somit wird zu der neuartigen Information, dass von den genannten Chromosomenregionen Effekte auf den Phänotyp Hernien ausgehen auch genannt, welche genetische Variation bevorzugt bei den betroffenen Tieren auftreten. Die Haplotypen wurden vom Programm Allegro gebildet und die Prozentzahlen durch einfaches Auszählen bestimmt.

Nachfolgend sind die Daten für die einzelnen assoziierten Chromosomenregionen auf den Chromosomen 12, 6, 3, 15 und 7 aufgelistet. Tabelle 19 fasst diese Experimentaldaten zusammen. Auf den Chromosomen 12, 6 und 3 befinden sich zwei unabhängige Chromosomenregionen, die mit dem Herniendefekt assoziiert sind, während auf den Chromosomen 7 und 15 jeweils ein Bereich definiert ist.

**Chromosom 12:**

**HAPLOTYP SSC12A**

Haplotyp SSC12A definiert Chromosomenbereich 40,2 cM bis 45 cM: Haplotypmarker 1 (SW1307 an Position 40,2 cM), Haplotypmarker 2 (GH an Position 45 cM): 51,25% der Betroffenen zeigen Haplotypen 13 (11,04%), 14 (17,08%) und 44 (23,13%); die restlichen Betroffenen verteilen sich auf 11 weitere Haplotypen. Wobei SW1307 4 Allele und 1=120bp, 2=122bp, 3=126bp und 4=132bp; wobei GH 4 Allele, wobei ein Ausgangsfragment von 605 bp aus dem GH-Gen (GenBank Acc. No. M17704) im Bereich -119 bis +486 mittels des Primers1 5'-CTGGGGAGCTTACAAACTCCTT-3' und des Primers2 5'-TTATCCATTAGCACATGCCTGCCAG-3'

**Tabelle 19: Haplotypen in den assoziierten Chromosomenregionen**

| Porcines Chromosom | Haplotyp-ID | N[1] | Haplotypmarker 1 & Position (cM) | Allele Haplotypmarker 1 | Haplotypmarker 2 & Position (cM) | Allele Haplotypmarker 2 | Haplotyp[2] | % bei betroffenen Tieren | N und % weiterer Haplotypen |
|---|---|---|---|---|---|---|---|---|---|
| 12 | SSC12A | 14 | SW1307; 40,2 | 1=120bp; 2=122bp; 3=126bp; 4=132bp; | GH; 45,0 | 1=605bp; 2=498&107bp; 3=449bp&156bp; 4=449bp,107bp&49bp; | 13 | 11,04 | |
| | | | | | | | 14 | 17,08 | |
| | | | | | | | 44 | 23,13 | |
| | | | | | | | | $\sum$ 51,25 | 11 (48,75) |
| 12 | SSC12B | 20 | SW168; 70,5 | 1=95bp; 2=97bp; 3=99bp; 4=101bp; 5=109bp; 6=111bp; | S0090; 80,2 | 1=241bp; 2=243bp; 3=245bp; 4=247bp; 5=249bp; 6=251bp; | 32 | 9,47 | |
| | | | | | | | 62 | 22,02 | |
| | | | | | | | 64 | 9,47 | |
| | | | | | | | 66 | 9,67 | |

| Porcines Chromosom | Haplotyp-ID | N[1] | Haplotypmarker 1 & Position (cM) | Allele Haplotypmarker 1 | Haplotypmarker 2 & Position (cM) | Allele Haplotypmarker 2 | Haplotyp[2] | % bei betroffenen Tieren | N und % weiterer Haplotypen |
|---|---|---|---|---|---|---|---|---|---|
| 6 | SSC6A | 31 | MP35; 0 | 1=160bp; 2=162bp; 3=164bp; 4=166bp; 5=172bp; 6=176bp | SW2406; 21,4 | 1=233bp; 2=230bp; 3=232bp; 4=237bp; 5=252bp; 6=254bp; 7=256bp; 8=258bp | | Σ 50,63 | 16 (49,37) |
| | | | | | | | 21 | 13,69 | |
| | | | | | | | 31 | 11,61 | |
| | | | | | | | 61 | 16,67 | |
| | | | | | | | 34 | 14,88 | |
| | | | | | | | 64 | 10,12 | |
| 6 | SSC6B | 11 | SW617; 86,5 | 1=139bp; 2=149bp | SWR987; 86,6 | 1=94bp; 2=98bp; 3=102bp; 4=104bp; 5=109bp; 6=115bp; 7=123bp | | Σ 66,97 | 26 (33,03) |
| | | | | | | | 12 | 28,98 | |
| | | | | | | | 16 | 19,91 | |
| | | | | | | | 23 | 28,54 | |

| Porcines Chromosom | Haplotyp-ID | N[1] | Haplotypmarker 1 & Position (cM) | Allele Haplotypmarker 1 | Haplotypmarker 2 & Position (cM) | Allele Haplotypmarker 2 | Haplotyp[2] | % bei betroffenen Tieren | N und % weiterer Haplotypen |
|---|---|---|---|---|---|---|---|---|---|
| | | | | | | | 24 | 14,60 | |
| | | | | | | | | Σ 92,03 | 7 (7,97) |
| 3 | SSC3A | 31 | SW72; 17,8 | 1=99bp; 2=101bp; 3=103bp; 4=107bp; 5=109bp; 6=111bp; | SE51018; 19,0 | 1=83bp; 2=85bp; 3=87bp; 4=89bp; 5=93bp; 6=96bp; 7=101bp; 8=103bp; 9=110bp; 10=112bp; | 15 | 24,27 | |
| | | | | | | | 16 | 10,24 | |
| | | | | | | | 45 | 12,97 | |
| | | | | | | | 46 | 15,43 | |
| | | | | | | | 55 | 10,25 | |
| | | | | | | | | Σ 73,16 | 26 (26,84) |
| 3 | SSC3B | 45 | SW2408; 94,2 | 1=175bp; 2=191bp; 3=196bp; 4=200bp; 5=202bp; | S0002; 102,2 | 1=153bp; 2=155bp; 3=157bp; 4=159bp; 5=161bp; 6=163bp; 7=165bp; 8=167bp; 9=169bp; 10=171bp; | 16 | 7,64 | |

| Porcines Chromosom | Haplotyp-ID | N[1) | Haplotypmarker 1 & Position (cM) | Allele Haplotypmarker 1 | Haplotypmarker 2 & Position (cM) | Allele Haplotypmarker 2 | Haplotyp[2) | % bei betroffenen Tieren | N und % weiterer Haplotypen |
|---|---|---|---|---|---|---|---|---|---|
| | | | | 6=204bp; 7=206bp; | | 11=179bp; 12=187bp; 13=189bp; | | | |
| | | | | | | | 46 | 5,99 | |
| | | | | | | | 56 | 9,30 | |
| | | | | | | | 59 | 7,44 | |
| | | | | | | | | $\sum$ 30,37 | 41 (69,63) |
| 15 | SSC15A | 53 | S0355; 13,8 | 1=246bp; 2=250bp; 3=252bp; 4=255bp; 5=257bp; 6=259bp; 7=267bp; 8=272bp; | SW919; 25,7 | 1=84bp; 2=94bp; 3=96bp; 4=100bp; 5=102bp; 6=114bp; 7=115bp; 8=116bp; 9=121bp; 10=123bp; 11=125bp; 12=128bp; 13=130bp; 14=132bp; 15=136bp; 16=138bp; 17=152bp; | 14 | 23,05 | |
| | | | | | | | 24 | 4,94 | |
| | | | | | | | 22 | 7,61 | |
| | | | | | | | | $\sum$ 35,60 | 50 (64,40) |

| Porcines Chromosom | Haplotyp-ID | N[1] | Haplotypmarker 1 & Position (cM) | Allele Haplotypmarker 1 | Haplotypmarker 2 & Position (cM) | Allele Haplotypmarker 2 | Haplotyp[2] | % bei betroffenen Tieren | N und % weiterer Haplotypen |
|---|---|---|---|---|---|---|---|---|---|
| 7 | SSC7A | 16 | SW472; 58,9 | 1=93bp; 2=95bp; 3=102bp; | SW2019; 60,5 | 1=126bp; 2=128bp; 3=130bp; 4=136bp; 5=139bp; 6=141bp; 7=143bp; | 12 | 14,20 | |
| | | | | | | | 13 | 9,88 | |
| | | | | | | | 14 | 9,88 | |
| | | | | | | | 23 | 18,21 | |
| | | | | | | | 24 | 11,42 | |
| | | | | | | | | Σ 63,59 | 11 (36,41) |

amplifiziert wurde und nach Restriktion mit dem Enzym Hhal oder einem Isoschizomer vier Allele aufzeigt, definiert als 1=605bp, 2=498bp und 107bp, 3=449bp und 156bp, sowie 4=449bp, 107bp und 49bp. Beispielsweise ist der Haplotyp13 daher durch das Allel 120bp des Markers SW1307 und durch das Allel 3 des Markers GH gebildet.

**HAPLOTYP SSC12B**

Haplotyp SSC12B definiert Chromosomenbereich 70,5 cM bis 80,2 cM: Haplotypmarker 1 (SW168 an Position 70,5 cM) und Haplotypmarker 2 (S0090 an Position 80,2 cM): 50,63% der Betroffenen zeigen Haplotypen 32 (9,47%), 62 (22,02%), 64 (9,47%) und 66 (9,67%); die restlichen Betroffenen verteilen sich auf 16 weitere Haplotypen. Wobei SW168 6 Allele und 1=95bp, 2=97bp, 3=99bp, 4=101bp, 5=109bp und 6=111bp; wobei S0090 6 Allele und 1=241bp, 2=243 bp, 3=245bp, 4=247bp, 5=249bp und 6=251 bp. Beispielsweise ist der Haplotyp62 daher durch das Allel 111bp des Markers SW168 und durch das Allel 243 des Markers S0090 gebildet.

**Chromosom 6:**

**HAPLOTYP SSC6A**

Haplotyp SSC6A definiert Chromosomenbereich 0 cM bis 21,4 cM: Haplotypmarker 1 (MP35 an Position 0 cM), Haplotypmarker 2 (SW2406 an Position 21,4cM): 66,97% der Betroffenen zeigen Haplotypen 21 (13,69%), 31 (11,61%), 61 (16,67%), 34 (14,88%) und 64 (10,12%); die restlichen Betroffenen verteilen sich auf 26 weitere Haplotypen. Wobei MP35 6 Allele und 1=160bp, 2=162bp, 3=164bp, 4=166bp, 5=172bp und 6=176bp; wobei SW2406 8 Allele und 1=223bp, 2=230bp, 3=232bp, 4=237bp, 5=252bp, 6=254bp, 7=256bp und 8=258bp. Beispielsweise ist der Haplotyp61 daher durch das Allel 176bp des Markers MP35 und durch das Allel 223bp des Markers SW2406 gebildet.

**HAPLOTYP SSC6B**

Haplotyp SSC6B definiert Chromosomenbereich 86,5 cM bis 86,6 cM: Haplotypmarker 1 (SW617 an Position 86,5 cM), Haplotypmarker 2 (SWR987 an Postion 86,6 cM): 92,03% der Betroffenen zeigen Haplotypen 12 (28,98%), 16 (19,91%), 23 (28,54%) und 24 (14,60%); die restlichen Betroffenen verteilen sich auf 7 weitere Haplotypen. Wobei SW617 2 Allele und 1=139bp, 2=149bp; wobei SWR987 7 Allele und 1 =94bp, 2=98bp, 3=102bp, 4=104bp, 5=109bp, 6=115bp und 7=123bp. Beispielsweise ist der Haplotyp16 daher durch das Allel 139bp des Markers SW617 und durch das Allel 115bp des Markers SWR987 gebildet.

**Chromosom 3:**

**HAPLOTYP SSC3A**

Haplotyp SSC3A definiert Chromosomenbereich 17,8 cM bis 19,0 cM: Haplotypmarker 1 (SW72 an Position 17,8 cM), Haplotypmarker 2 (SE51018 an Position 19 cM): 73,16% der Betroffenen zeigen Haplotypen 15 (24,27%), 16 (10,24%), 45 (12,97%), 46 (15,43%) und 55 (10,25%); die restlichen Betroffenen verteilen sich auf 26 weitere Haplotypen. Wobei SW72 6 Allele und 1=99bp, 2=101 bp, 3=103bp, 4=107bp, 5=109bp und 6=111 bp; wobei SE51018 10 Allele und 1=83bp, 2=85bp, 3=87bp, 4=89bp, 5=93bp, 6=96bp, 7=101bp, 8=103bp, 9=110bp und 10=112bp. Beispielsweise ist der Haplotyp15 daher durch das Allel 99bp des Markers SW72 und durch das Allel 93bp des Markers SE51018 gebildet.

**HAPLOTYP SSC3B**

Haplotyp SSC3B definiert Chromosomenbereich 94,2 cM bis 102,2 cM: Haplotypmarker 1 (SW2408 an Position 94,2 cM), Haplotypmarker 2 (S0002 an Postion 102,2 cM): 30,37% der Betroffenen zeigen Haplotypen 16 (7,64%), 46 (5,99%), 56 (9,30%) und 59 (7,44%); die restlichen Betroffenen verteilen sich auf 41 weitere Haplotypen. Wobei SW2408 7 Allele und 1=175bp, 2=191bp, 3=196bp, 4=200bp, 5=202bp, 6=204bp und 7=206bp; wobei S0002 13 Allele und 1=153bp, 2=155bp, 3=157bp, 4=159bp, 5=161bp, 6=163bp, 7=165bp, 8=167bp, 9=169bp, 10=171bp, 11=179bp, 12=187bp und 13=189bp. Beispielsweise ist der Haplotyp16 daher durch das Allel 175bp des Markers SW2408 und durch das Allel 163bp des Markers S0002 gebildet.

**Chromosom 15:**

**HAPLOTYP SSC15A**

Haplotyp SSC15A definiert Chromosomenbereich 13,8 cM bis 25,7 cM: Haplotypmarker 1 (S0355 an Position 13,8cM), Haplotypmarker 2 (SW919 an Position 25,7cM): 35,60% der Betroffenen zeigen Haplotypen 14 (23,05%), 24 (4,94%) und 22 (7,61%); die restlichen Betroffenen verteilen sich auf 50 weitere Haplotypen. Wobei: S0355 8 Allele und 1=246 bp, 2=250 bp, 3= 252bp, 4=255bp, 5=257bp, 6= 259bp, 7=267bp und 8=272bp; wobei: SW919 17 Allele und 1=84bp, 2=94bp, 3=96bp, 4=100bp, 5=102bp, 6=114bp, 7=115bp, 8=116bp, 9=121bp, 10=123bp, 11=125bp, 12=128bp, 13=130bp, 14=132bp, 15=136bp, 16=138bp und 17=152bp. Beispielsweise ist der Haplotyp14 daher durch das Allel 246bp des Markers S0355 und durch das Allel 100bp des Markers SW919 gebildet.

**Chromosom 7:**

**HAPLOTYP SSC7A**

Haplotyp SSC7A definiert Chromosomenbereich 58,9 cM bis 60,5 cM: Haplotypmarker 1 (SW472 an Position 58,9 cM), Haplotypmarker 2 (SW2019 an Position 60,5 cM): 63,59% der Betroffenen zeigen Haplotypen 12 (14,20%), 13 (9,88%), 14 (9,88%), 23 (18,21%) und 24 (11,42%); die restlichen Betroffenen verteilen sich auf 11 weitere Haplotypen. Wobei SW472 3 Allele und 1=93bp, 2=95bp und 3=102bp; wobei SW2019 7 Allele und 1=126bp, 2=128bp, 3=130bp, 4=136bp, 5=139bp, 6=141bp und 7=143bp. Beispielsweise ist der Haplotyp12 daher durch das Allel 93bp des Markers SW472 und durch das Allel 128bp des Markers SW2019 gebildet.

**Patentansprüche**

1. Verwendung einer ersten Nukleinsäure zur Bestimmung der Prädisposition zur Ausprägung oder Vererbung des Phänotypus "Hernia inguinalis/scrotalis" bei einem Säuger, wobei die erste Nukleinsäure eine Länge von mindestens 8 Nukleotiden aufweist und identisch oder im wesentlichen identisch ist mit einer zweiten Nukleinsäure, die vorkommt,

   (a) auf Chromosom 12 von Sus scrofa im Bereich zwischen 19,3 cM und 85,05 cM;
   (b) auf Chromosom 6 von Sus scrofa im Bereich zwischen 71,4 cM und 96,1 cM oder im Bereich von 0 cM;
   (c) auf Chromosom 3 von Sus scrofa im Bereich zwischen 12,4 cM und 19 cM oder im Bereich zwischen 98,2 cM und 102,2 cM;
   (d) auf Chromosom 7 von Sus scrofa im Bereich zwischen 58,5 cM und 59,7 cM;
   (e) auf Chromosom 15 von Sus scrofa im Bereich zwischen 13,8 cM und 25,7 cM; oder
   (f) in dem chromosomalen Bereich eines anderen Säugers, der zu den in (a) bis (e) genannten Bereichen ortholog ist;

   wobei die erste Nukleinsäure in dem oben genannten Bereich lokalisiert ist oder 5 Megabasen stromaufwärts und/ oder stromabwärts hiervon.

2. Verwendung einer Kombinationen von mindestens zwei oder mehr der in Anspruch 1 genannten Nukleinsäuren zur Bestimmung der Ausprägung oder Vererbung des Phänotypus "Hernia inguinalis/scrotalis".

**3.** Verwendung nach einem der Ansprüche 1 bis 2, wobei die zweite Nukleinsäure ein SNP, ein Mikrosatellit oder eine den SNP oder Mikrosatelliten flankierende Sequenz ist, wobei die flankierende Sequenz einen Bereich von bis zu 5 Megabasen stromaufwärts und stromabwärts des Mikrosatelliten umfasst.

**4.** Verwendung nach Anspruch 3, wobei der Mikrosatellit oder SNP ausgewählt wird aus der Gruppe bestehend aus

(a) den auf Chromosom 12 von Sus scrofa gelegenen Mikrosatelliten S0229 im Bereich von 19,3 cM, SW1307 im Bereich von 40,2 cM, ein SNP im Bereich des Gens GH im Bereich von 45 cM, SW874 im Bereich von 64,7 cM, SW168 im Bereich von 70,5 cM, S0090 im Bereich von 80,2 cM;
(b) den auf Chromosom 6 von Sus scrofa gelegenen Mikrosatelliten MP35 im Bereich von 0 cM, SW1067 im Bereich von 71,4 cM, ein SNP im Bereich von 75 cM im Bereich des Gens RYR1, SW1376 im Bereich von 76,5 cM; SW122 im Bereich von 83,4 cM, SW617 im Bereich von 86,5 cM, SWR987 im Bereich von 86,6 cM, SW316 im Bereich von 89,3 cM, SW2505 im Bereich von 90,2 cM;
(c) den auf Chromosom 3 von Sus scrofa gelegenen Mikrosatelliten SW2021 im Bereich von 12,4 cM, SW2429 im Bereich von 17,2 cM, SW833 im Bereich von 17,3 cM, SW72 im Bereich von 17,8 cM, SE51018 im Bereich von 19 cM; S0002 im Bereich von 102,2 cM;
(d) dem auf Chromosom 7 von Sus scrofa gelegenen Mikrosatelliten SW472 im Bereich von 58,9 cM; und
(e) den auf Chromosom 15 von Sus scrofa gelegenen Mikrosatelliten S0355 im Bereich von 13,8 cM, SW919 im Bereich von 25,7 cM.

**5.** Verwendung nach einem der Ansprüche 1 bis 4, wobei der Nachweis von zumindest einem spezifischen Allel erfolgt.

**6.** Verwendung nach Anspruch 5, wobei zwei oder mehr Allele nachgewiesen werden.

**7.** Verwendung nach einem der Ansprüche 1 bis 6, wobei die zweite Nukleinsäure von einem auf Chromosom 12 von Sus scrofa gelegenen Gen oder von einem orthologen Gen eines anderen Säugers abgeleitet ist.

**8.** Verwendung nach Anspruch 7, wobei das Gen ausgewählt wird aus der Gruppe bestehend aus Wachstumshormon-Gen (GH), SRY (sex determining region Y)-box 9 (*SOX9*), Paired box homeotic Gen 8 (*PAX8*), Homeobox B-Gencluster (*HOXB*).

**9.** Verwendung nach einem der Ansprüche 1 bis 6, wobei die zweite Nukleinsäure von einem auf Chromosom 6 von Sus scrofa gelegenen Gen oder einem orthologen Gen eines anderen Säugers abgeleitet ist.

**10.** Verwendung nach Anspruch 9, wobei das Gen ausgewählt wird aus der Gruppe bestehend aus Cytochrom b light (alpha) chain *(CYBA)*, Metallothionein 2A *(MT2A)*, Transforming Growth Factor beta 1 (*TGFB1*), $\alpha$-3-Laminin *(LAMA3)*, Extrazelluläres Glycoprotein (*EMILIN*2), Apolipoprotein E 1-Gen *(APOE1)*, Alpha 1 B Gylcoprotein (A1BG).

**11.** Verwendung nach einem der Ansprüche 1 bis 6, wobei die zweite Nukleinsäure von einem auf Chromosom 3 von Sus scrofa gelegenen Gen oder einem orthologen Gen eines anderen Säugers abgeleitet ist.

**12.** Verwendung nach Anspruch 11, wobei das Gen ausgewählt wird aus der Gruppe bestehend aus ß-Glucuronidase *(GUSB)*, Cytochrom P450 subfamily IIIA (CYP3A), Cytochrom P450 CYP3A29 (CYP3A29), Porcines Homeobox Gen (HBX24), Luteinizing hormone/choriogonadothrophic receptor *(LHCGR)*, Apolipoprotein B (*APOB*).

**13.** Verwendung nach einem der Ansprüche 1 bis 6, wobei die zweite Nukleinsäure von einem auf Chromosom 7 von Sus scrofa gelegenen Gen oder einem orthologen Gen eines anderen Säugers abgeleitet ist.

**14.** Verwendung nach Anspruch 13, wobei das Gen ausgewählt wird aus der Gruppe bestehend aus Tumor Nekrosefaktor alpha (TNFA), Tumor Nekrosefaktor beta (TNFB), Major Histokompatibilitätskomplex *(SLA)*, MHC class IPD14 major transplantation antigen (*MHCTA1*), Prolactin (*PRL*).

**15.** Verwendung nach einem der Ansprüche 1 bis 6, wobei die zweite Nukleinsäure von einem auf Chromosom 15 von Sus scrofa gelegenen Gen oder einem orthologen Gen eines anderen Säugers abgeleitet ist.

**16.** Verwendung nach Anspruch 15, wobei das Gen ausgewählt wird aus der Gruppe bestehend aus Integrin $\alpha$-V (*ITGAV*), Collagen-III-$\alpha$-1 (*COL3A1*), Homeobox D-Gencluster *(HOXD)*, Engrailed 1-Gen *(EN1).*

**17.** Verwendung einer in einem der Ansprüche 1 bis 16 genannten zweiten Nukleinsäure zur Selektion von nicht-menschlichen Säugern ohne Phänotypus "Hernia inguinalis/scrotalis".

**18.** Verwendung nach einem der Ansprüche 1 bis 17, wobei der Säuger ausgewählt wird aus der Gruppe bestehend aus Sus scrofa, Rind, Pferd, Hund und Mensch.

**19.** Verwendung nach einem der Ansprüche 1 bis 18, wobei Sus scrofa ausgewählt wird aus der Gruppe bestehend aus Deutsche Landrasse, Deutsches Edelschwein, Hampshire, Pietrain, Schwäbisch-Hällisches Schwein, Bunte Bentheimer, Angler Sattelschwein oder hierauf basierenden Hybriden oder Züchtungen.

**20.** Verwendung nach Anspruch 19, wobei zumindest einer der in Tabelle 19 definierten Haplotypen

(a) SSC12A: 13, 14 oder 44;
(b) SSC12B: 32, 62, 64 oder 66;
(c) SSC6A: 21, 31, 61, 34 oder 64;
(d) SSC6B: 12, 16, 23 oder 24;
(e) SSC3A: 15, 16, 45, 46 oder 55;
(f) SSC3B: 16, 46, 56 oder 59;
(g) SSC15A: 14, 22 oder 24;
(h) SSC7A: 12, 13, 14, 23 oder 24

nachgewiesen wird.

**21.** Verwendung nach einem der Ansprüche 1 bis 20, wobei ein Genomscreen an mehreren Tieren einer Population durchgeführt wird.

**22.** In vitro Verfahren zur Bestimmung der Prädisposition zur Ausprägung oder Vererbung des Phänotypus "Hernia inguinalis/scrotalis", wobei man die Säuger, deren befruchtete oder unbefruchtete Eizellen, oder deren Sperma auf die Anwesenheit, Beschaffenheit oder Ausprägung einer in einem der Ansprüche 1 bis 19 genannten zweiten Nukleinsäure testet.

**23.** Verfahren nach Anspruch 22, wobei der Säuger ausgewählt wird aus der Gruppe bestehend aus Sus scrofa, Rind, Pferd, Hund und Mensch.

**24.** Verfahren nach Anspruch 23, wobei Sus scrofa ausgewählt wird aus der Gruppe bestehend aus Deutsche Landrasse, Deutsches Edelschwein, Hampshire, Pietrain, Schwäbisch-Hällisches Schwein, Bunte Bentheimer, Angler Sattelschwein oder hierauf basierenden Hybriden oder Züchtungen.

**25.** Verfahren nach einem der Ansprüche 22 bis 24, wobei man

(a) eine PCR Amplifikation mit komplementären Primern mit einer Länge von mindestens 8 Nukleotiden durchführt, wobei ein Primer an den + Strang und ein weiterer Primer in entgegengesetzter Orientierung den - Strang der in einem der Ansprüchen 1 bis 20 genannten zweiten Nukleinsäure bindet;
(b) eine Hybridisierung durchführt, wobei eine Hybridisierungssonde mit einer Länge von mindestens 8 Nukleotiden an die in einem der Ansprüchen 1 bis 20 genannte zweite Nukleinsäure bindet;
(c) eine Sequenzierung der in einem der Ansprüchen 1 bis 20 genannten zweiten Nukleinsäure durchführt; oder
(d) eine Detektion mit einem spezifischen Antikörper oder Antikörperfragment oder Antikörperderivat oder Aptamer durchführt, wobei der Antikörper oder das Antiköperfragment oder das Antikörperderivat oder der Aptamer spezifisch gegen eine in einem der Ansprüchen 1 bis 20 genannte erste oder zweite Nukleinsäure gerichtet ist.

**26.** Verfahren nach einem der Ansprüche 22 bis 25, wobei ein Genomscreen an mehreren Säugern einer Population durchgeführt wird.

**27.** Verfahren nach einem der Ansprüche 24 bis 26, wobei zumindest einer der in Tabelle 19 definierten Haplotypen

(a) SSC12A: 13, 14 oder 44;
(b) SSC12B: 32, 62, 64 oder 66;
(c) SSC6A: 21, 31, 61, 34 oder 64;

(d) SSC6B: 12, 16, 23 oder 24;
(e) SSC3A: 15, 16, 45, 46 oder 55;
(f) SSC3B: 16, 46, 56 oder 59;
(g) SSC15A: 14, 22 oder 24;
(h) SSC7A: 12, 13, 14, 23 oder 24

nachgewiesen wird.

**28.** Kit mindestens enthaltend

(a) ein Primerpaar zur Amplifikation der in den Ansprüchen 1 bis 20 genannten zweiten Nukleinsäure, wobei jeweils ein Primer an den + Strang und ein weiterer Primer an den - Strang dieser Nukleinsäure bindet; oder
(b) eine Hybridisierungssonde mit einer Länge von mindestens 8 Nukleotiden die an die in einem der Ansprüchen 1 bis 29 genannte zweite Nukleinsäure bindet; oder
(c) einen spezifischen Antikörper oder ein Antikörperfragment oder ein Antikörperderivat oder ein Aptamer das an die in einem der Ansprüchen 1 bis 20 genannte erste oder zweite Nukleinsäure bindet;

in einem oder mehreren Behältern.

**Figur 1**

FIGUR 2

**FIGUR 3**

**FIGUR 4**

**FIGUR 5**

**FIGUR 6**

**FIGUR 7**

**FIGUR 8**

**FIGUR 9**

**FIGUR 10**

**FIGUR 11**

**FIGUR 12**